# EUROPEAN PATENT SPECIFICATION

(11) **EP 3 762 382 B1**
(45) Date of publication and mention of the grant of the patent: **29.04.2026**
(21) Application number: 19709664.7
(22) Date of filing: 04.03.2019
(51) Int. Cl.: C07D 405/14, C07D 491/147, C07D 491/048, C07D 409/14, C07D 405/04, C07C 211/00, C07D 333/78, C07D 307/93, C07D 519/00, C09K 11/00, C07F 7/08

(54) **MATERIALS FOR ORGANIC ELECTROLUMINESCENT DEVICES**
MATERIALIEN FÜR ORGANISCHE ELEKTROLUMINESZENTE VORRICHTUNGEN
MATÉRIAUX POUR DISPOSITIFS ÉLECTROLUMINESCENTS ORGANIQUES

(30) Priority: 06.03.2018 EP 18160182
(43) Date of publication of application: 13.01.2021
(73) Proprietor: Merck Patent GmbH, 64293 Darmstadt (DE)
(72) Inventor: LINGE, Rouven, 64291 DARMSTADT (DE); MEYER, Sebastian, 60594 FRANKFURT AM MAIN (DE); RODRIGUEZ, Lara-Isabel, 64285 DARMSTADT (DE)
(74) Representative: Merck Patent Association
(86) International application number: PCT/EP2019/055254
(87) International publication number: WO 2019/170572

(56) References cited:
- CN-A- 107 573 306
- KR-A- 20150 114 636
- KR-A- 20180 048 302
- SHIH-HSUN LIN ET AL: "Isotruxene-Derived Cone-Shaped Organic Dyes for Dye-Sensitized Solar Cells", JOURNAL OF ORGANIC CHEMISTRY, vol. 75, no. 22, 19 November 2010 (2010-11-19), pages 7877 - 7886, XP055581435, ISSN: 0022-3263, DOI: 10.1021/jo101831p

## Description

The present invention relates to a compound of the formula (3A), (3B) or (3C) to the use of the compound in an electronic device, and to an electronic device comprising a compound of the formula (3A), (3B) or (3C). The present invention furthermore relates to a formulation comprising one or more compounds of the formula (3A), (3B) or (3C).

The development of functional compounds for use in electronic devices is currently the subject of intensive research. The aim is, in particular, the development of compounds with which improved properties of electronic devices in one or more relevant points can be achieved, such as, for example, power efficiency and lifetime of the device as well as colour coordinates of the emitted light.

In accordance with the present invention, the term electronic device is taken to mean, inter alia, organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

Of particular interest is the provision of compounds for use in the last-mentioned electronic devices called OLEDs. The general structure and the functional principle of OLEDs are known to the person skilled in the art and are described, for example, in US 4539507.

Further improvements are still necessary with respect to the performance data of OLEDs, in particular with a view to broad commercial use, for example in display devices or as light sources. Of particular importance in this connection are the lifetime, the efficiency and the operating voltage of the OLEDs and as well as the colour values achieved. In particular, in case of blue-emitting OLEDs, there is potential for improvement with respect to the lifetime and the efficiency of the devices.

An important starting point for achieving the said improvements is the choice of the emitter compound and of the host compound employed in the electronic device.

Blue-fluorescent emitters known from the prior art are a multiplicity of compounds. Arylamines containing one or more condensed aryl are known from the prior art. Arylamines containing dibenzofuran groups (for example in US 2017/0012214) are also known from the prior art. Further, the use of diaryldiamino-indenofluorene derivatives to which a benzofuran or benzothiophene group is condensed as blue emitter material is also known from CN 107573306.

However, there is still a need for further fluorescent emitters, especially blue-fluorescent emitters, which may be employed in OLEDs and lead to OLEDs having very good properties in terms of lifetime, color emission and efficiency. More particularly, there is a need for blue-fluorescent emitters combining very high efficiencies, very good lifetime and suitable colour coordinates.

Furthermore, it is known that an OLED may comprise different layers, which may be applied either by vapour deposition in a vacuum chamber or by processing from a solution. The processes based on vapour deposition lead to very good results but such processes are quite complex and expensive. Therefore, there is also a need for OLED materials that can be easily and reliably processed from solution. In this case, the materials should have good solubility properties in the solution that comprises them.

The present invention is thus based on the technical object of providing compounds which are suitable for use in electronic devices, such as OLEDs, more particularly as blue-fluorescent emitters or matrix materials and, which are suitable for vacuum processing or for solution processing.

In investigations on novel compounds for use in electronic devices, it has now been found, that compounds of formulae (3A), (3B) or (3C) as defined below are eminently suitable for use in electronic devices. In particular, they achieve one or more, preferably all, of the above-mentioned technical objects.

The invention thus relates to compounds of formula (3A), (3B) or (3C) 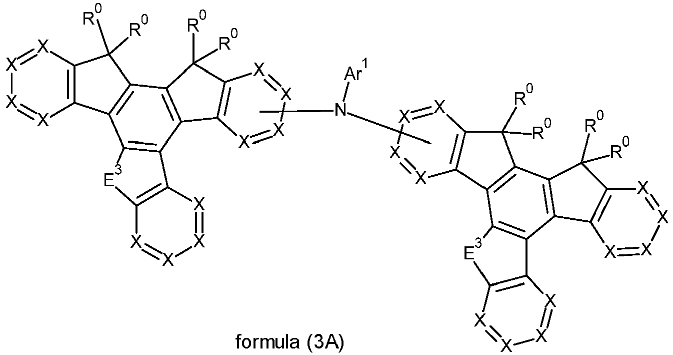 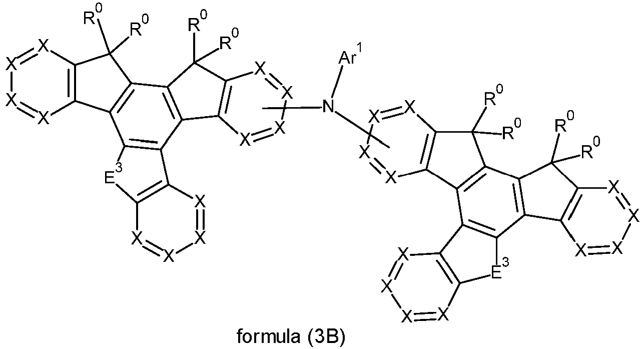 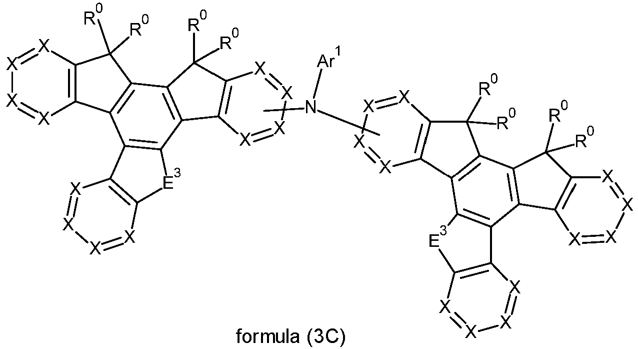 where the following applies to the symbols and indices used:
- E³: stands for -O-;
- Ar¹: stands on each occurrence, identically or differently, for an aromatic or heteroaromatic ring system having 5 to 30 aromatic ring atoms, which may in each case be substituted by one or more radicals R¹;
- X: stands for CR² or N; or X stands for C, if it is bonded to the nitrogen atom as depicted in formulae (3A), (3B) and (3C);
- R⁰,: stands on each occurrence, identically or differently, for H, D, F, a straight-chain alkyl group having 1 to 10 C atoms or branched or a cyclic alkyl group having 3 to 10 C atoms, each of which may be substituted by one or more radicals R, where one or more H atoms may be replaced by D or F, an aromatic or heteroaromatic ring system having 5 to 18 aromatic ring atoms, which may in each case be substituted by one or more radicals R; where two adjacent radicals R⁰ may form an aliphatic or aromatic ring system with one another, which may be substituted by one or more radicals R;
- R¹, R²: stand on each occurrence, identically or differently, for H, D, F, CN, a straight-chain alkyl group having 1 to 10 C atoms or a branched or a cyclic alkyl group having 3 to 10 C atoms, each of which may be substituted by one or more radicals R, where one or more H atoms may be replaced by D or F, an aromatic or heteroaromatic ring system having 6 to 18 aromatic ring atoms, which may in each case be substituted by one or more radicals R;
- R: stands on each occurrence, identically or differently, for H, D, F, CN, a straight-chain alkyl group having 1 to 10 C atoms or a branched or a cyclic alkyl group having 3 to 10 C atoms, each of which may be substituted by one or more radicals R', an aromatic or heteroaromatic ring system having 5 to 30 aromatic ring atoms, which may in each case be substituted by one or more radicals R';
- R': stands on each occurrence, identically or differently, for H, D, F, Cl, Br, I, CN, a straight-chain alkyl, alkoxy or thioalkyl groups having 1 to 10 C atoms or branched or cyclic alkyl, alkoxy or thioalkyl groups having 3 to 10 C atoms.

When two adjacent radicals R⁰ form a ring system with one another, they preferably form a ring which results in the formation of a spiro structure as follows: 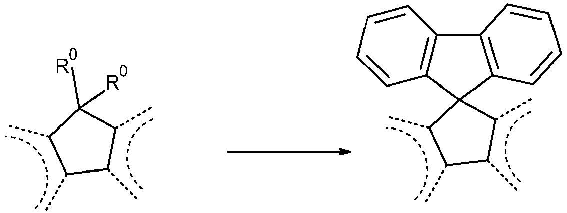 where the dashed lines indicate the adjacent aromatic or heteroaromatic rings and where the benzene rings in the spiro structure may be substituted by one or more radicals R at any free positions.

Preferably, R² stands for H.

The following definitions of chemical groups apply for the purposes of the present application:
Adjacent substituents in the sense of the present invention are substituents which are bonded to atoms which are linked directly to one another, or which are bonded to the same atom.

An aryl group in the sense of this invention contains 6 to 60 aromatic ring atoms, preferably 6 to 40 aromatic ring atoms, more preferably 6 to 20 aromatic ring atoms; a heteroaryl group in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aromatic ring system contains 6 to 60 C atoms in the ring system, preferably 6 to 40 C atoms, more preferably 6 to 20 C atoms. A heteroaromatic ring system in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 30 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphtha-cene, pentacene, benzopyrene, biphenyl, biphenylene, terphenyl, terphenyl-ene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

A straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl or 2,2,2-trifluoroethyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-tri-fluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethyl-thio.

The formulation that two radicals may form a ring with one another is, for the purposes of the present application, intended to be taken to mean, inter alia, that the two radicals are linked to one another by a chemical bond. This is illustrated by the following schemes:

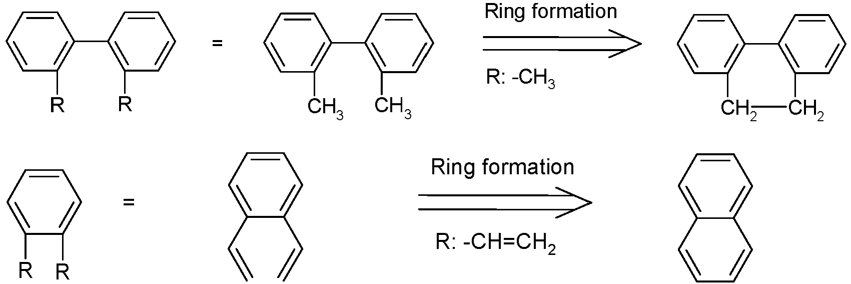

Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

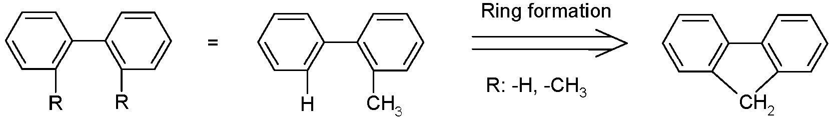

Suitable aromatic or heteroaromatic ring systems for Ar¹ are selected from the group consisting of benzene, naphthalene, anthracene, phenanthrene, biphenyl, terphenyl, quaterphenyl, fluorene, spirobifluorene, cis- or trans-indenofluorene, truxene, benzofuran, dibenzofuran, benzothiophene, dibenzothiophene, carbazole, indolocarbazole, indenocarbazole, pyridine, pyrimidine, triazine, quinoline, acridine, phenanthridine, benzoquinoline, phenothiazine, phenoxazine, benzopyrimidine, quinoxaline, pyrazine, phenazine, each of which may be substituted by one or more radicals R¹.

Very suitable aromatic or heteroaromatic ring systems for Ar¹ are the groups of the following formulae (A-1) to (A-54), 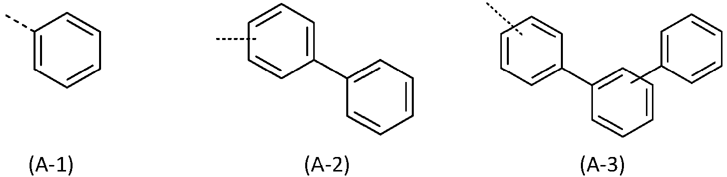 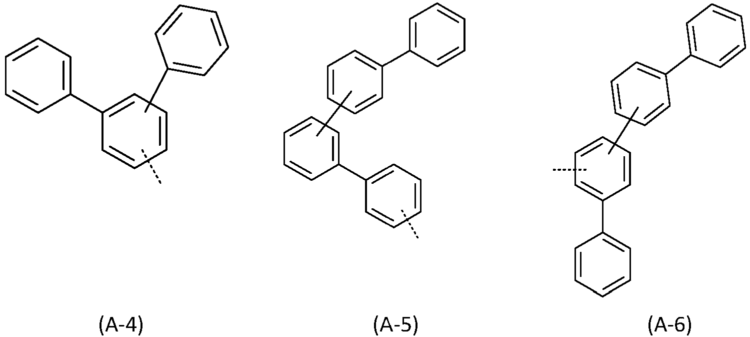 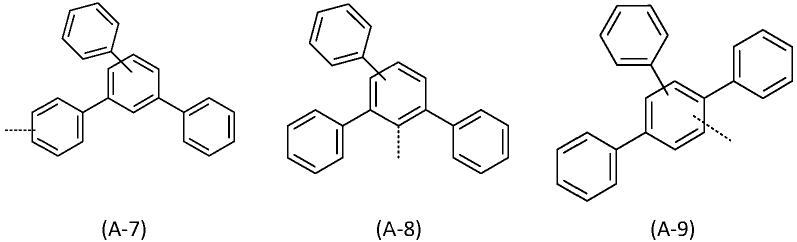 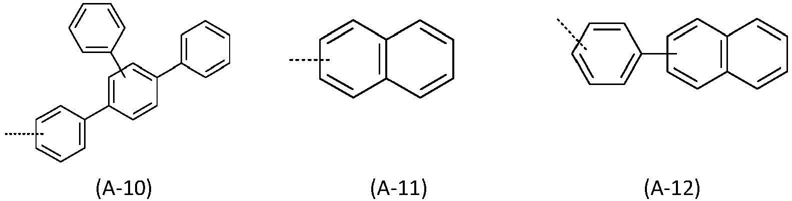 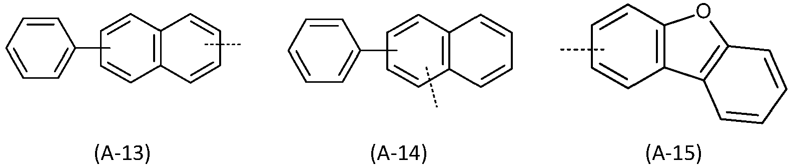 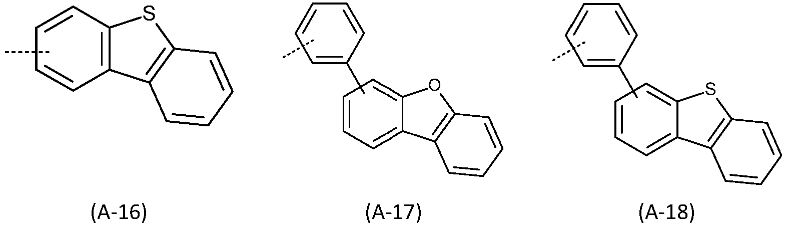 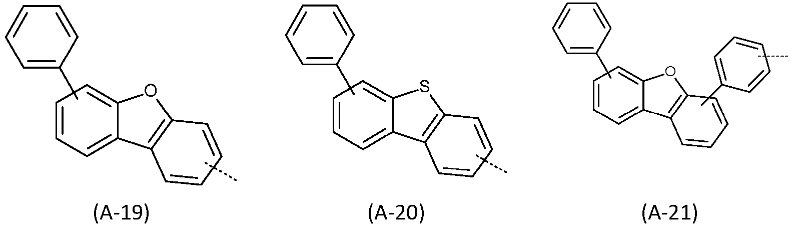 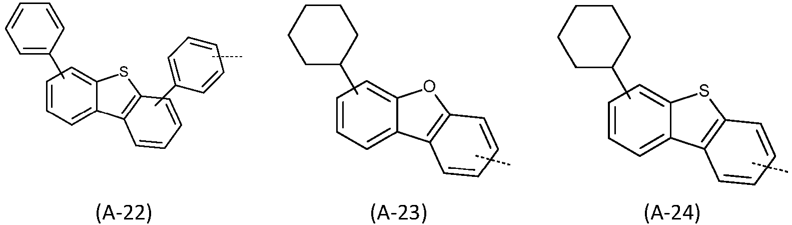 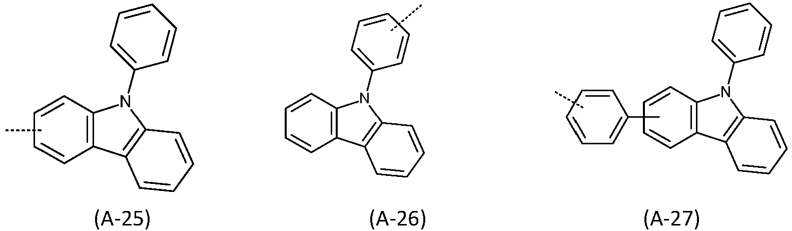 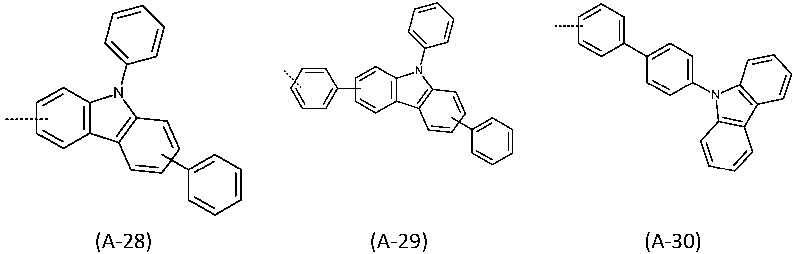 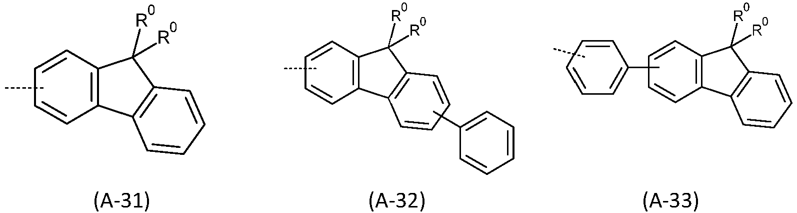 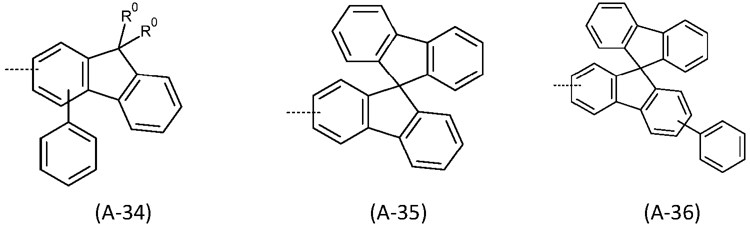 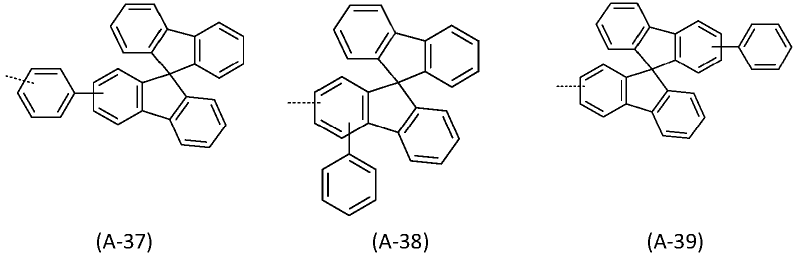 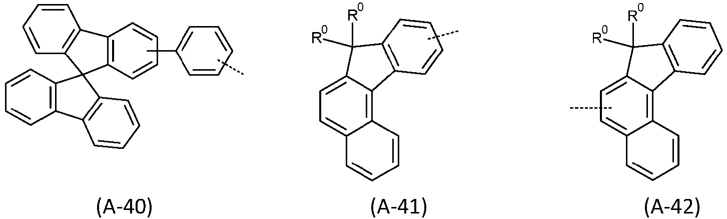 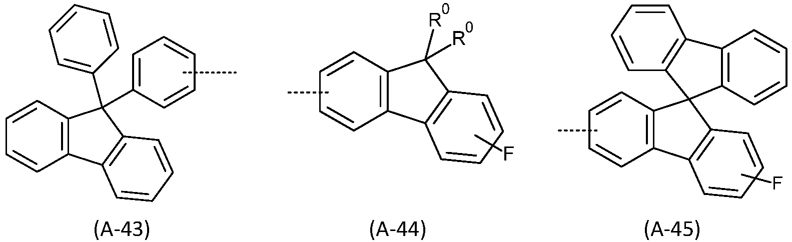 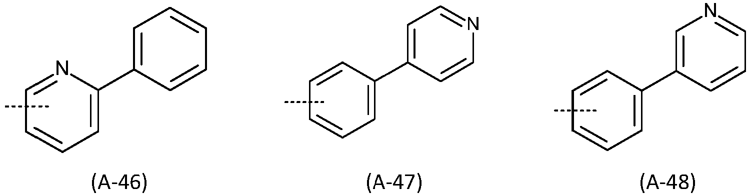 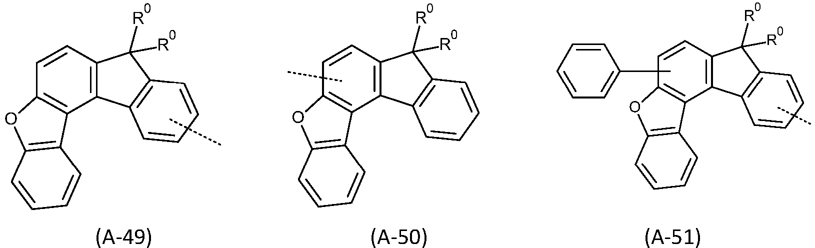 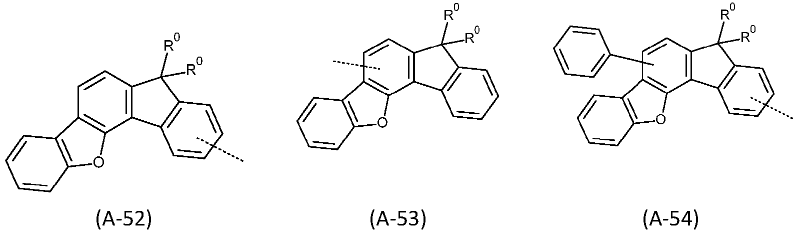
where the dashed bond in formulae (A-1) to (A-54) indicates the bond to the nitrogen atom of the group -NAr¹,
where the groups of formulae (A-1) to (A-54) may further be substituted at each free position by a group R¹ as defined above and where the group R⁰, in formulae (A-31) to (A-34), (A-41), (A-42), (A-44) and (A-49) to (A-54), has the same definition as above.

Particularly suitable aromatic or heteroaromatic ring systems for Ar¹ are selected from aromatic or heteroaromatic ring systems of the formulae (A-1) to (A-54), are the groups (Ar-1) to (Ar-202), 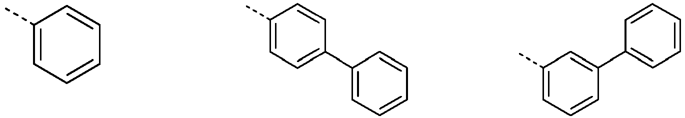 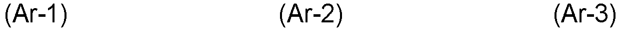 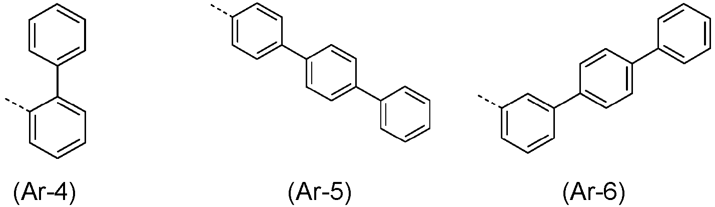 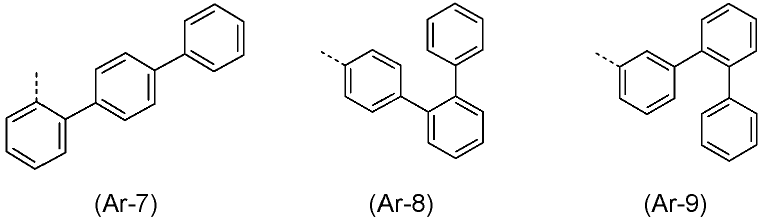 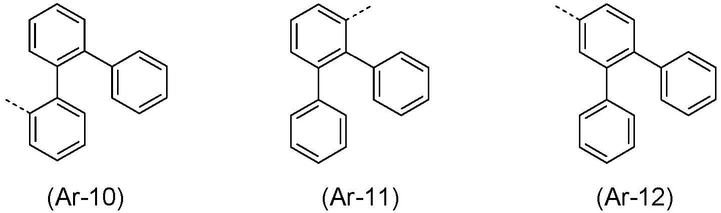 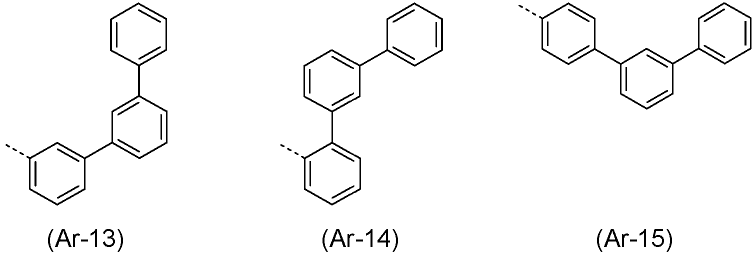 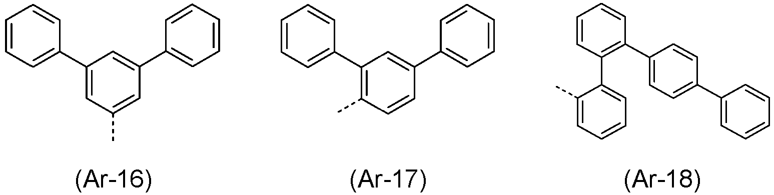 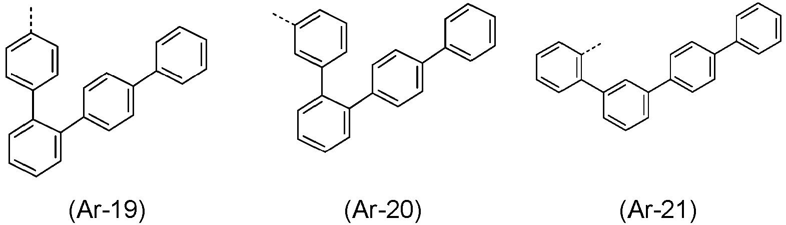 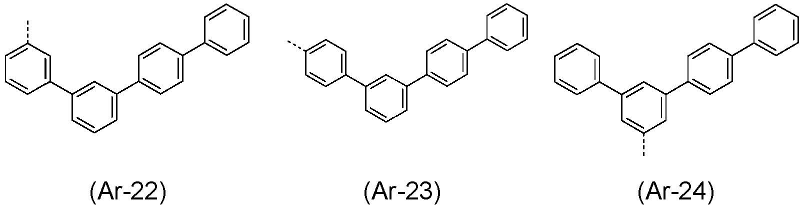 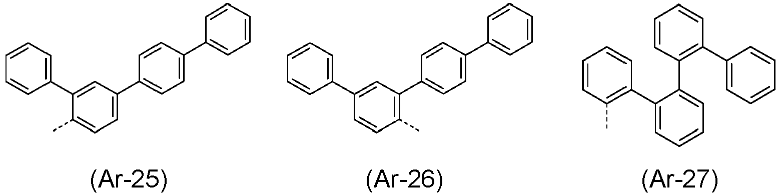 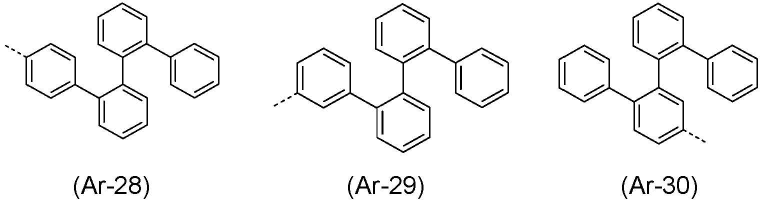 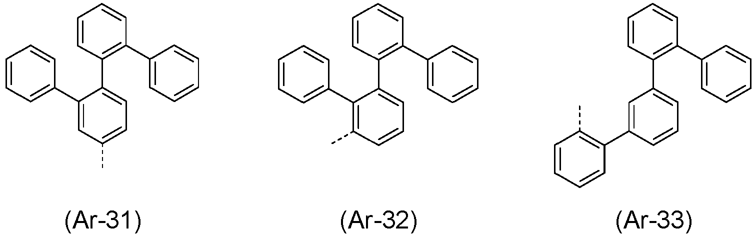 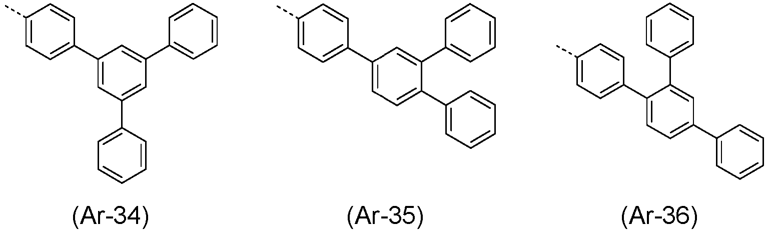 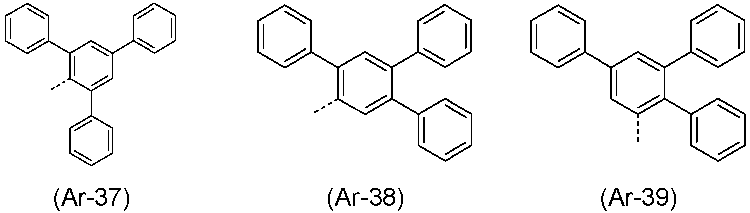 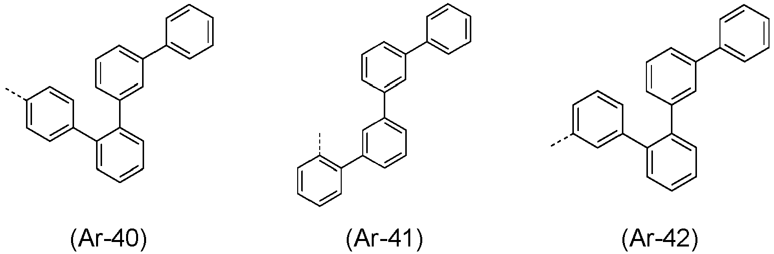 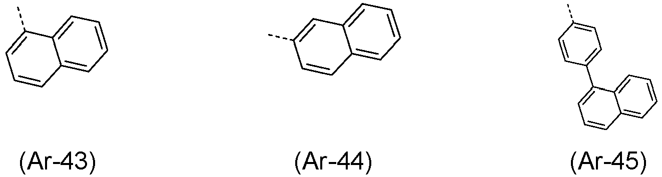 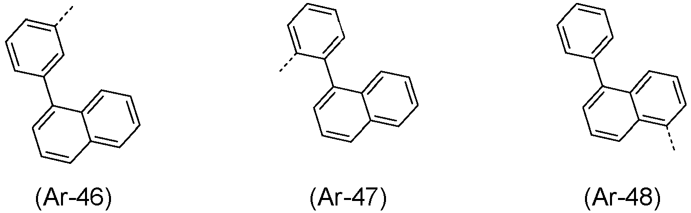 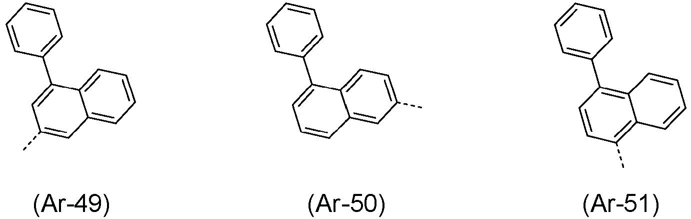 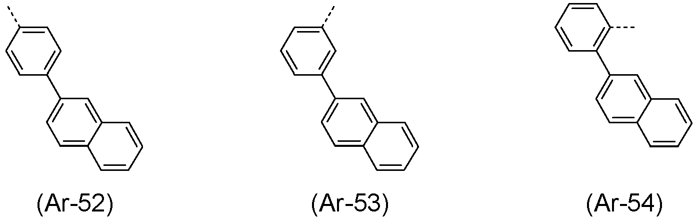 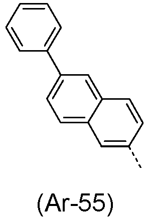 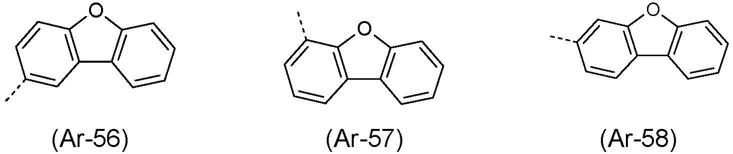 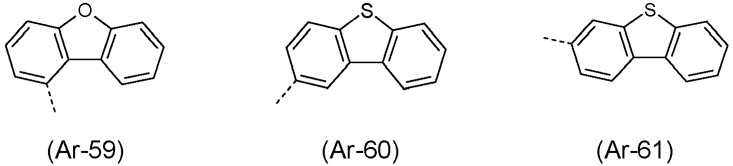 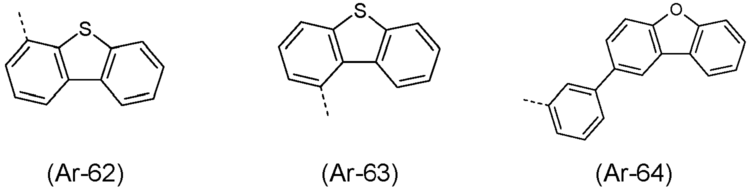 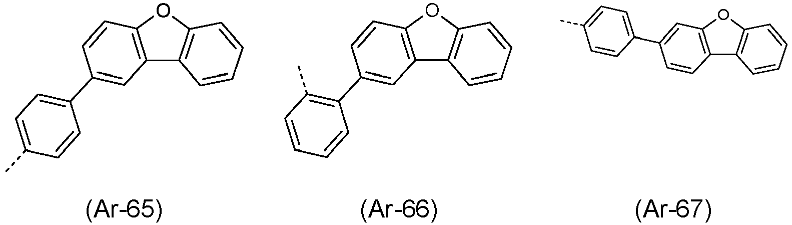 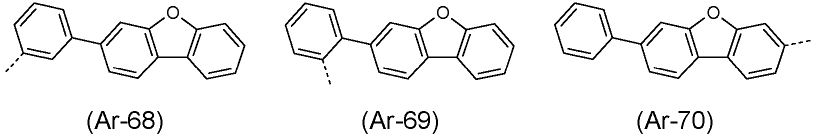 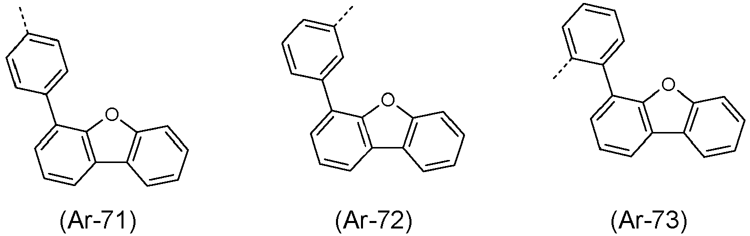 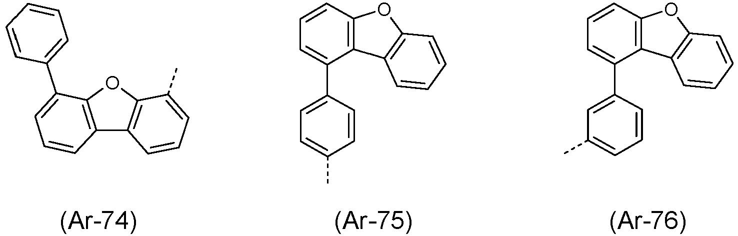 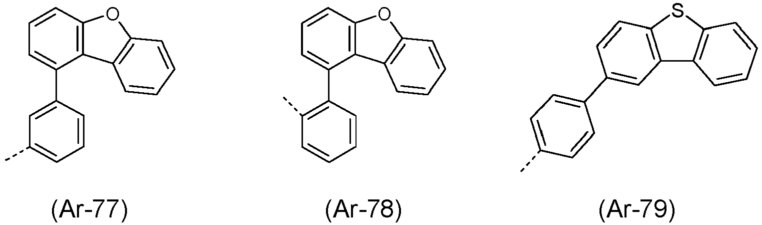 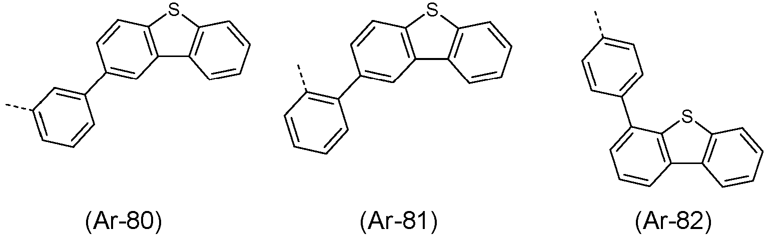 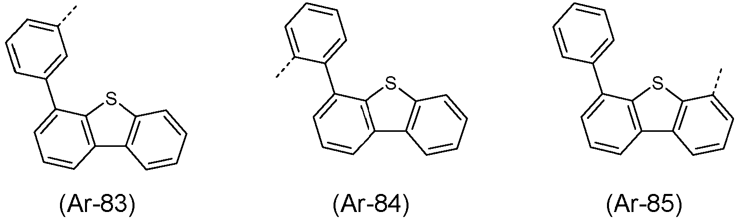 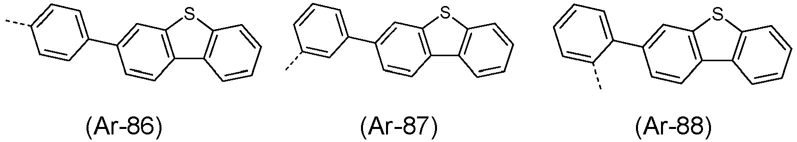 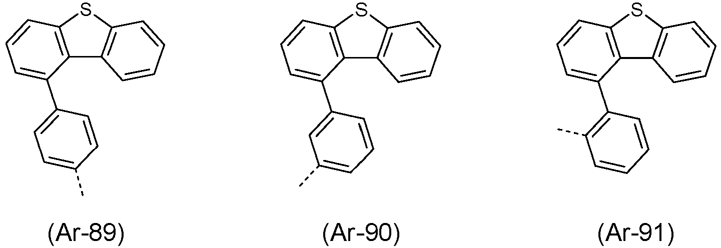 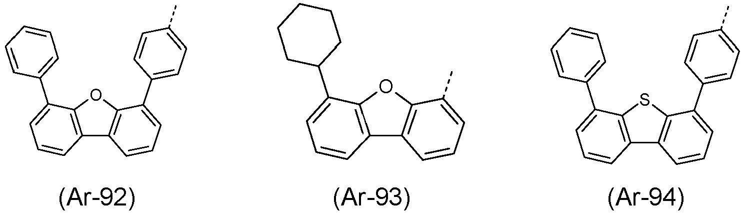 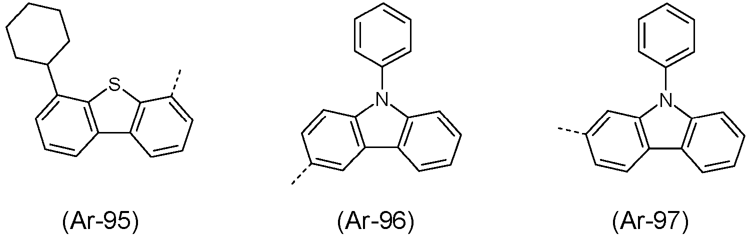 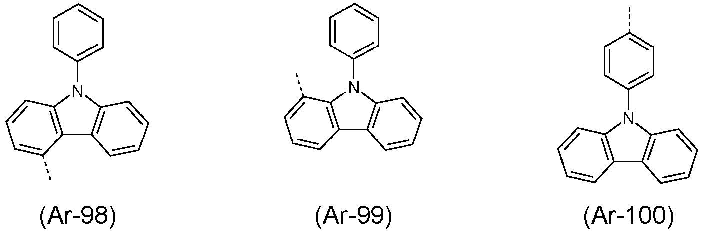 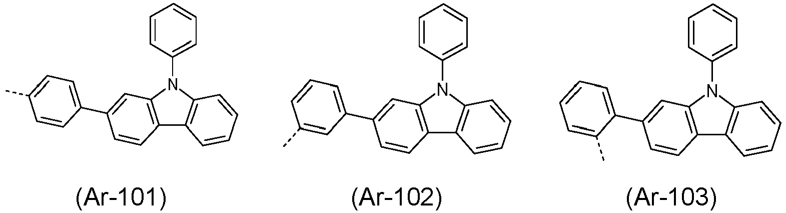 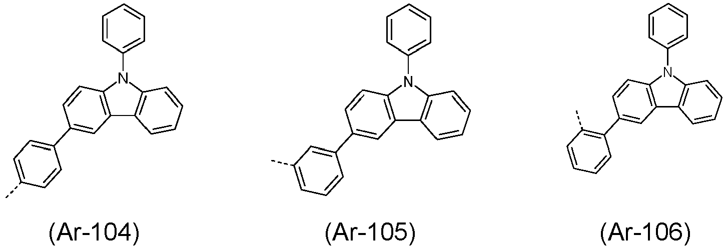 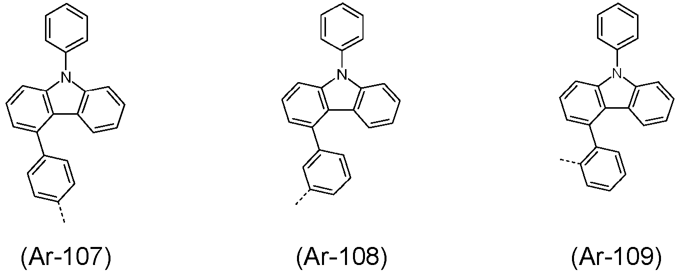 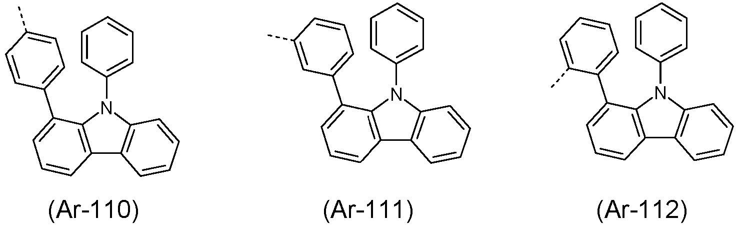 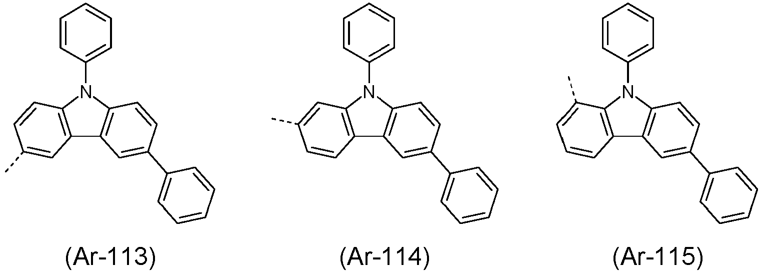 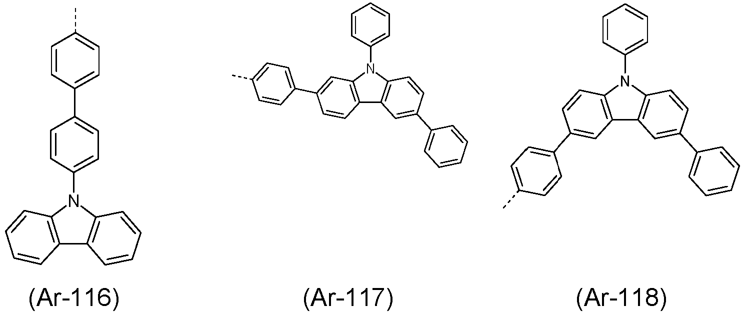 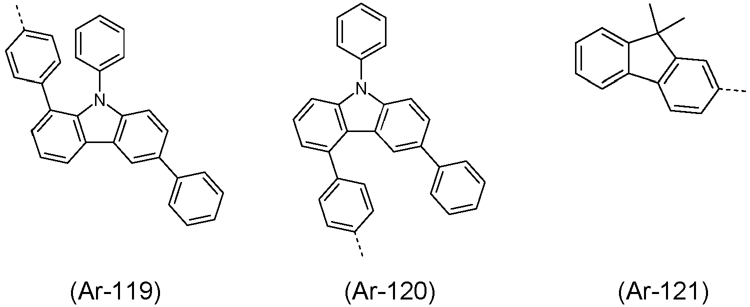 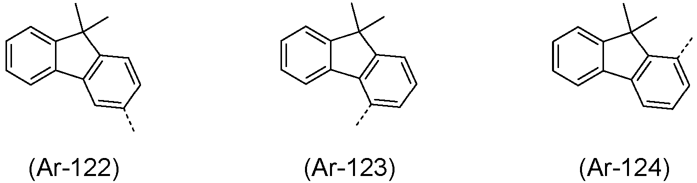 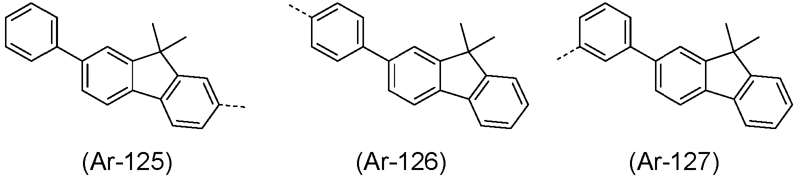 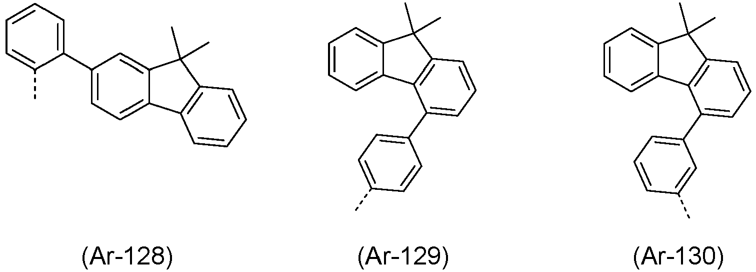 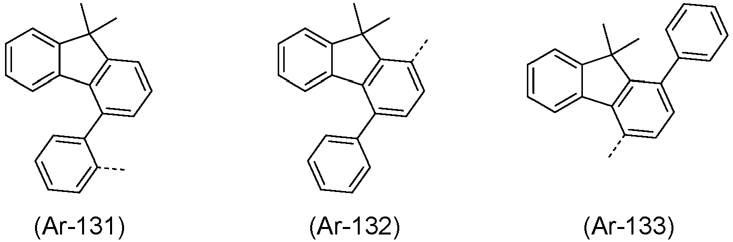 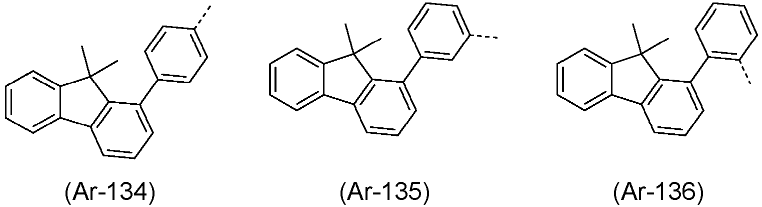 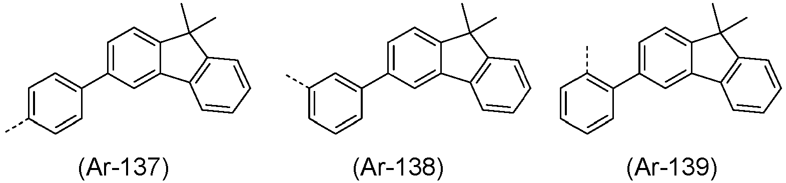 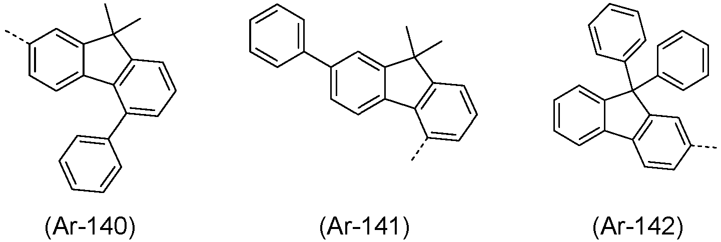 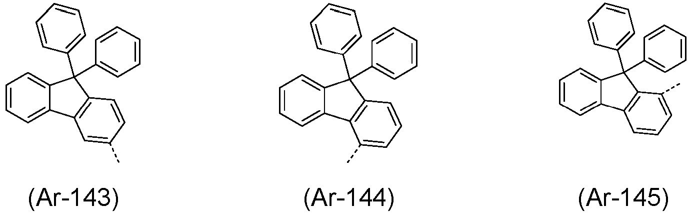 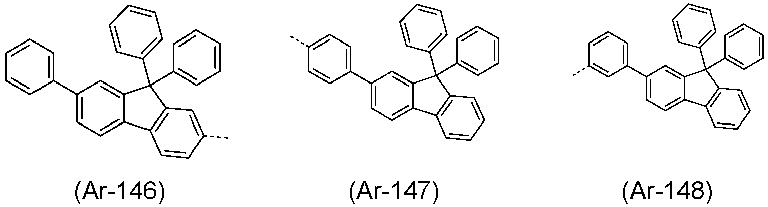 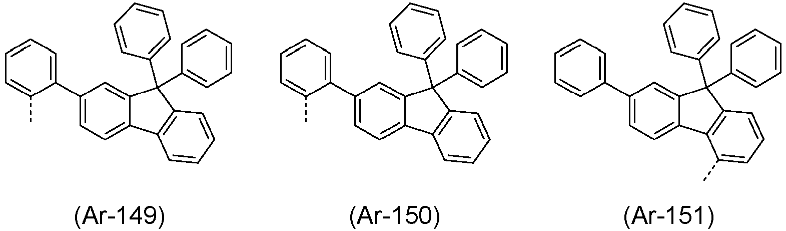 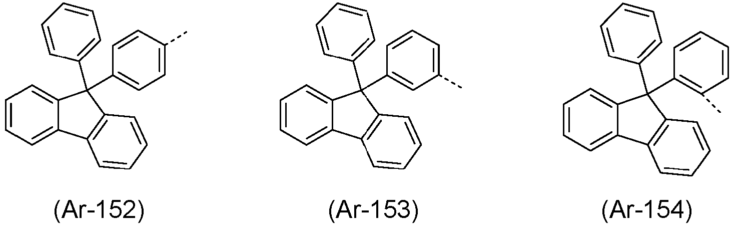 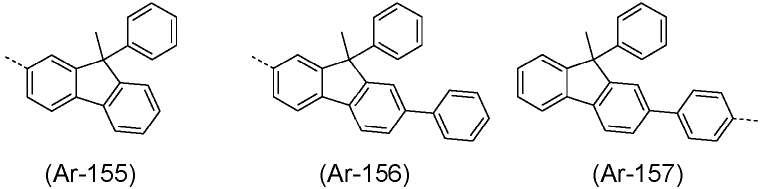 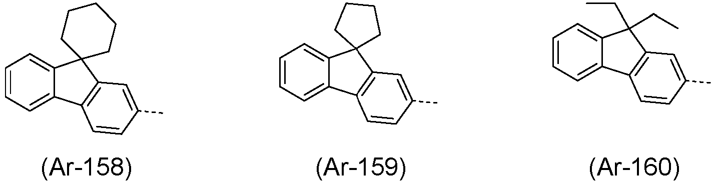 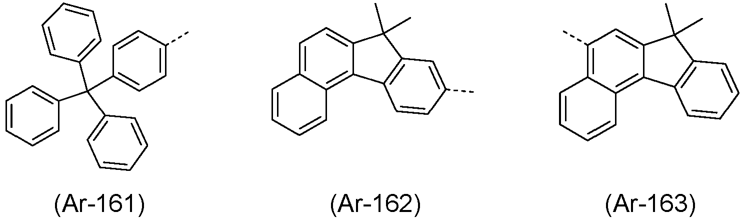 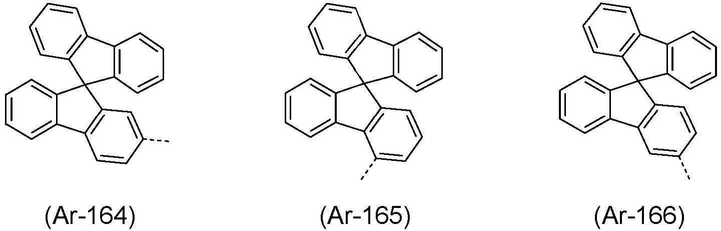 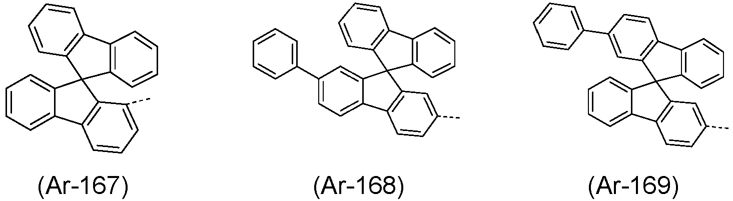 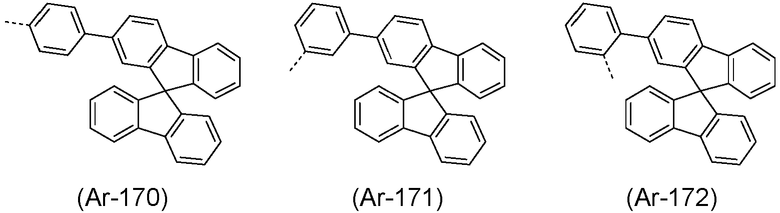 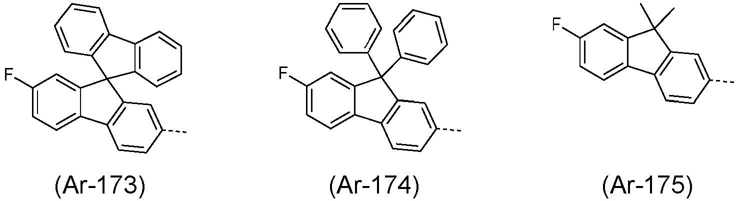 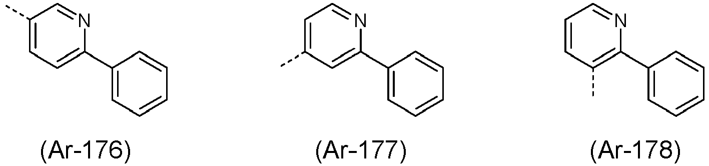 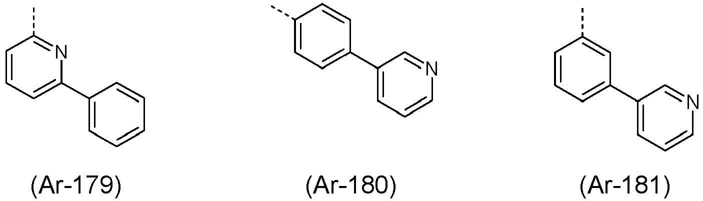 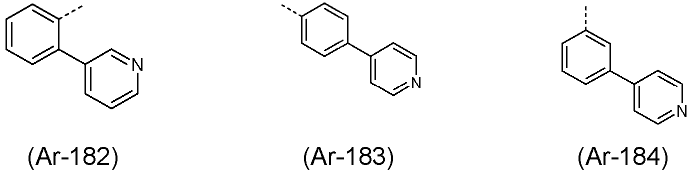 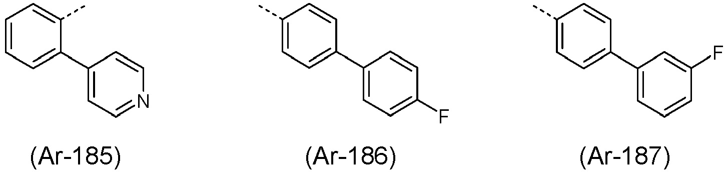 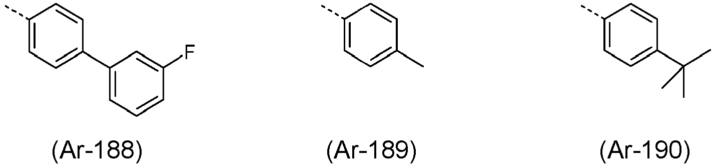 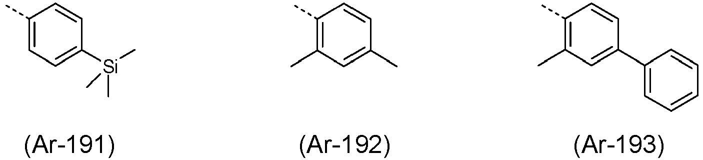 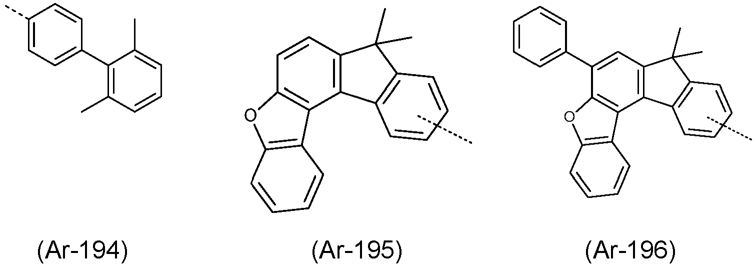 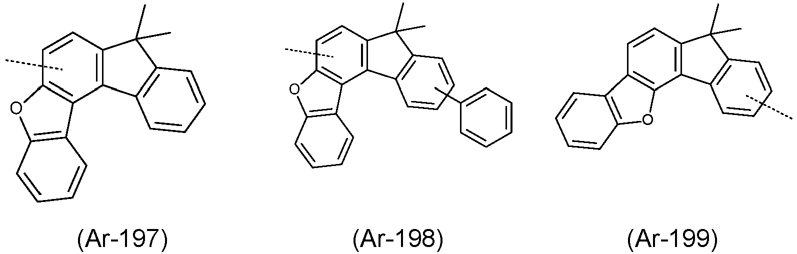 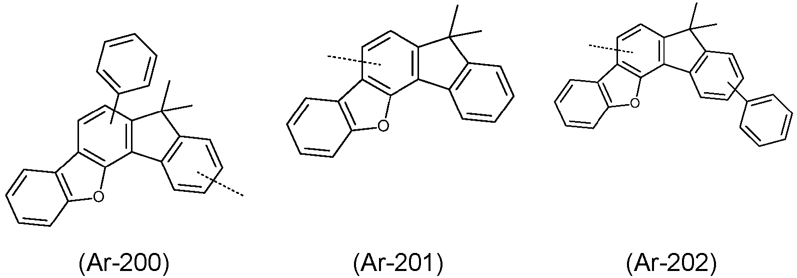
where the dashed bond in formulae (Ar-1) to (Ar-202) indicates the bond to the nitrogen atom of the group -NAr¹, and
where the groups of formulae (Ar-1) to (Ar-202) may further be substituted at each free position by a group R¹ as defined above.

In accordance with the invention, X stands for CR² or N; or X stands for C, if it is bonded to the nitrogen atom as depicted in formulae (3A), (3B) or (3C). Preferably, there is 0, 1, 2 or 3 groups X which stand for N in each 6-membered ring comprising the groups X. Very preferably, X stands for CR² or for C, if it is bonded the nitrogen atom as depicted in formulae (3A), (3B) or (3C).

In accordance with a preferred embodiment, the inventive compounds are selected from the compounds of formulae (3A-1) to (3C-1), 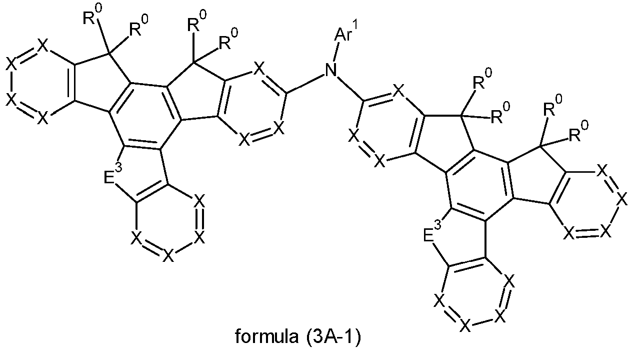 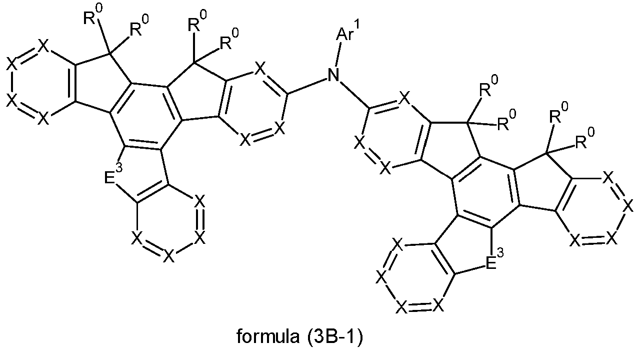 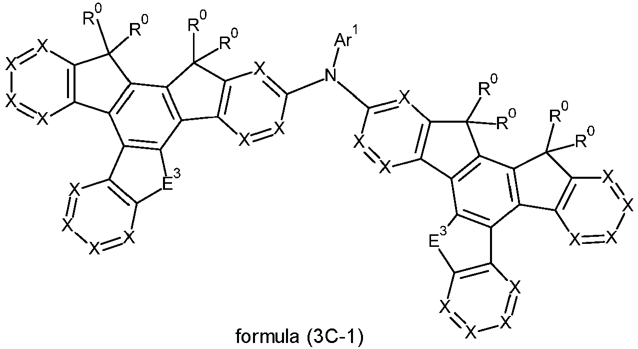 where the symbol E³ stands for -O-, and where the symbols X, R⁰ and Ar¹ have the same meaning as above.

The following compounds are examples of compounds of formulae (3A), (3B) or (3C):

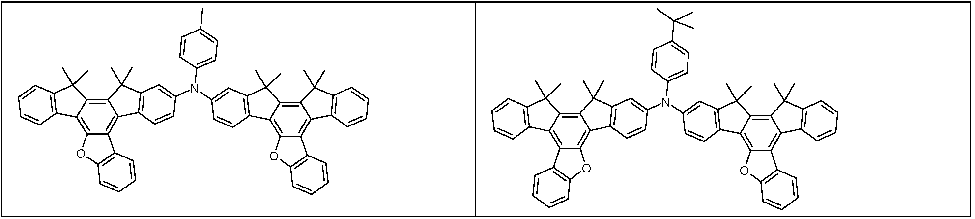

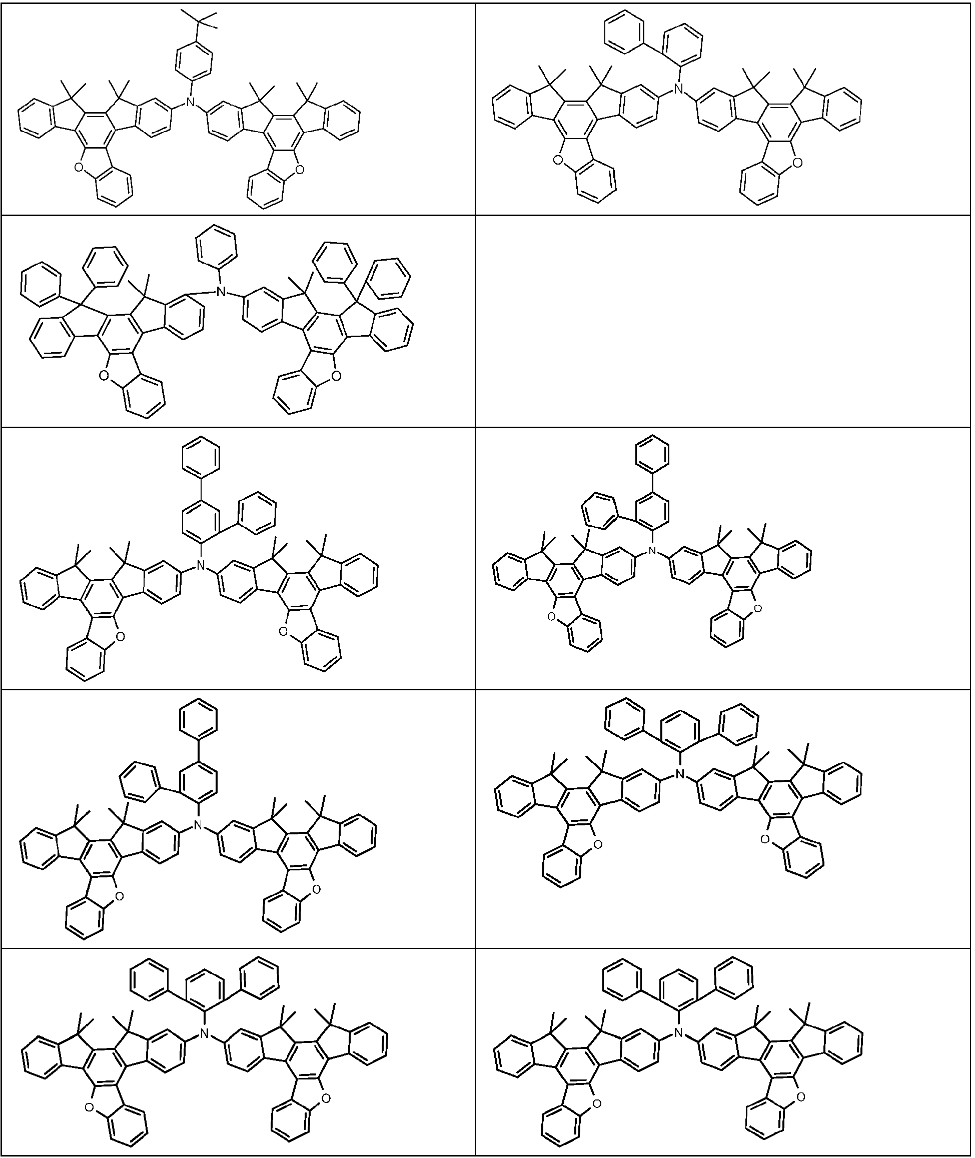

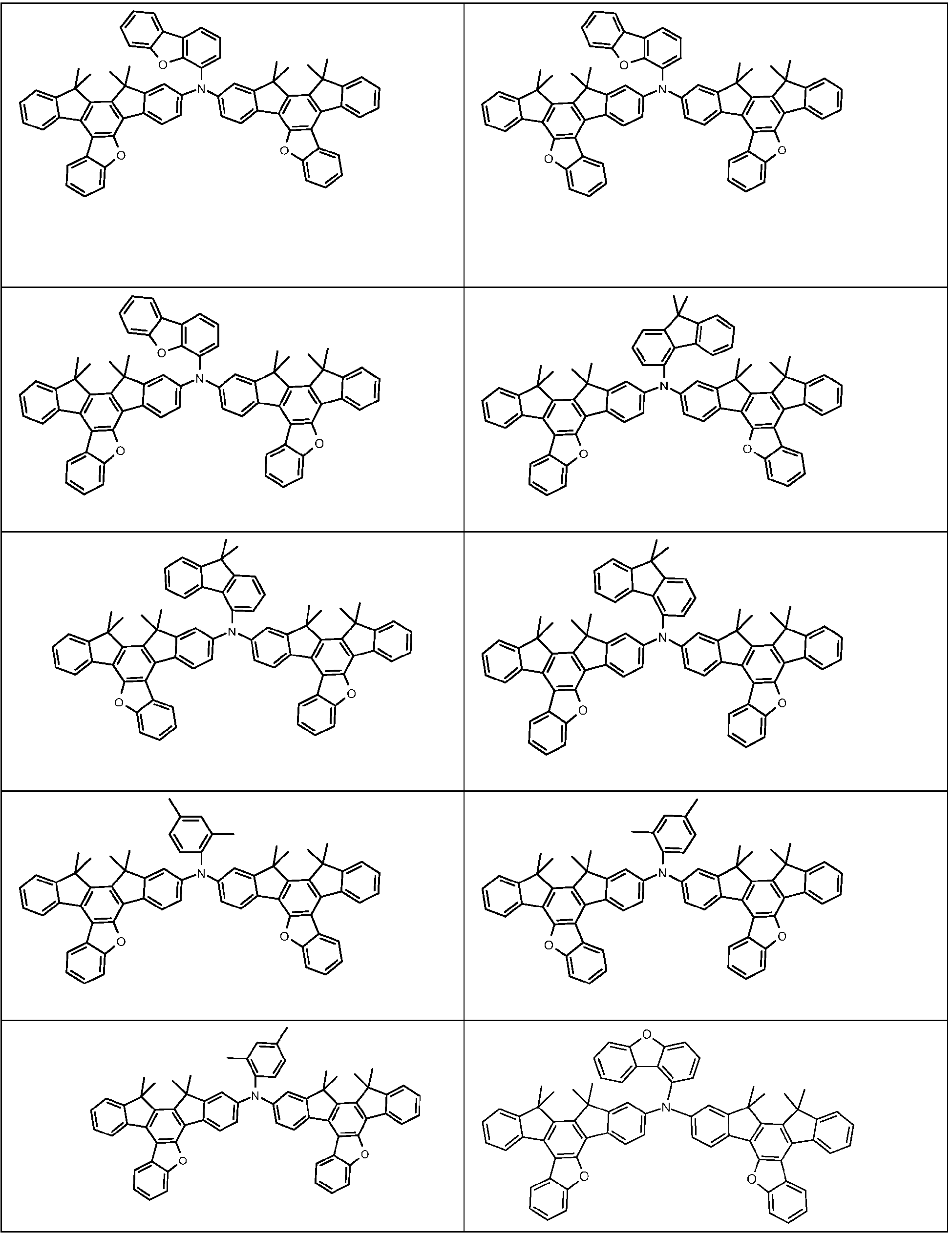

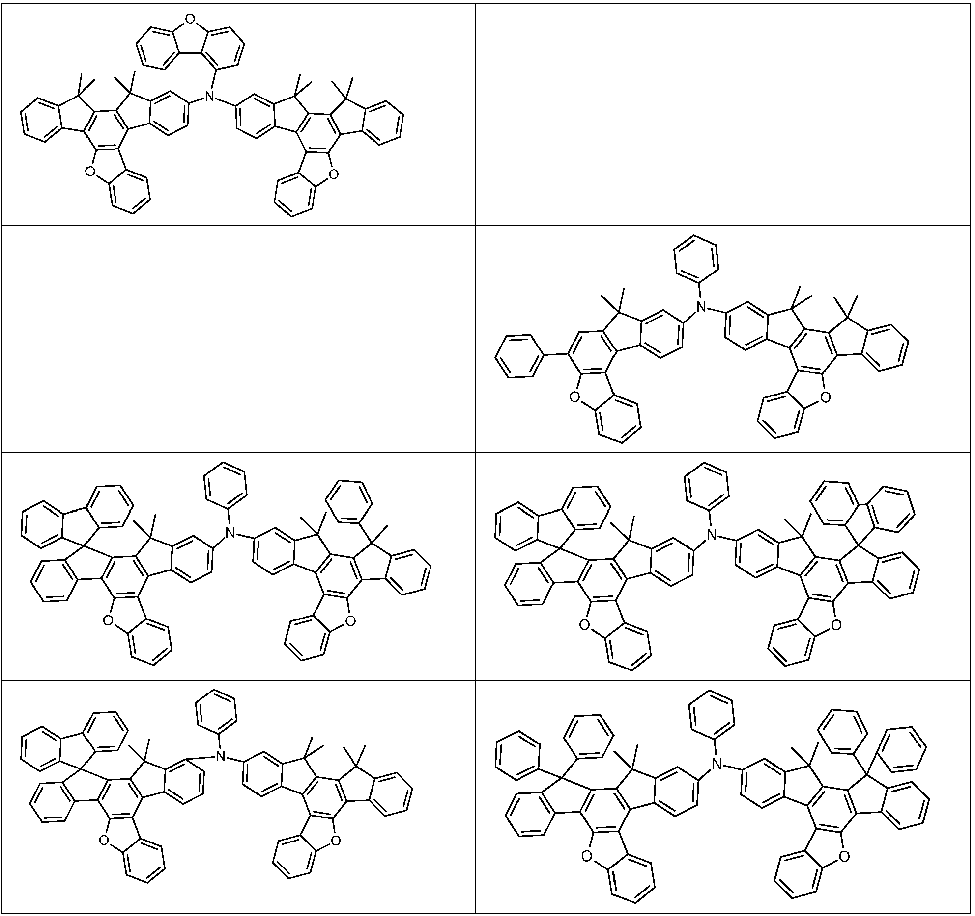

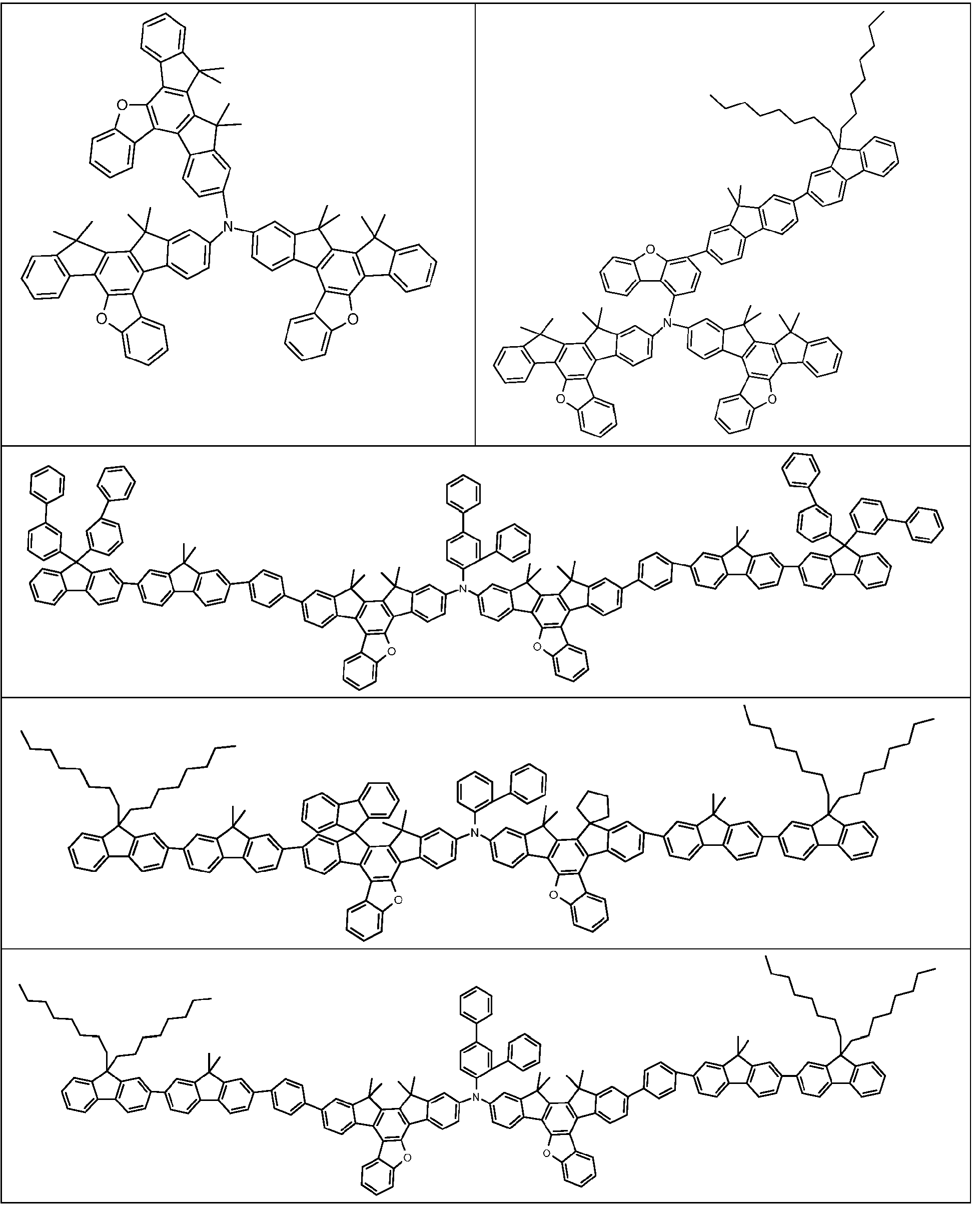

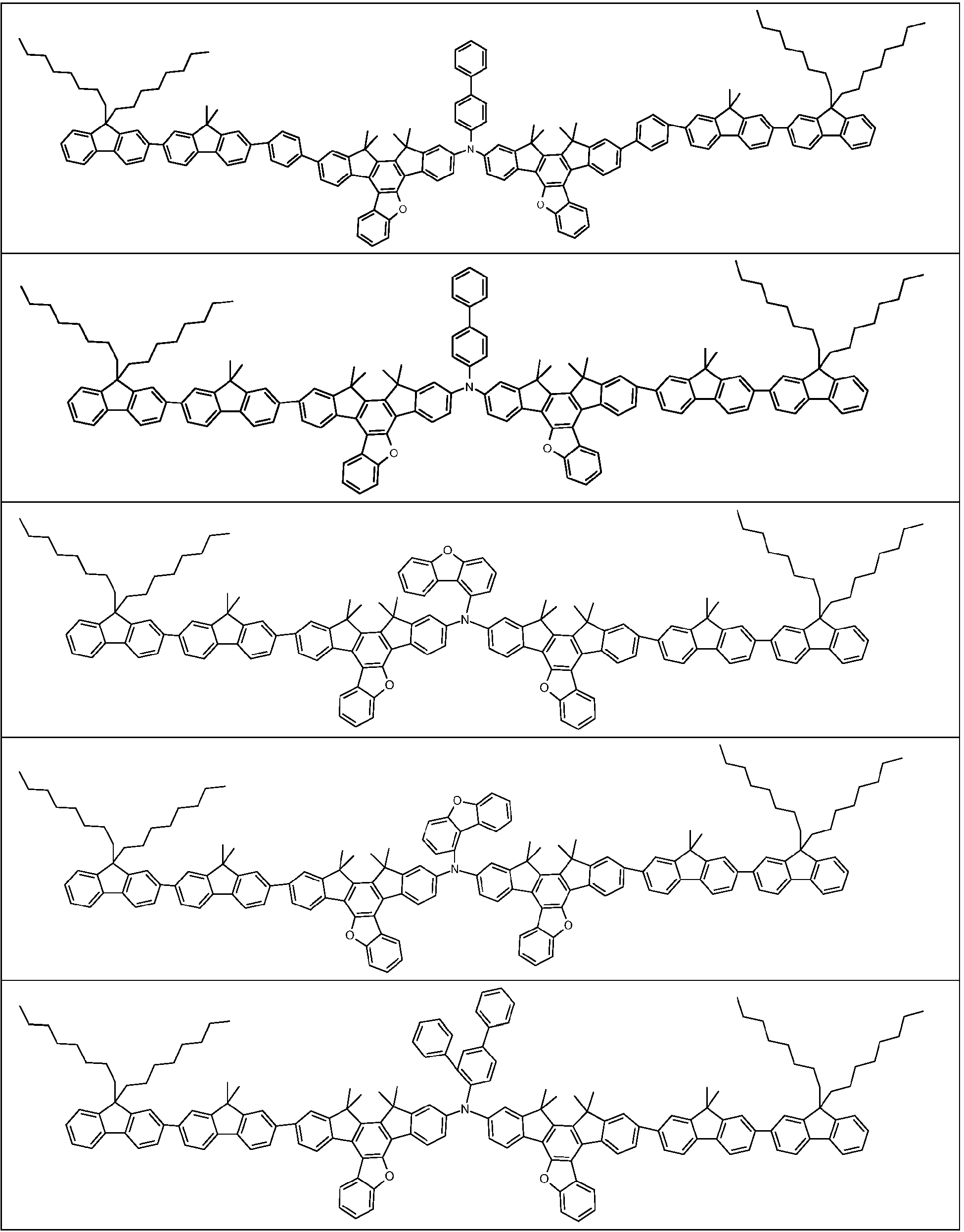

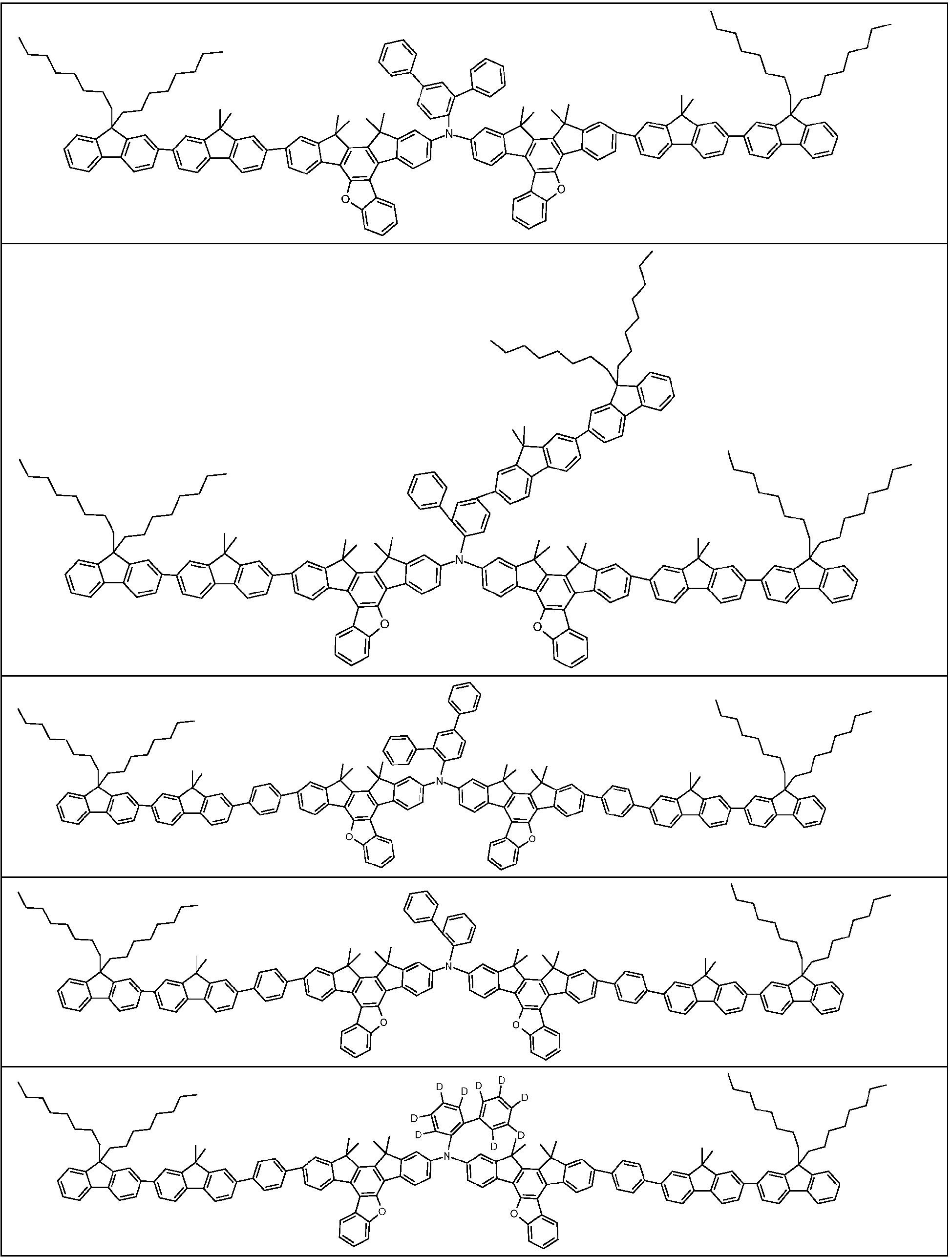

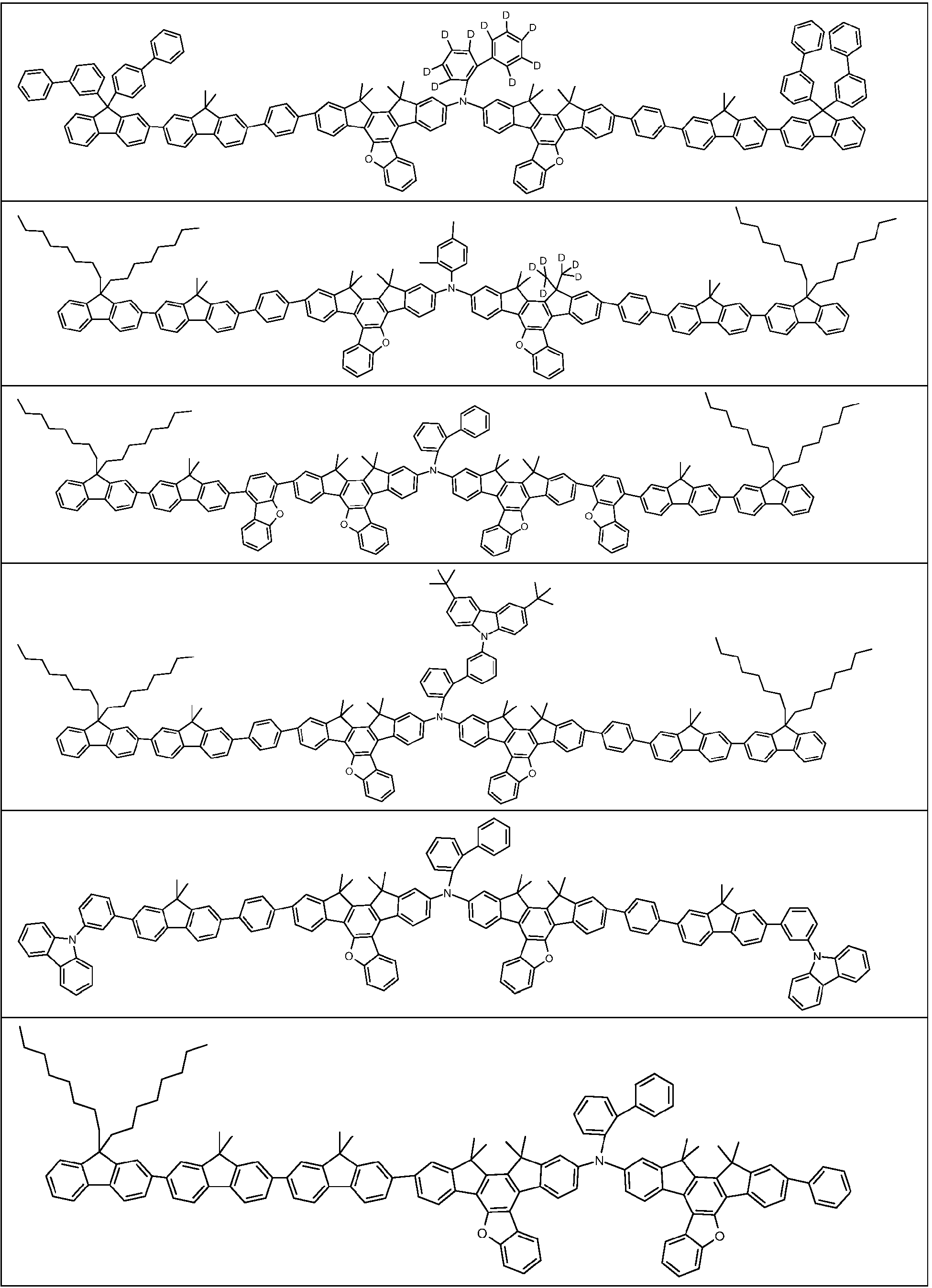

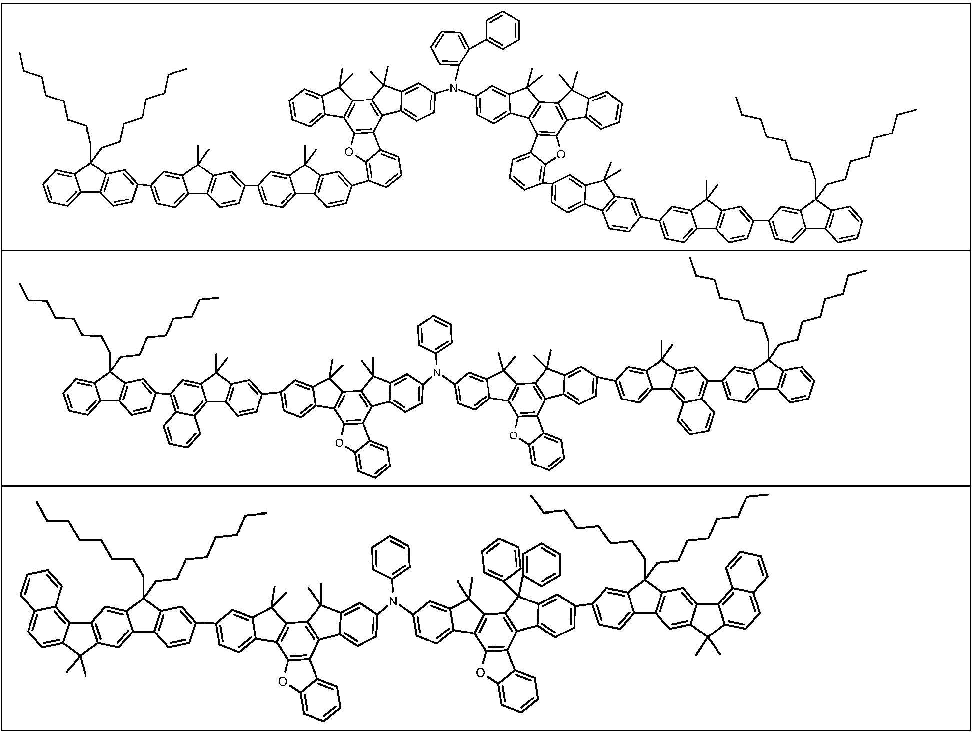

The compounds according to the invention can be prepared by synthesis steps known to the person skilled in the art, such as, for example, bromination, Suzuki coupling, Ullmann coupling, Hartwig-Buchwald coupling, etc. An example of a suitable synthesis process is depicted in general terms in Schemes 1 to 3 below. 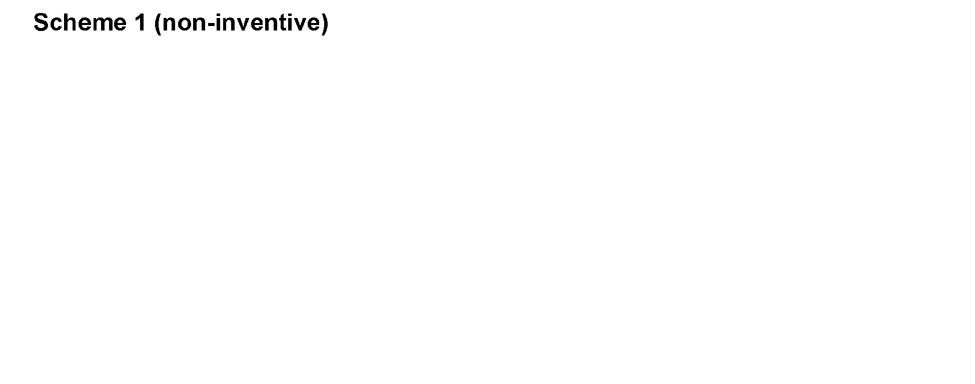 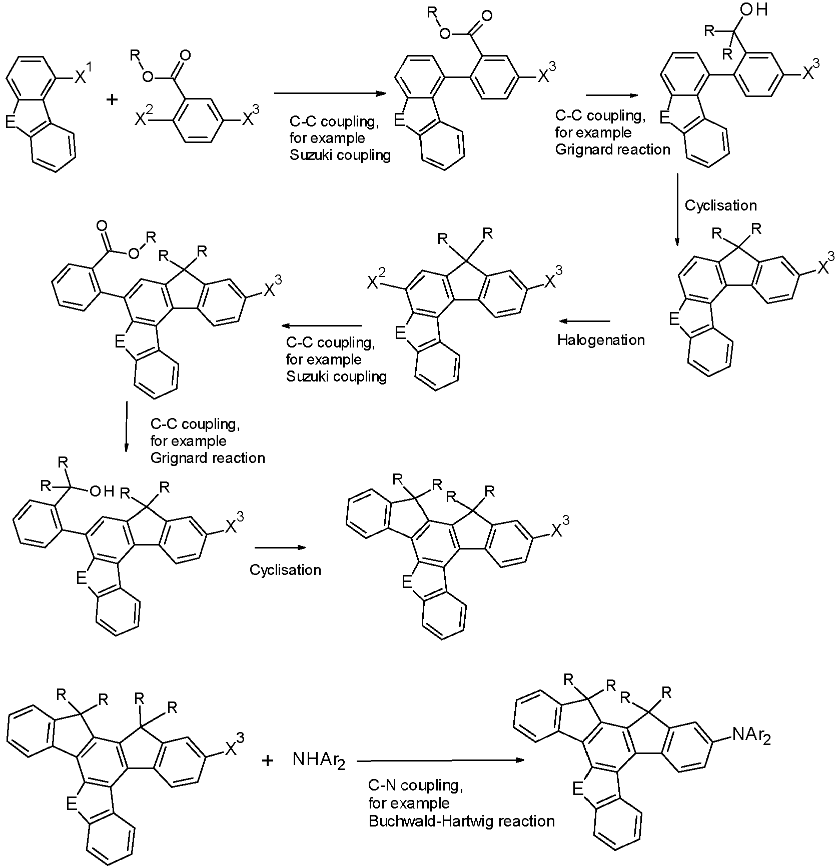
E is O or S or C(R)₂, where R is a substituent
X¹ and X³ are leaving groups (like halogens or boronic acid derivatives)
Ar is an aromatic or heteroaromatic ring system (identical or differenton each occurence)

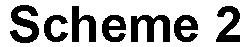

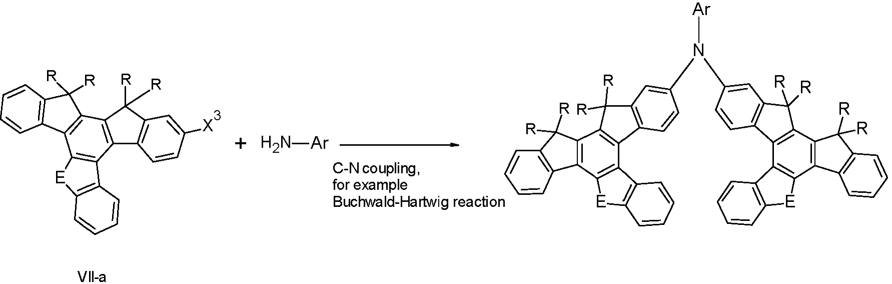

E is O or S or C(R)₂, where R is a substituent
X¹ and X³ are leaving groups (like halogens or boronic acid derivatives)
Ar is an aromatic or heteroaromatic ring system (identical or differenton each occurence)

The compounds of formulae (3A), (3B) or (3C) may be synthesized as described above, where a truxene derivative comprising a divalent bridge -O- is first synthesized using as a precursor a dibenzofuran or dibenzothiophene derivative comprising a reactive leaving group (like halogen or boronic acid) and at least one arylamine is bonded to the truxene derivative via a C-N coupling reaction.

For the processing of the compounds according to the invention from the liquid phase, for example by spin coating or by printing processes, formulations of the compounds according to the invention are necessary. These formulations can be, for example, solutions, dispersions or emulsions. It may be preferred to use mixtures of two or more solvents for this purpose. Suitable and preferred solvents are, for example, toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol - monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The present invention therefore furthermore relates to a formulation comprising a compound according to the invention and at least one further compound. The further compound may be, for example, a solvent, in particular one of the above-mentioned solvents or a mixture of these solvents. However, the further compound may also be at least one further organic or inorganic compound which is likewise employed in the electronic device, for example a matrix material, in particular a matrix material for a fluorescent dopant and/or a further emitting compound. Suitable matrix materials and further emitting compounds are indicated below in connection with the organic electroluminescent device. This further compound may also be polymeric.

The compounds and mixtures according to the invention are suitable for use in an electronic device. An electronic device here is taken to mean a device which comprises at least one layer which comprises at least one organic compound. However, the component here may also comprise inorganic materials or also layers built up entirely from inorganic materials.

The present invention therefore furthermore relates to the use of the compounds or mixtures according to the invention in an electronic device, in particular in an organic electroluminescent device.

The present invention again furthermore relates to an electronic device comprising at least one of the compounds or mixtures according to the invention mentioned above. The preferences stated above for the compound also apply to the electronic devices.

The electronic device is preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells, organic optical detectors, organic photoreceptors, organic field-quench devices (O-FQDs), light-emitting electrochemical cells (LECs), organic laser diodes (O-lasers) and "organic plasmon emitting devices" (D. M. Koller et al., Nature Photonics 2008, 1-4), preferably organic electroluminescent devices (OLEDs, PLEDs), in particular phosphorescent OLEDs.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. It is likewise possible for interlayers, which have, for example, an exciton-blocking function, to be introduced between two emitting layers. However, it should be pointed out that each of these layers does not necessarily have to be present. The organic electroluminescent device here may comprise one emitting layer or a plurality of emitting layers. If a plurality of emission layers is present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). These can be fluorescent or phosphorescent emission layers or hybrid systems, in which fluorescent and phosphorescent emission layers are combined with one another.

The compound according to the invention in accordance with the embodiments indicated above can be employed in various layers, depending on the precise structure and on the substitution. Preference is given to an organic electroluminescent device comprising a compound of the formulae (3A), (3B) or (3C) or in accordance with the preferred embodiments as fluorescent emitters, emitters showing TADF (Thermally Activated Delayed Fluorescence), matrix material for fluorescent emitters. Particularly preferred is an organic electroluminescent device comprising a compound of the formulae (3A), (3B) or (3C) or in accordance with the preferred embodiments as fluorescent emitters, more particularly blue-emitting fluorescent compounds.

The compounds of formulae (3A), (3B) or (3C) can also be employed in an electron-transport layer and/or in an electron-blocking or exciton-blocking layer and/or in a hole-transport layer, depending on the precise substitution. The preferred embodiments indicated above also apply to the use of the materials in organic electronic devices.

The compound according to the invention is particularly suitable for use as blue-emitting emitter compound. The electronic device concerned may comprise a single emitting layer comprising the compound according to the invention or it may comprise two or more emitting layers. The further emitting layers here may comprise one or more compounds according to the invention or alternatively other compounds.

If the compound according to the invention is employed as a fluorescent emitting compound in an emitting layer, it is preferably employed in combination with one or more matrix materials. A matrix material here is taken to mean a material which is present in the emitting layer, preferably as the principal component, and which does not emit light on operation of the device.

The proportion of the emitting compound in the mixture of the emitting layer is between 0.1 and 50.0%, preferably between 0.5 and 20.0%, particularly preferably between 1.0 and 10.0%. Correspondingly, the proportion of the matrix material or matrix materials is between 50.0 and 99.9%, preferably between 80.0 and 99.5%, particularly preferably between 90.0 and 99.0%.

The specifications of the proportions in % are, for the purposes of the present application, taken to mean % by vol. if the compounds are applied from the gas phase and % by weight if the compounds are applied from solution.

Preferred matrix materials for use in combination with fluorescent emitting compounds are selected from the classes of the oligoarylenes (for example 2,2',7,7'-tetraphenylspirobifluorene in accordance with EP 676461 or dinaphthylanthracene), in particular the oligoarylenes containing condensed aromatic groups, the oligoarylenevinylenes (for example DPVBi or spiro-DPVBi in accordance with EP 676461), the polypodal metal complexes (for example in accordance with WO 2004/081017), the hole-conducting compounds (for example in accordance with WO 2004/058911), the electron-conducting compounds, in particular ketones, phosphine oxides, sulfoxides, etc. (for example in accordance with WO 2005/084081 and WO 2005/084082), the atropisomers (for example in accordance with WO 2006/048268), the boronic acid derivatives (for example in accordance with WO 2006/117052) or the benzanthracenes (for example in accordance with WO 2008/145239). Particularly preferred matrix materials are selected from the classes of the oligoarylenes, comprising naphthalene, anthracene, benzanthracene and/or pyrene or atropisomers of these compounds, the oligoarylenevinylenes, the ketones, the phosphine oxides and the sulfoxides. Very particularly preferred matrix materials are selected from the classes of the oligoarylenes, comprising anthracene, benzanthracene, benzophenanthrene and/or pyrene or atropisomers of these compounds. An oligoarylene in the sense of this invention is intended to be taken to mean a compound in which at least three aryl or arylene groups are bonded to one another.

Particularly preferred matrix materials for use in combination with the compounds of the formulae (3A), (3B) or (3C) in the emitting layer are depicted in the following table.

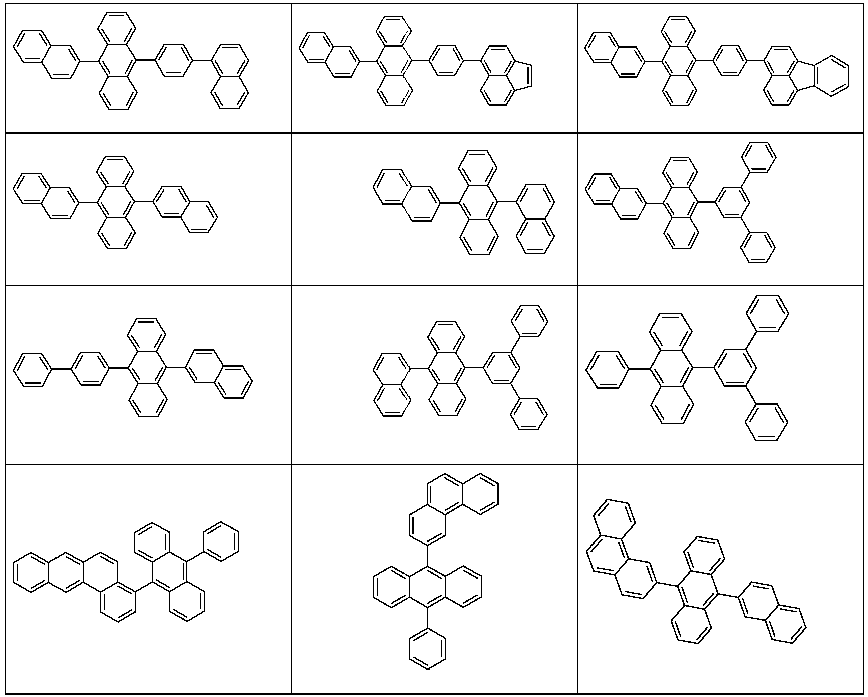

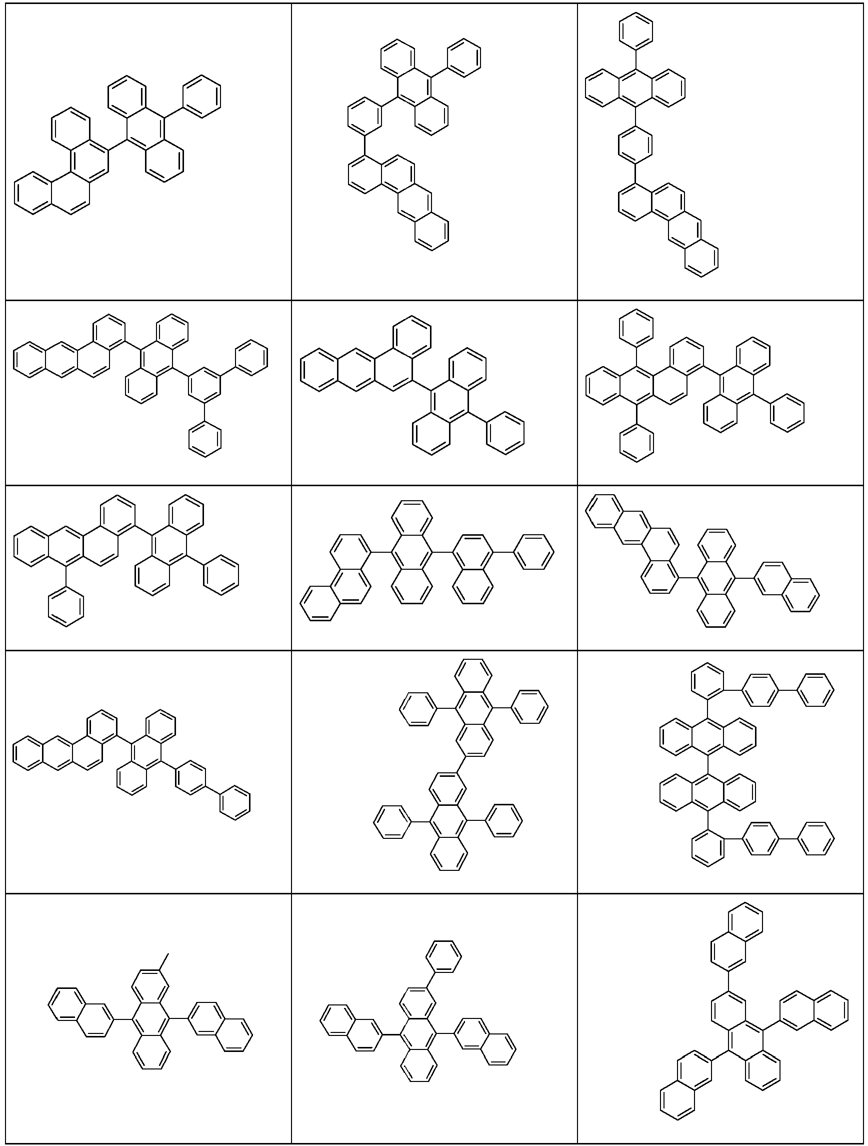

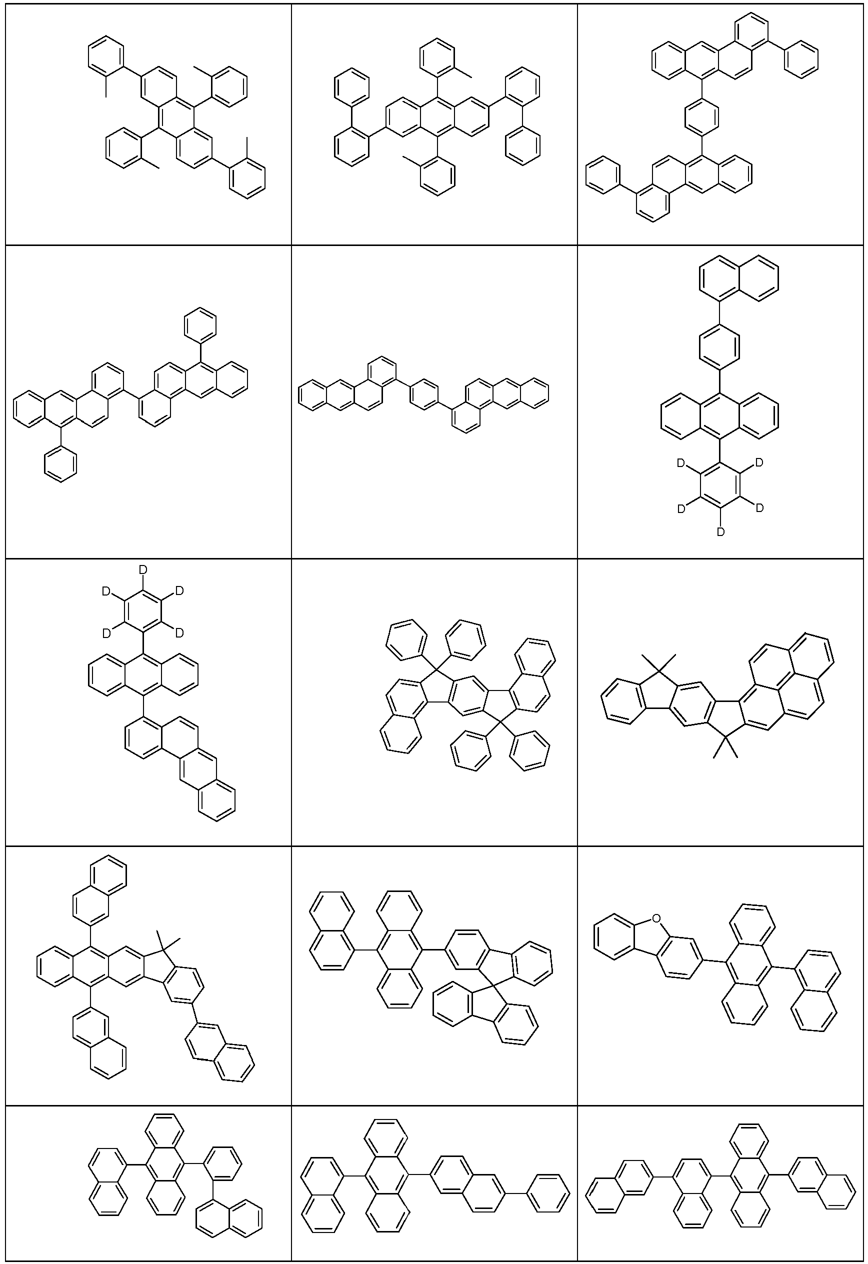

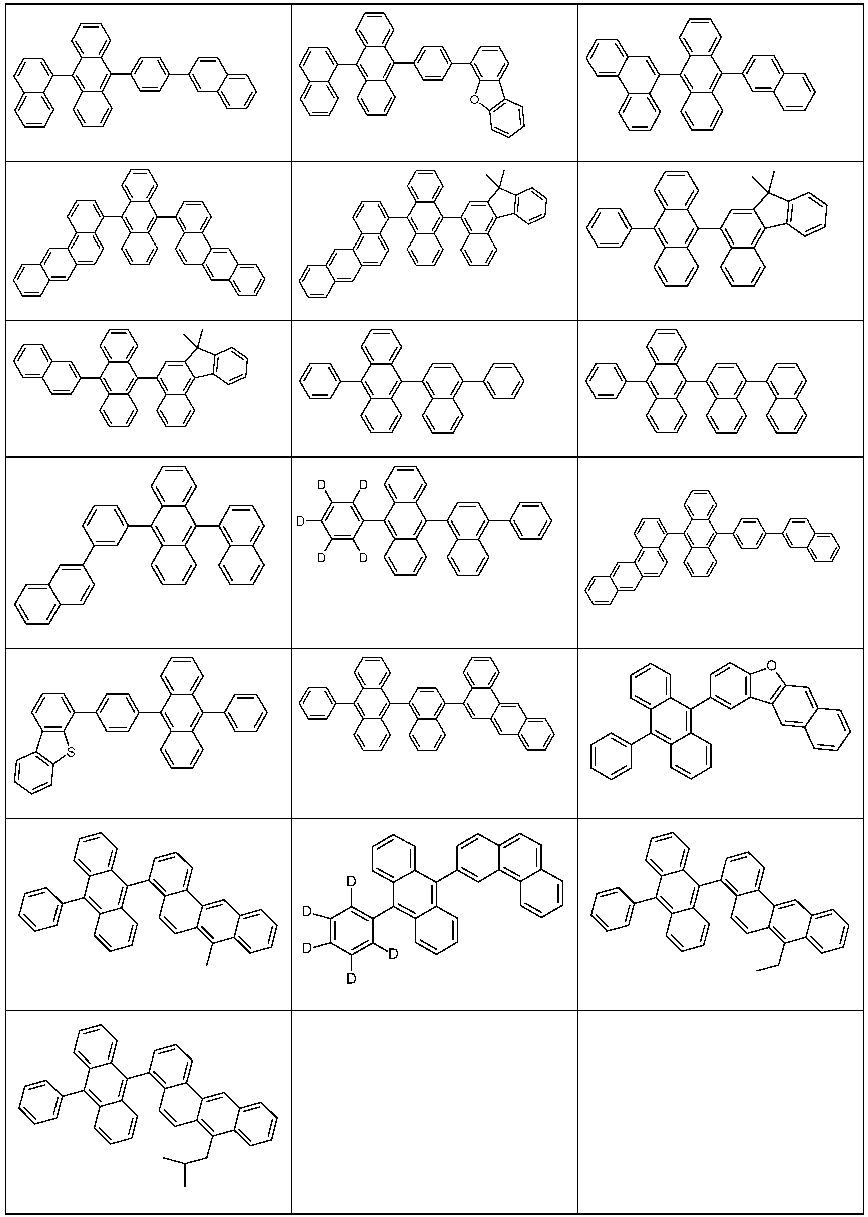

If the compound according to the invention is employed as a fluorescent emitting compound in an emitting layer, it may be employed in combination with one or more other fluorescent emitting compounds.

Preferred fluorescent emitters, besides the compounds according to the invention, are selected from the class of the arylamines. An arylamine in the sense of this invention is taken to mean a compound which contains three substituted or unsubstituted aromatic or heteroaromatic ring systems bonded directly to the nitrogen. At least one of these aromatic or heteroaromatic ring systems is preferably a condensed ring system, particularly preferably having at least 14 aromatic ring atoms. Preferred examples thereof are aromatic anthracenamines, aromatic anthracenediamines, aromatic pyrenamines, aromatic pyrenediamines, aromatic chrysenamines or aromatic chrysenediamines. An aromatic anthracenamine is taken to mean a compound in which one diarylamino group is bonded directly to an anthracene group, preferably in the 9-position. An aromatic anthracene-diamine is taken to mean a compound in which two diarylamino groups are bonded directly to an anthracene group, preferably in the 9,10-position. Aromatic pyrenamines, pyrenediamines, chrysenamines and chrysenediamines are defined analogously thereto, where the diarylamino groups are preferably bonded to the pyrene in the 1-position or in the 1,6-position. Further preferred emitters are indenofluorenamines or indenofluorene-diamines, for example in accordance with WO 2006/108497 or WO 2006/ 122630, benzoindenofluorenamines or benzoindenofluorenediamines, for example in accordance with WO 2008/006449, and dibenzoindenofluoren-amines or dibenzoindenofluorenediamines, for example in accordance with WO 2007/140847, and the indenofluorene derivatives containing condensed aryl groups which are disclosed in WO 2010/012328. Still further preferred emitters are benzanthracene derivatives as disclosed in WO 2015/158409, anthracene derivatives as disclosed in WO 2017/036573, fluorene dimers like in WO 2016/150544 or phenoxazine derivatives as disclosed in WO 2017/028940 and WO 2017/028941. Preference is likewise given to the pyrenarylamines disclosed in WO 2012/048780 and WO 2013/185871. Preference is likewise given to the benzoindenofluorenamines disclosed in WO 2014/037077, the benzofluorenamines disclosed in WO 2014/106522 and the indenofluorenes disclosed in WO 2014/111269 or WO 2017/036574.

Examples of preferred fluorescent emitting compounds, besides the compounds according to the invention, which can be used in combination with the compounds of the invention in an emitting layer or which can be used in another emitting layer of the same device are depicted in the following table:

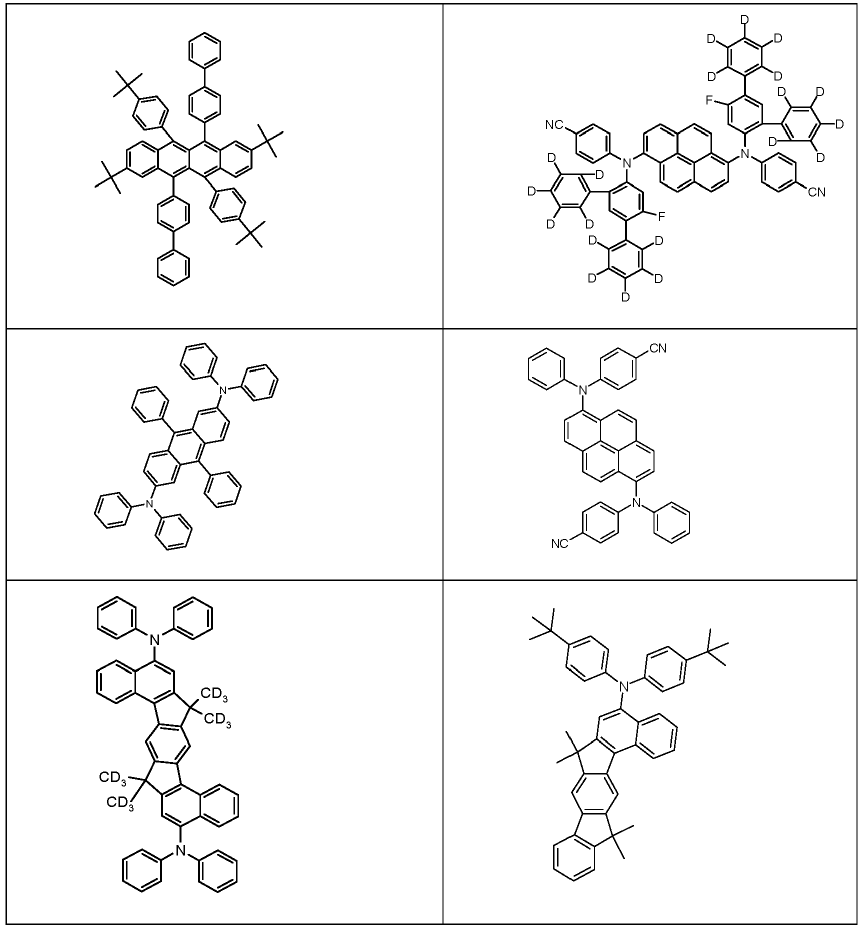

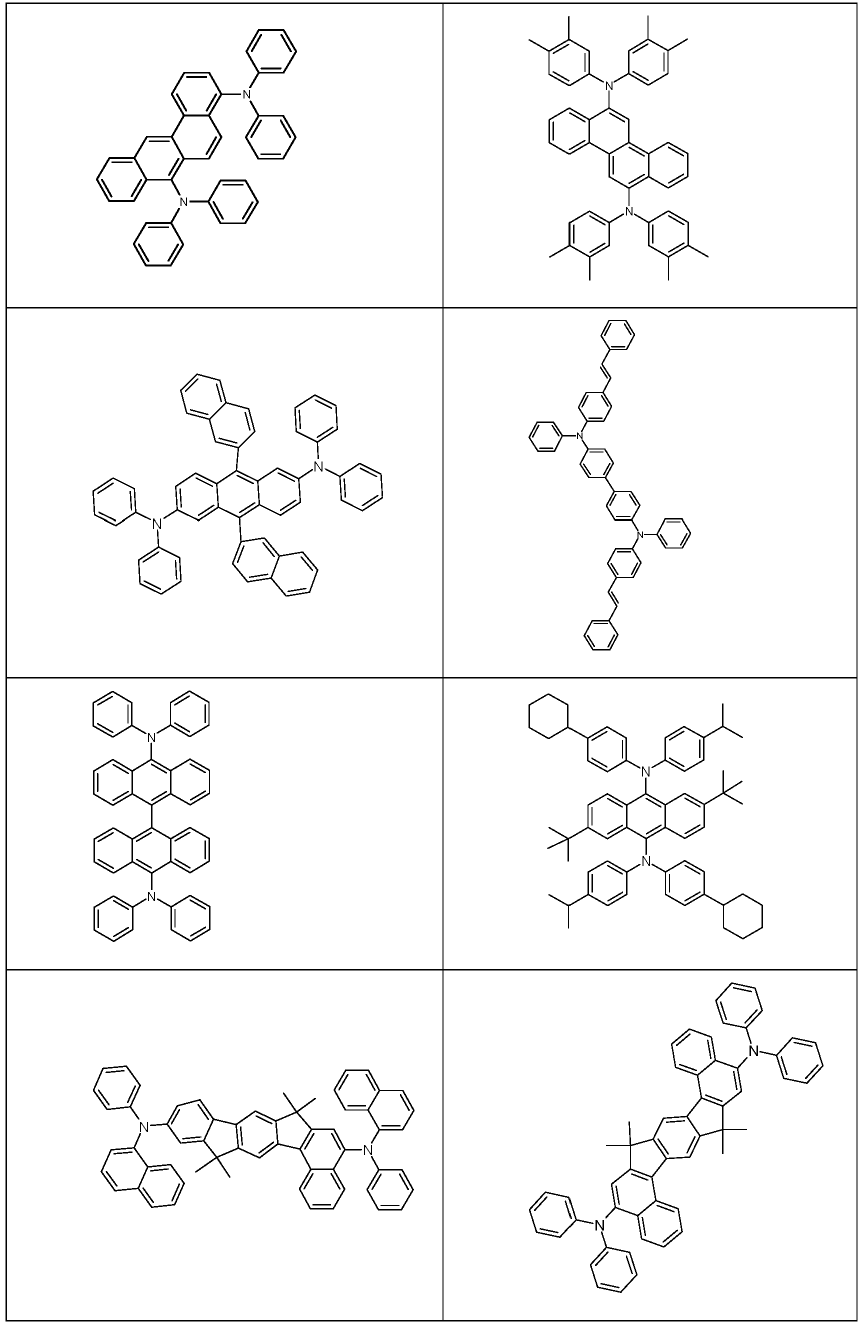

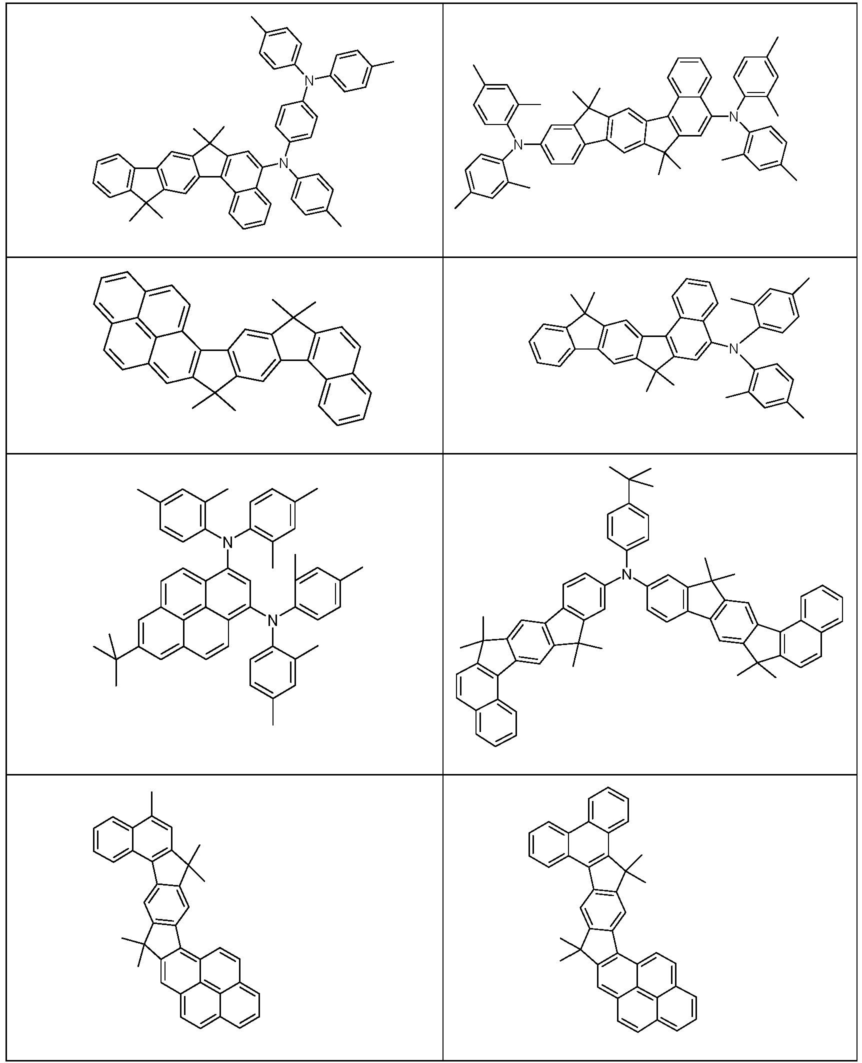

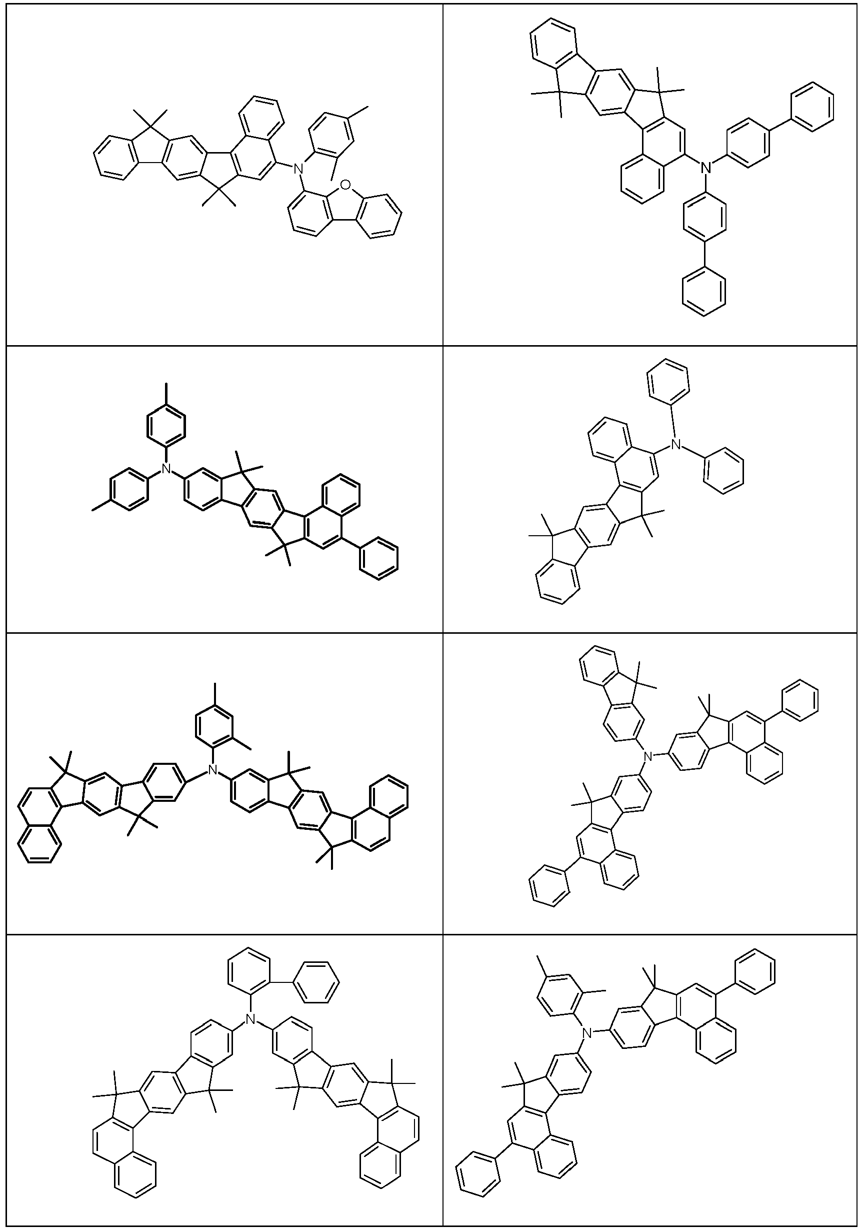

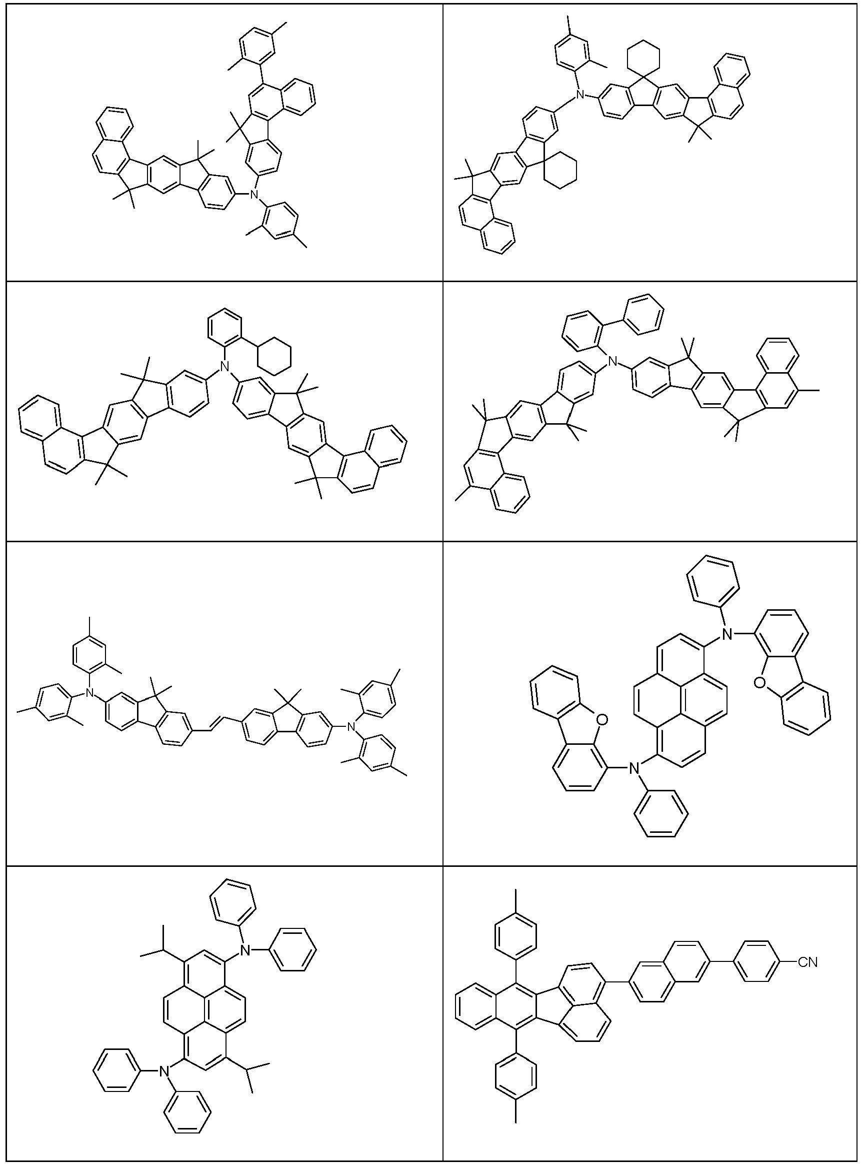

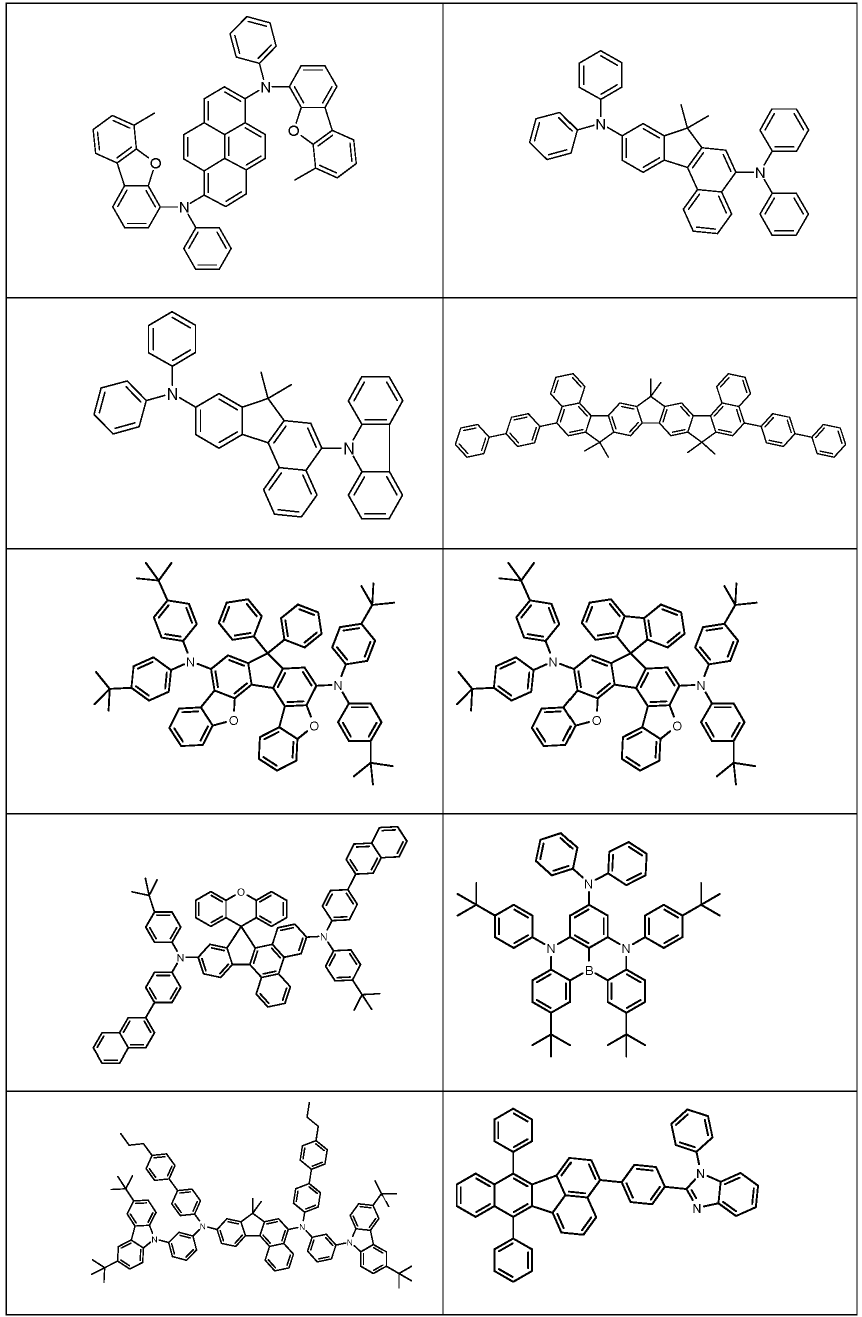

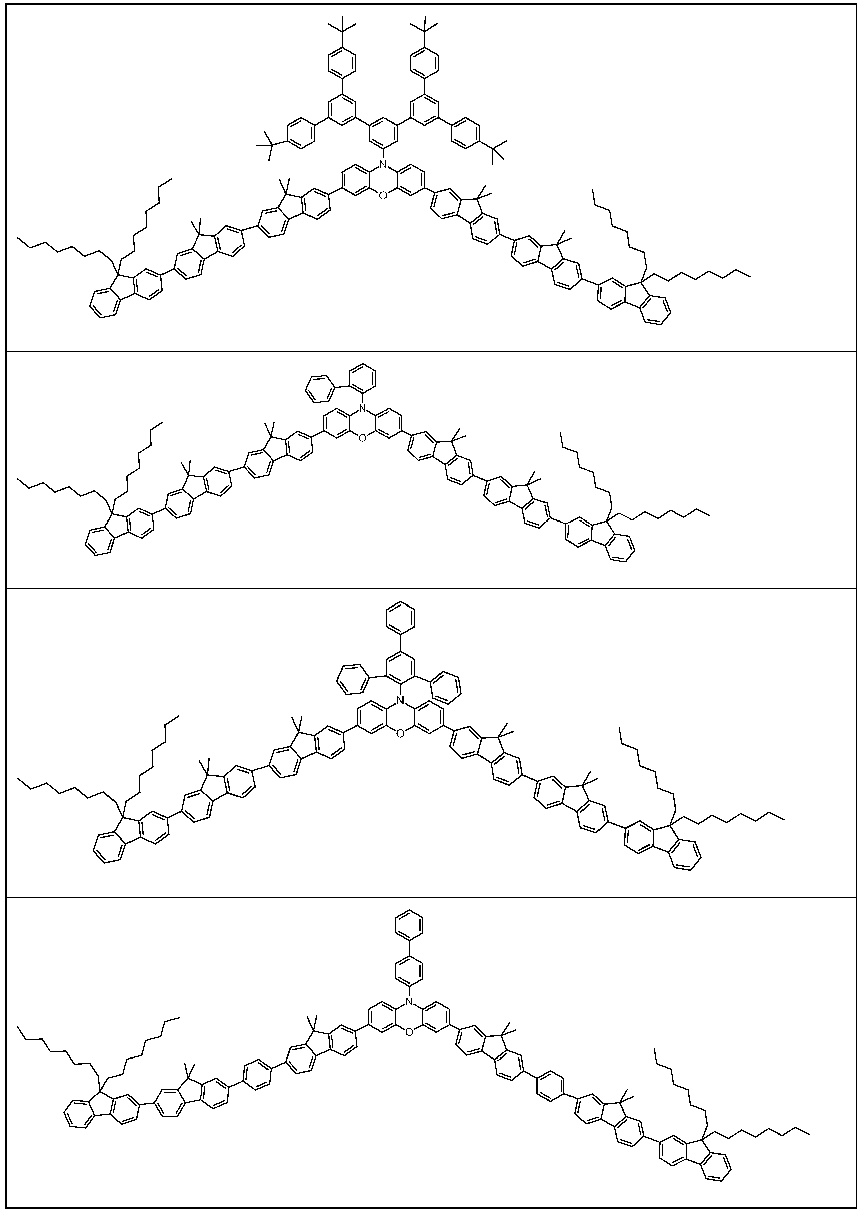

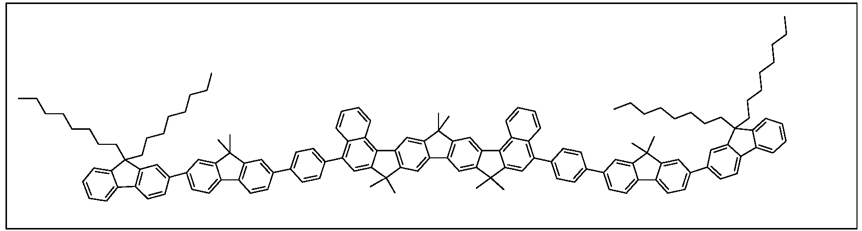

The compounds according to the invention can also be employed in other layers, for example as hole-transport materials in a hole-injection or hole-transport layer or electron-blocking layer or as matrix materials in an emitting layer, preferably as matrix materials for phosphorescent emitters.

If the compound of the formulae (3A), (3B) or (3C) is employed as hole-transport material in a hole-transport layer, a hole-injection layer or an electron-blocking layer, the compound can be employed as pure material, i.e. in a proportion of 100%, in the hole-transport layer, or it can be employed in combination with one or more further compounds. According to a preferred embodiment, the organic layer comprising the inventive compound then additionally comprises one or more p-dopants. The p-dopants employed in accordance with the present invention are preferably organic electron-acceptor compounds which are able to oxidise one or more of the other compounds of the mixture.

Particularly preferred embodiments of p-dopants are the compounds disclosed in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 and WO 2012/095143.

If the inventive compound is employed as matrix material in combination with a phosphorescent emitter in an emitting layer, the phosphorescent emitter is preferably selected from the classes and embodiments of phosphorescent emitters indicated below. Furthermore, one or more further matrix materials are preferably present in the emitting layer in this case.

So-called mixed-matrix systems of this type preferably comprise two or three different matrix materials, particularly preferably two different matrix materials. It is preferred here for one of the two materials to be a material having hole-transporting properties and for the other material to be a material having electron-transporting properties. The compound of the formulae (3A), (3B) or (3C) is preferably the material having hole-transporting properties.

However, the desired electron-transporting and hole-transporting properties of the mixed-matrix components may also be combined mainly or completely in a single mixed-matrix component, where the further mixed-matrix component or components satisfy other functions. The two different matrix materials may be present here in a ratio of 1:50 to 1:1, preferably 1:20 to 1:1, particularly preferably 1:10 to 1:1 and very particularly preferably 1:4 to 1:1. Mixed-matrix systems are preferably employed in phosphorescent organic electroluminescent devices. Further details on mixed-matrix systems are contained, inter alia, in the application WO 2010/108579.

Particularly suitable matrix materials which can be used as matrix components of a mixed-matrix system in combination with the compounds according to the invention are selected from the preferred matrix materials for phosphorescent emitters indicated below or the preferred matrix materials for fluorescent emitters, depending on what type of emitter compound is employed in the mixed-matrix system.

Generally preferred classes of material for use as corresponding functional materials in the organic electroluminescent devices according to the invention are indicated below.

Suitable phosphorescent emitters are, in particular, compounds which emit light, preferably in the visible region, on suitable excitation and in addition contain at least one atom having an atomic number greater than 20, preferably greater than 38 and less than 84, particularly preferably greater than 56 and less than 80. The phosphorescent emitters used are preferably compounds which contain copper, molybdenum, tungsten, rhenium, ruthenium, osmium, rhodium, iridium, palladium, platinum, silver, gold or europium, in particular compounds which contain iridium, platinum or copper.

For the purposes of the present invention, all luminescent iridium, platinum or copper complexes are regarded as phosphorescent compounds.

Examples of the phosphorescent emitters described above are revealed by the applications WO 2000/70655, WO 2001/41512, WO 2002/02714, WO 2002/15645, EP 1191613, EP 1191612, EP 1191614, WO 2005/ 033244, WO 2005/019373 and US 2005/0258742. In general, all phosphorescent complexes as used in accordance with the prior art for phosphorescent OLEDs and as are known to the person skilled in the art in the area of organic electroluminescent devices are suitable for use in the devices according to the invention. The person skilled in the art will also be able to employ further phosphorescent complexes without inventive step in combination with the compounds according to the invention in OLEDs. Preferred matrix materials for phosphorescent emitters are aromatic ketones, aromatic phosphine oxides or aromatic sulfoxides or sulfones, for example in accordance with WO 2004/013080, WO 2004/093207, WO 2006/005627 or WO 2010/006680, triarylamines, carbazole derivatives, for example CBP (N,N-biscarbazolylbiphenyl) or the carbazole derivatives disclosed in WO 2005/039246, US 2005/0069729, JP 2004/288381, EP 1205527 or WO 2008/086851, indolocarbazole derivatives, for example in accordance with WO 2007/063754 or WO 2008/056746, indenocarbazole derivatives, for example in accordance with WO 2010/136109, WO 2011/ 000455 or WO 2013/041176, azacarbazole derivatives, for example in accordance with EP 1617710, EP 1617711, EP 1731584, JP 2005/347160, bipolar matrix materials, for example in accordance with WO 2007/137725, silanes, for example in accordance with WO 2005/111172, azaboroles or boronic esters, for example in accordance with WO 2006/117052, triazine derivatives, for example in accordance with WO 2010/015306, WO 2007/063754 or WO 2008/056746, zinc complexes, for example in accordance with EP 652273 or WO 2009/062578, diazasilole or tetraazasilole derivatives, for example in accordance with WO 2010/054729, diazaphosphole derivatives, for example in accordance with WO 2010/054730, bridged carbazole derivatives, for example in accordance with US 2009/0136779, WO 2010/050778, WO 2011/042107, WO 2011/088877 or WO 2012/143080, triphenylene derivatives, for example in accordance with WO 2012/048781, or lactams, for example in accordance with WO 2011/ 116865 or WO 2011/137951.

Besides the compounds according to the invention, suitable charge-transport materials, as can be used in the hole-injection or hole-transport layer or electron-blocking layer or in the electron-transport layer of the electronic device according to the invention, are, for example, the compounds disclosed in Y. Shirota et al., Chem. Rev. 2007, 107(4), 953-1010, or other materials as are employed in these layers in accordance with the prior art.

Materials which can be used for the electron-transport layer are all materials as are used in accordance with the prior art as electron-transport materials in the electron-transport layer. Particularly suitable are aluminium complexes, for example Alq₃, zirconium complexes, for example Zrq₄, lithium complexes, for example LiQ, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Furthermore, suitable materials are derivatives of the above-mentioned compounds, as disclosed in JP 2000/053957, WO 2003/060956, WO 2004/028217, WO 2004/080975 and WO 2010/ 072300.

Preferred hole-transport materials which can be used in a hole-transport, hole-injection or electron-blocking layer in the electroluminescent device according to the invention are indenofluorenamine derivatives (for example in accordance with WO 06/122630 or WO 06/100896), the amine derivatives disclosed in EP 1661888, hexaazatriphenylene derivatives (for example in accordance with WO 01/049806), amine derivatives containing condensed aromatic rings (for example in accordance with US 5,061,569), the amine derivatives disclosed in WO 95/09147, monobenzoindenofluoren-amines (for example in accordance with WO 08/006449), dibenzoindeno-fluorenamines (for example in accordance with WO 07/140847), spiro-bifluorenamines (for example in accordance with WO 2012/034627 or WO 2013/120577), fluorenamines (for example in accordance with the as yet unpublished applications EP 12005369.9, EP 12005370.7 and EP 12005371.5), spirodibenzopyranamines (for example in accordance with WO 2013/083216) and dihydroacridine derivatives (for example in accordance with WO 2012/150001). The compounds according to the invention can also be used as hole-transport materials.

The cathode of the organic electroluminescent device preferably comprises metals having a low work function, metal alloys or multilayered structures comprising various metals, such as, for example, alkaline-earth metals, alkali metals, main-group metals or lanthanoids (for example Ca, Ba, Mg, Al, In, Mg, Yb, Sm, etc.). Also suitable are alloys comprising an alkali metal or alkaline-earth metal and silver, for example an alloy comprising magnesium and silver. In the case of multilayered structures, further metals which have a relatively high work function, such as, for example, Ag or Al, can also be used in addition to the said metals, in which case combinations of the metals, such as, for example, Ca/Ag, Mg/Ag or Ag/Ag, are generally used. It may also be preferred to introduce a thin interlayer of a material having a high dielectric constant between a metallic cathode and the organic semiconductor. Suitable for this purpose are, for example, alkali metal fluorides or alkaline-earth metal fluorides, but also the corresponding oxides or carbonates (for example LiF, Li₂O, BaF₂, MgO, NaF, CsF, Cs₂CO₃, etc.). Furthermore, lithium quinolinate (LiQ) can be used for this purpose. The layer thickness of this layer is preferably between 0.5 and 5 nm.

The anode preferably comprises materials having a high work function. The anode preferably has a work function of greater than 4.5 eV vs. vacuum. Suitable for this purpose are on the one hand metals having a high redox potential, such as, for example, Ag, Pt or Au. On the other hand, metal/metal oxide electrodes (for example Al/Ni/NiOₓ, Al/PtOₓ) may also be preferred. For some applications, at least one of the electrodes must be transparent or partially transparent in order to facilitate either irradiation of the organic material (organic solar cells) or the coupling-out of light (OLEDs, O-lasers). Preferred anode materials here are conductive mixed metal oxides. Particular preference is given to indium tin oxide (ITO) or indium zinc oxide (IZO). Preference is furthermore given to conductive, doped organic materials, in particular conductive doped polymers.

The device is appropriately (depending on the application) structured, provided with contacts and finally sealed, since the lifetime of the devices according to the invention is shortened in the presence of water and/or air.

In a preferred embodiment, the organic electroluminescent device according to the invention is characterised in that one or more layers are coated by means of a sublimation process, in which the materials are applied by vapour deposition in vacuum sublimation units at an initial pressure of less than 10⁻⁵ mbar, preferably less than 10⁻⁶ mbar. However, it is also possible here for the initial pressure to be even lower, for example less than 10⁻⁷ mbar.

Preference is likewise given to an organic electroluminescent device, characterised in that one or more layers are coated by means of the OVPD (organic vapour phase deposition) process or with the aid of carrier-gas sublimation, in which the materials are applied at a pressure of between 10^{- 5} mbar and 1 bar. A special case of this process is the OVJP (organic vapour jet printing) process, in which the materials are applied directly through a nozzle and are thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, screen printing, flexographic printing, nozzle printing or offset printing, but particularly preferably LITI (light induced thermal imaging, thermal transfer printing) or ink-jet printing. Soluble compounds of the formulae (3A), (3B) or (3C) are necessary for this purpose. High solubility can be achieved through suitable substitution of the compounds.

Also possible are hybrid processes, in which, for example, one or more layers are applied from solution and one or more further layers are applied by vapour deposition. Thus, it is possible, for example, to apply the emitting layer from solution and to apply the electron-transport layer by vapour deposition. These processes are generally known to the person skilled in the art and can be applied by him without inventive step to organic electroluminescent devices comprising the compounds according to the invention.

In accordance with the invention, the electronic devices comprising one or more compounds according to the invention can be employed in displays, as light sources in lighting applications and as light sources in medical and/or cosmetic applications (for example light therapy).

The invention will now be explained in greater detail by the following examples, without wishing to restrict it thereby.

### A) Syntheses Examples

### Scheme synthesis example of intermediate VII-a

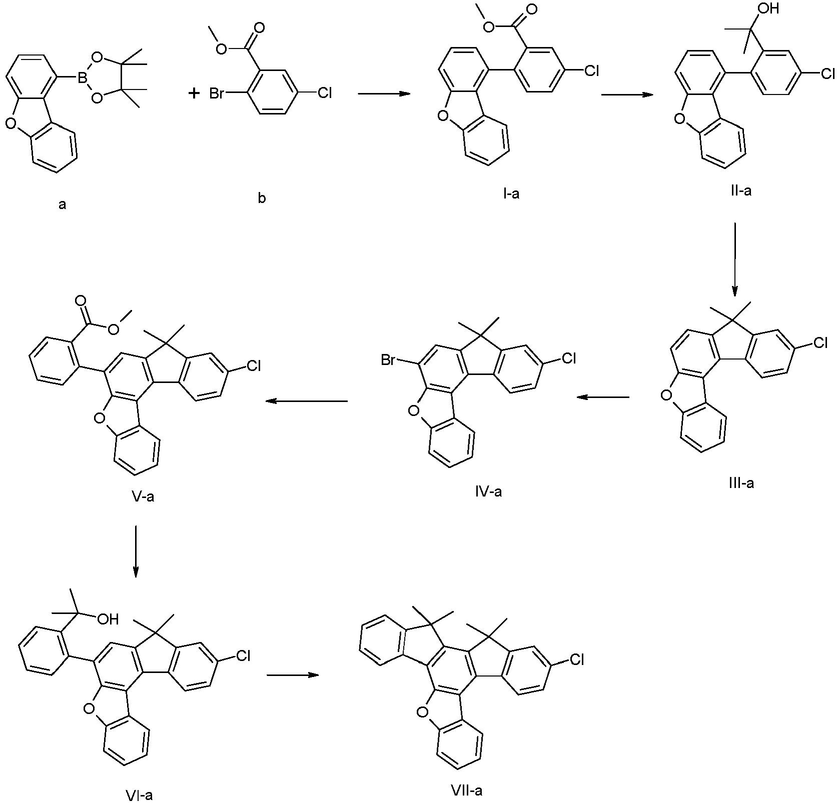

### Synthesis Intermediate I-a:

117.9 g (401 mmol) starting material a, 100 g (401 mmol) starting material b and 203.1 g (882 mmol) potassium phosphate monohydrate are mixed in 1.6 L toluene/water/dioxane (2:1:1) and degassed. To the mixture, palladium acetate (0.9 g, 4 mmol) and tri-ortho-tolylphosphine (2.44 g, 8 mmol) are added and the mixture is stirred at reflux for 16 h. After cooling the mixture to room temperature, the phases are separated. The aqueous phase is further extracted with ethyl acetate (2 x 300 mL). The combined organic phases are washed multiple times with water, dried over sodium sulfate and finally removed in vacuum. The crude is filtered over a plug of SiO₂/Al₂O₃ using ethyl acetate as solvent. After removing the solvent in vacuum, an oil is obtained in quantitative yield.

In a similar manner, the following compounds can also be prepared:

| Example | Starting material | Starting material | Intermediate I |
|---|---|---|---|
| **I-b** | 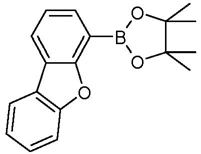 | 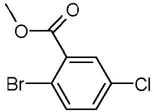 | 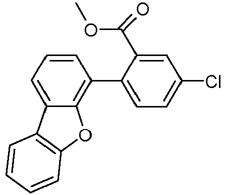 |
| **I-c** | 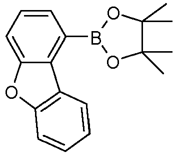 | 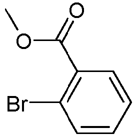 | 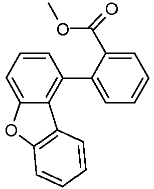 |

### Synthesis Intermediate II-a:

MeMgCl (461 mL, 3 M in THF, 1.38 mol) is added dropwise to a pre-cooled THF suspension (0 °C, 1.5 L) of compound **I-a** (135 g, 0.4 mol) and CeCl₃ (199 g, 0.8 mol). After completion of the reaction, a saturated aqueous solution of NH₄Cl is added to quench the excess of MeMgCl, and the organic phase is extracted three times with ethyl acetate. The organic fractions are combined and washed with water and brine, successively. The volatiles were removed in vacuum to yield the desired product. 129 g (96 %).

In a similar manner, the following compounds can also be prepared:

| Example | Intermediate I | Intermediate II |
|---|---|---|
| **II-b** | 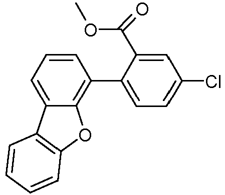 | 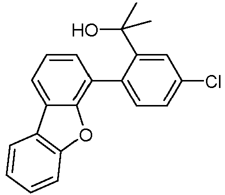 |
| **II-c** | 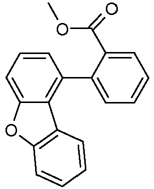 | 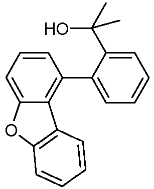 |
| **II-d** *instead of MeMgCl Phenylmagnesium chloride (100-59-4) is used | 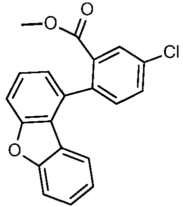 | 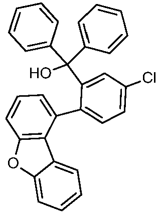 |

### Synthesis Intermediate III-a:

To a solution of compound **II-a** (129 g, 383 mmol) in toluene (1 L), 50 g of Amberlyst-15 are added. The mixture is stirred at reflux overnight. The mixture is cooled down to room temperature and the Amberlyst-15 filtered off. The solvent is removed in vacuum and the crude product is purified by column chromatography (SiO₂, heptane). Yield: 106.2 g (87 %).

In a similar manner, the following compounds can also be prepared:

| Example | Intermediate II | Intermediate III |
|---|---|---|
| **III-b** | 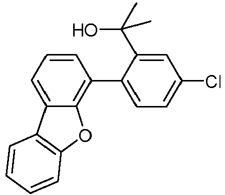 | 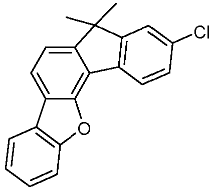 |
| **III-c** | 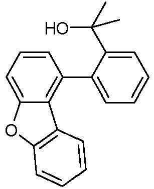 | 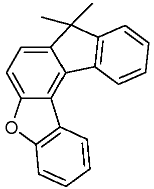 |
| **III-d** | 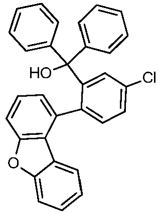 | 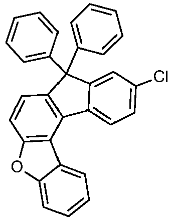 |

### Synthesis Intermediate IV-a:

To a solution of compound **III-a** (100 g, 314 mmol) in CH₂Cl₂ (1.2 L), N-bromosuccinimide (55.83 g, 314 mmol) and HBr (32 % solution in acetic acid, 0.5 mL) are added. The reaction is heated at 30 °C for 4 days. After completion of the reaction, Na₂S₂O₃ (300 mL, saturated aqueous solution) is added and the mixture is stirred vigorously for 30 minutes. The phases are separated and the organic phase is washed several times with water. The solvent is removed in vacuum and the crude product vigorously stirred with ethanol to yield a white solid. Yield: 119.8 g (96%).

In a similar manner, the following compounds can also be prepared:

| Example | Intermediate III | Intermediate IV |
|---|---|---|
| **IV-b** | 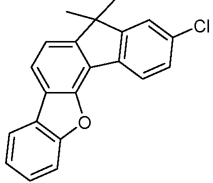 | 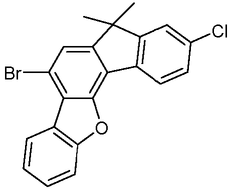 |
| **IV-c** | 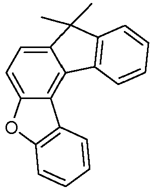 | 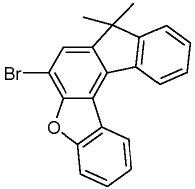 |
| **IV-d** | 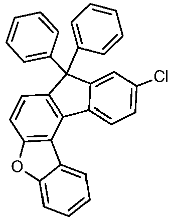 | 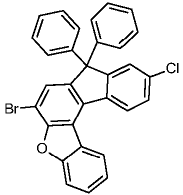 |

### Synthesis Intermediate V-a:

27.5 g (69.1 mmol) **IV-a,** 13.1 g (72.6 mmol) (2-methoxycarbonylphenyl)boronic acid and 35.0 g (152.1 mmol) potassium phosphate monohydrate are mixed in 300 mL toluene and degassed. To the mixture, 2-dicyclohexylphosphino-2',4',6'-triisopropyl-1,1'-biphenyl)[2-(2'-amino-1,1'-biphenyl)]palladium(II) methanesulfonate (1.18 g, 2.07 mmol) is added and the mixture is stirred at reflux for 3 h. After cooling the mixture to room temperature, 300 mL water are added and the phases are separated. The organic phase is washed multiple times with water and the solvent is removed in vacuum. Afterwards, the crude product is purified by column chromatography (SiO₂, toluene). Yield: 28.2 g (90 %).

In a similar manner, the following compounds can also be prepared:

| Examples | Intermediate IV | Intermediate V |
|---|---|---|
| **V-b** | 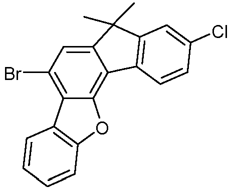 | 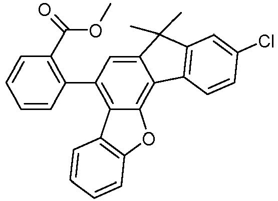 |
| **V-c** | 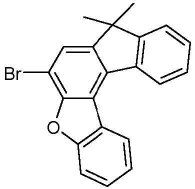 | 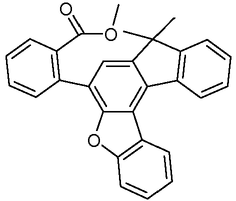 |
| **V-d** | 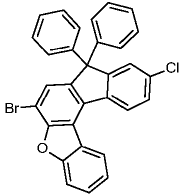 | 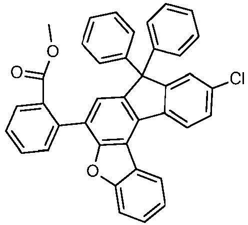 |

### Synthesis Intermediate VI-a:

Intermediate **VI-a** can be prepared in a similar manner as intermediate **II-a** starting from **V-a** (28.0 g, 61.8 mmol), MeMgCl (71 mL, 3 M in THF, 213 mmol) and CeCl₃ (30,5 g, 124 mmol) in 350 mL of THF. Yield: 25.5 g (91 %).

In a similar manner, the following compounds can also be prepared:

| Examples | Intermediate V | Intermediate VI |
|---|---|---|
| **VI-b** | 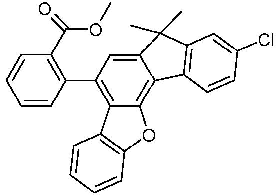 | 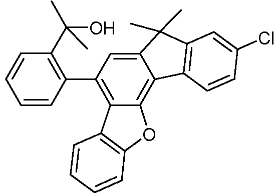 |
| **VI-c** | 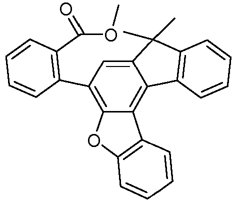 | 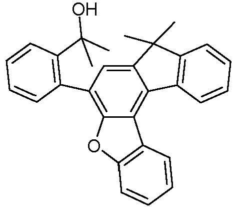 |
| **VI-d*** | 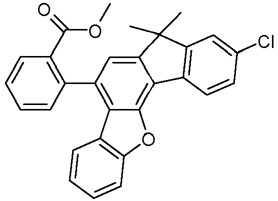 | 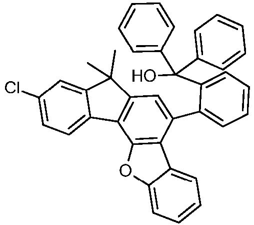 |
| *instead of MeMgCl Phenylmagnesium chloride (100-59-4) is used | | |
| **VI-e** | 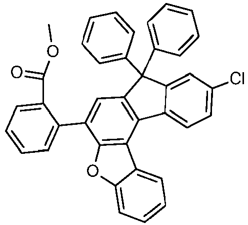 | 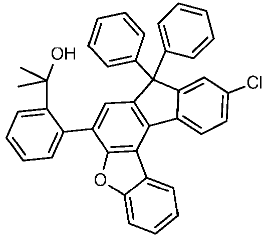 |
| **VI-f*** | 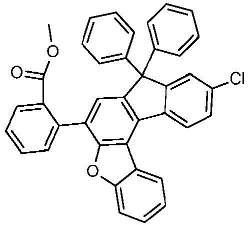 | 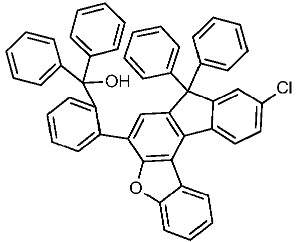 |
| *instead of MeMgCl Phenylmagnesium chloride (100-59-4) is used | | |

### Synthesis Intermediate VII-a:

Intermediate **VII-a** can be synthesized following the same procedure as intermediate **III-a** starting from 25 g (55 mmol) of **VI-a** and 12 g of Amberlyst-15 in 300 mL of toluene. Yield: 20.4 g (85%).

In a similar manner, the following compounds can also be prepared:

| Example s | Intermediate VI | Intermediate VII |
|---|---|---|
| **VII-b** | 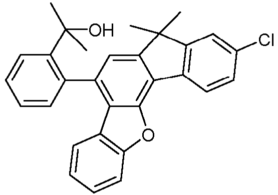 | 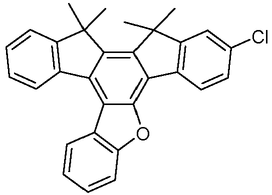 |
| **VII-c** | 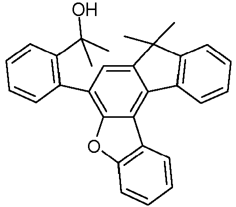 | 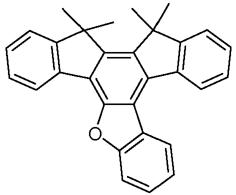 |
| **VII-d** | 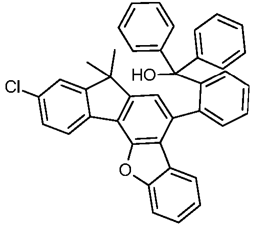 | 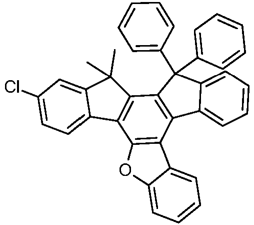 |
| **VII-e** | 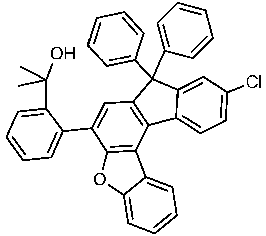 | 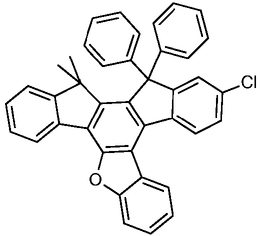 |
| **VII-f** | 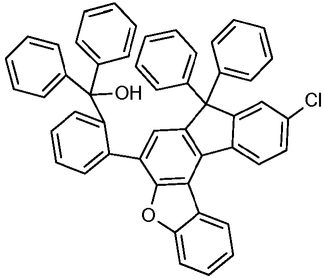 | 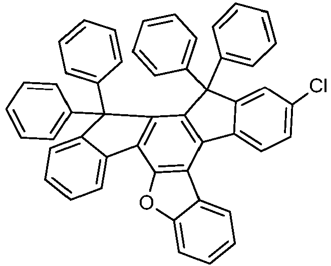 |

### Scheme synthesis example of Intermediate VIII

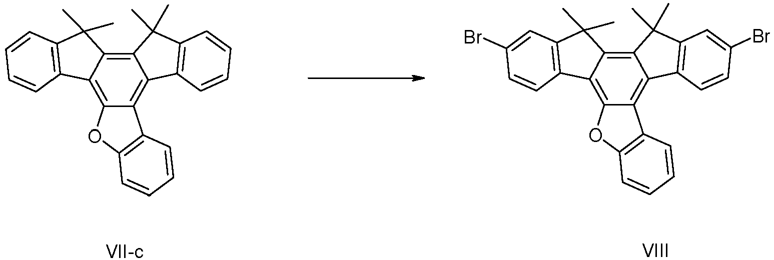

### Synthesis of Intermediate VIII:

Intermediate **VIII** can be synthesized following the same procedure as intermediate **IV-a** starting from 15 g (37.5 mmol) of **VII-c** and 13.3 g (75 mmol) of N-bromosuccinimide in 300 mL CH₂Cl₂. Yield: 15.3 g (73%).

In a similar manner following compounds can be synthesized, by using stoichiometric amounts of N-bromsuccinimid.

| Example s | Intermediate VII | Intermediate VIII |
|---|---|---|
| **VIII-a** | 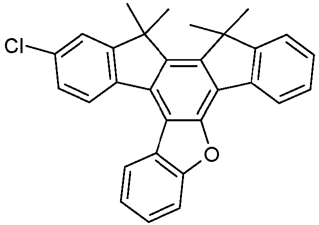 | 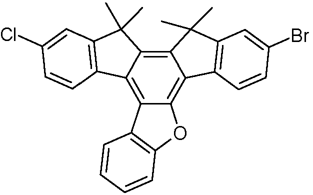 |
| **VIII-b** | 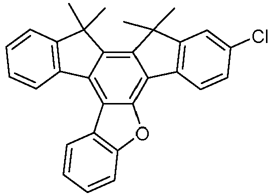 | 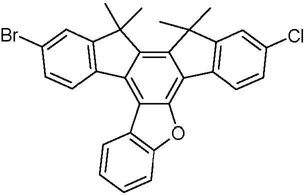 |
| **VIII-c** | 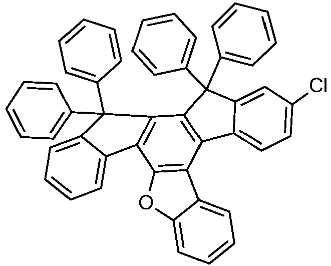 | 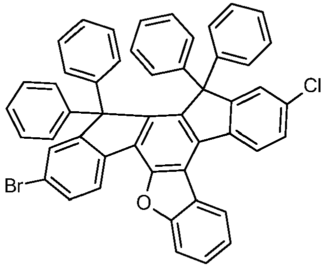 |

### Synthesis of compound E-Int1.1

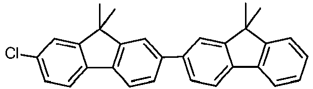

30 g (97.5 mmol) 2-Bromo-7-Chloro-9,9-dimethyl-9H-fluorene (see JP 2003277305 A), 25.5 g (107.3 mmol) (9,9-dimethylfluoren-2-yl)boronic acid 90 g (390mmol), 0.9 g (4 mmol) palladium(II)acetate and 3.6 g (11.7 mmol) tri(o-tolyl)-phosphine are dissolved in 1 liter of a toluene, dioxane, water mixture (1:1:1) and stirred at reflux overnight. After cooling down to room temperature 200 mL toluene are added and the organic phase is separated and washed with water (2x200 mL) the combined organic phases are concentrated under reduced pressure. The residue is purified by recrystallization from toluene/heptane.
Yield: 39.1 g (93 mmol; 96%)

Following compounds can be synthesized in an analogous manner:

| Compound | Starting material A | Starting material B | Product |
|---|---|---|---|
| **E-Int1.2** | 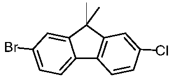 see | 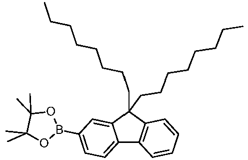 | 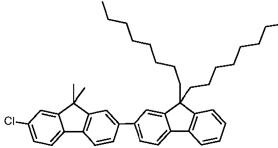 |
| | JP 2003277305 A | | |
| **E-Int1.3** | 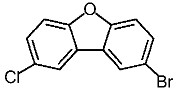 see | 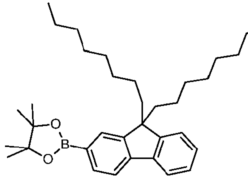 | 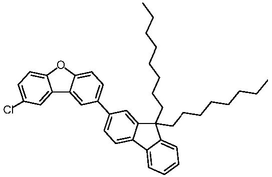 |
| | KR 2015136028 A | | |

### Synthesis of EG1

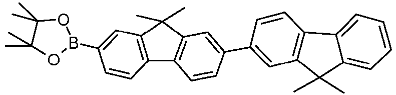

40 g (95 mmol) **E-Int1.1,** 38.6 g (152 mmol) bis-(pinacolato)-diboron, 4.2 g (5.7 mmol) trans-dichloro(tricyclohexylphosphine)palladium(II) and 28 g (285 mmol) potassium acetate are dissolved in 400 mL dioxane and stirred for 16 h at reflux. The reaction mixture is allowed to cool to room temperature and 400 mL toluene are added. The organic phase is separated, washed with water (2 x 200 mL) and filtered through Celite. The solution is concentrated to dryness under reduced pressure. The residue is purified by recrystallization from toluene/heptane.
Yield: 36 g (70 mmol; 74 %)

Following compounds can be synthesized in an analogous manner:

| Compound | Starting material | Product |
|---|---|---|
| **EG2** | **E-Int1.2** | 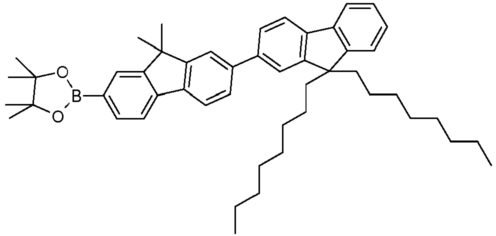 |
| **EG3** | **E-Int1.3** | 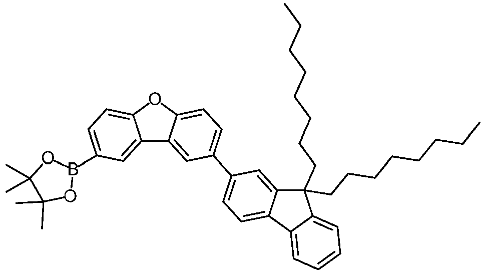 |

### Synthesis of E-Int2.1

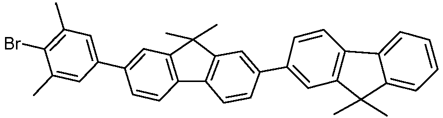

5.5 g (17.8 mmol) 2-Bromo-5-iodo-1,3-dimethylbenzene, 6.5 g (12.7 mmol) **EG1**, 366 mg (0.3 mmol) tetrakis(triphenylphosphin)-palladium(0) and 2.7 g (13 mmol) sodium carbonate are dissolved in 200 mL toluene, ethanol and water (2:1:1) and stirred for 16 hours at 90 °C. After cooling down to room temperature 100 ml toluene are added, the organic phase is separated and washed with water (2 x 50 mL). The organic phase is concentrated to dryness under reduced pressure. The residue is purified by recrystallization from toluene/heptane.
Yield: 6.2 g (11 mmol; 86 %)

The following compounds can be synthesized in an analogous manner:

| Compound | Boronate | Starting material B | Product |
|---|---|---|---|
| **E-Int2.2** | EG2 | 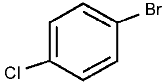 CAS 106-39-8 | 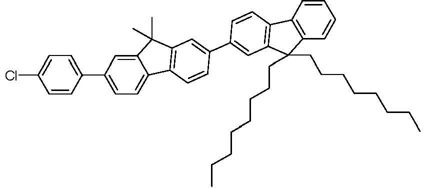 |
| **E-Int2.3** | EG2 | 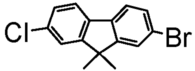 see | 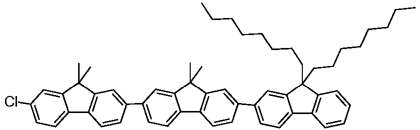 |
| | | JP 2003277305 A | |
| **E-Int2.4** | CAS 1679-18-1 | E-Int2.1 | 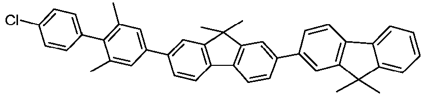 |

### Synthesis of EG4 to EG6:

Compounds **EG3** to **EG5** can be synthesized in an analogous manner to **EG1:**

| Compound | Starting material | Product |
|---|---|---|
| **EG4** | **E-Int2.4** | 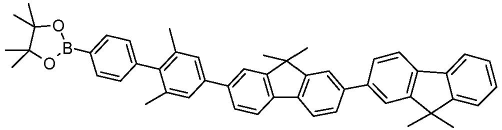 |
| **EG5** | **E-Int2.3** | 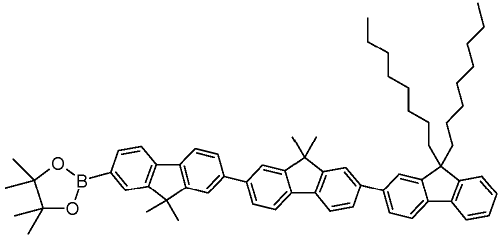 |
| **EG6** | **E-Int2.2** | 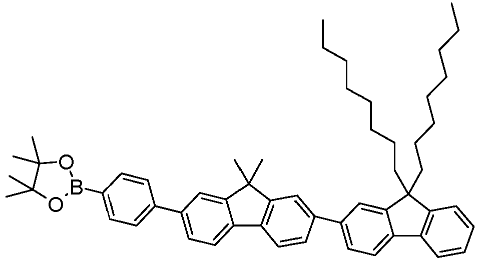 |

### Synthesis of the amine (EA.1):

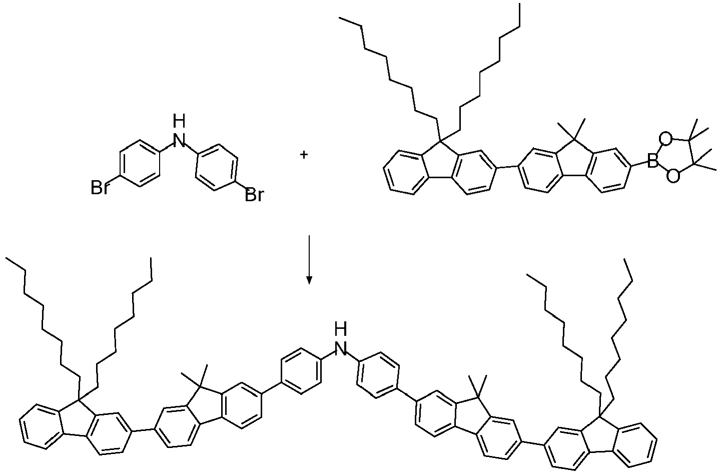

12.2 g (37.3 mmol) bis-(4-bromo-phenyl)-amine, 55.5 g (78.4 mmol) EG2, 37.8 g (164.2 mmol) potassiumphosphate monohydrate and 1.2 g (1.5 mmol) XPhos Pd Gen 3 (CAS 1445085-55-1) are added to 600 mL THF/water (2:1) and stirred at 65 °C. After 16 h the mixture is cooled to room temperature diluted with toluene and water. The organic phase is collected, the aqueous phase is extracted further with toluene. The combined organics are washed with brine, collected, dried with Na₂SO₄, filtered and concentrated. The resulting residue is deposited in 1L EtOH and stirred vigorously until a free flowing precipitate is formed. The precipitate is collected by filtration and washing with EtOH. The material is taken up in DCM and filtered through SiO2. The filtrate is concentrated to dryness. Yield: 44.7 g (33.6 mmol; 90%)

The following compounds **EA.2** to **EA.6** can be synthesized in analogous manner:

| Comp. | SM1 | SM2 | Product |
|---|---|---|---|
| **EA.2** | **EG1** | 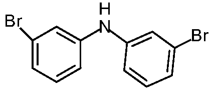 CAS 1383254-17-8 | 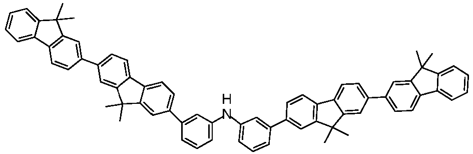 |
| **EA.3** | **EG1** | 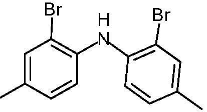 CAS 27996-13-0 | 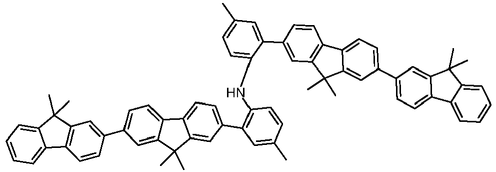 |
| **EA.4** | **EG4** | 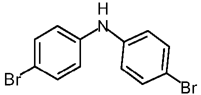 CAS 16929-17-4 | 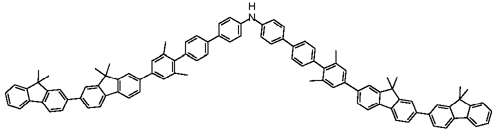 |
| **EA.5** | **EG3** | 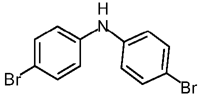 CAS 16929-17-4 | 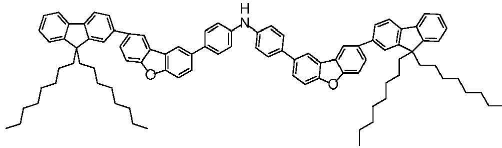 |
| **EA.6** | **EG2** | 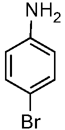 CAS 106-40-1 | 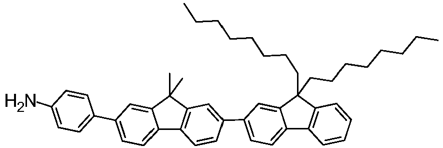 |

### Synthesis of E-VIII

Compounds **E-VIII-a** to **E-VIII-d** can be synthesized in similar manner like **EA.1** by using stoichiometric compounds of the boronate.

| Compound | SM | Boronate | Product |
|---|---|---|---|
| **E-VIII-a** | **VIII-a** | **EG6** | 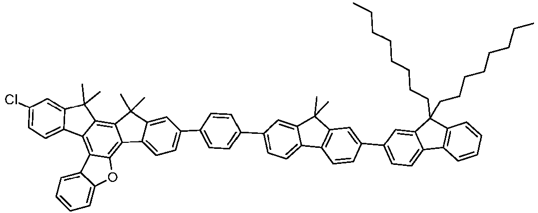 |
| **E-VIII-b** | **VIII-b** | **EG6** | 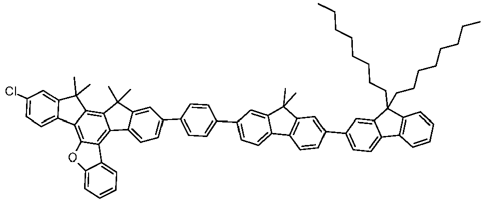 |
| **E-VIII-c** | **VIII-b** | **EG2** | 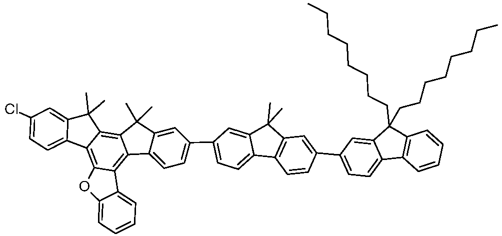 |
| **E-VIII-d** | **VIII-b** | **EG5** | 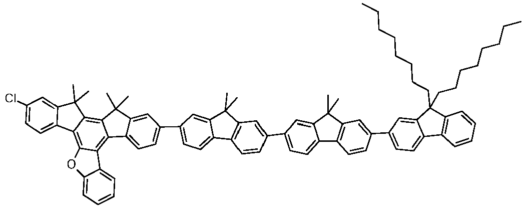 |
| **E-VIII-e** | **VIII-c** | **EG6** | 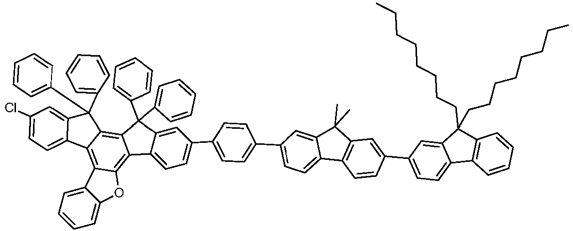 |

### Scheme synthesis example of compound 1

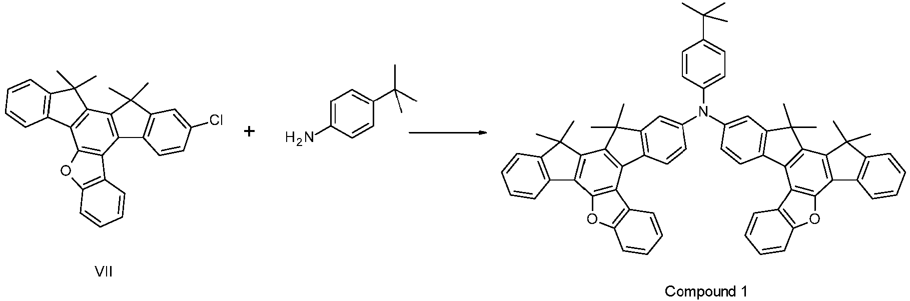

### Synthesis of compound 1

2.8 g (18.6 mmol) 4-tert-butylaniline, 16.1 g (37.2 mmol) **VII** and 10.7 g (111.4 mmol) sodium tertbutylate are mixed in 400 mL toluene and degassed. Afterwards, 0.46 g (1.15 mmol) S-Phos and 0.13 g (0.57 mmol) palladium acetate are added and the mixture is stirred at reflux for 16 h. After cooling the mixture at room temperature, 200 mL of water is added and the phases are separated. The crude product is filtrate over a plug of aluminium oxide using toluene as solvent. The product is further purified by several recrystallizations from toluene/heptane. Yield: 11.2 g (64 %).

In a similar manner, the next compounds can be prepared:

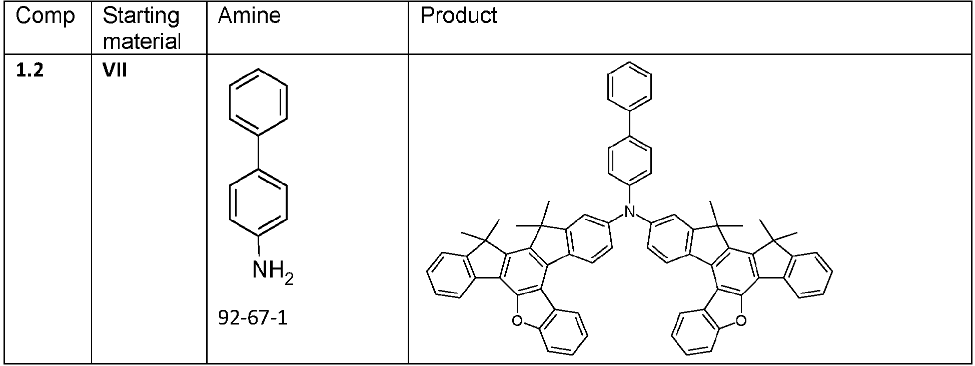

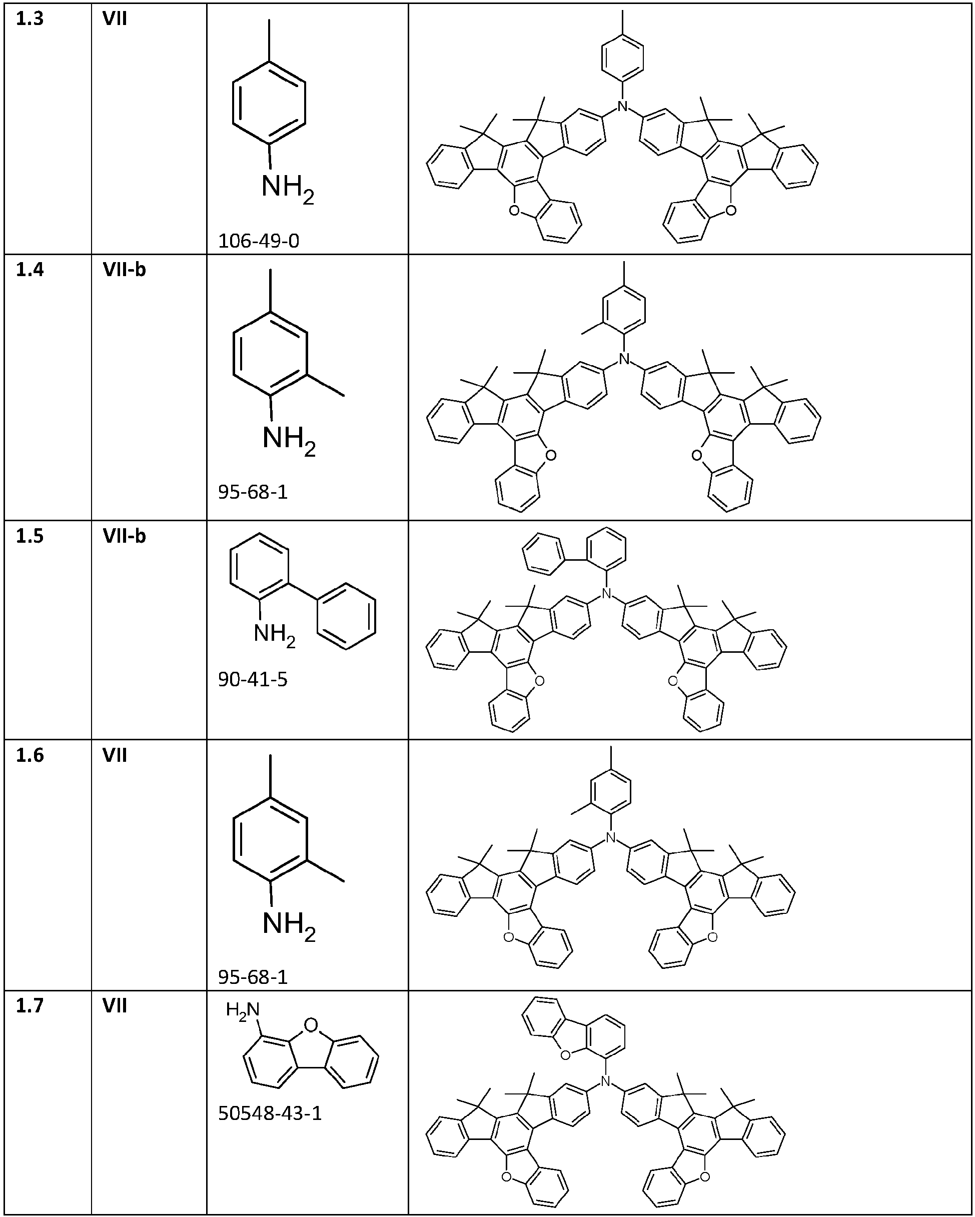

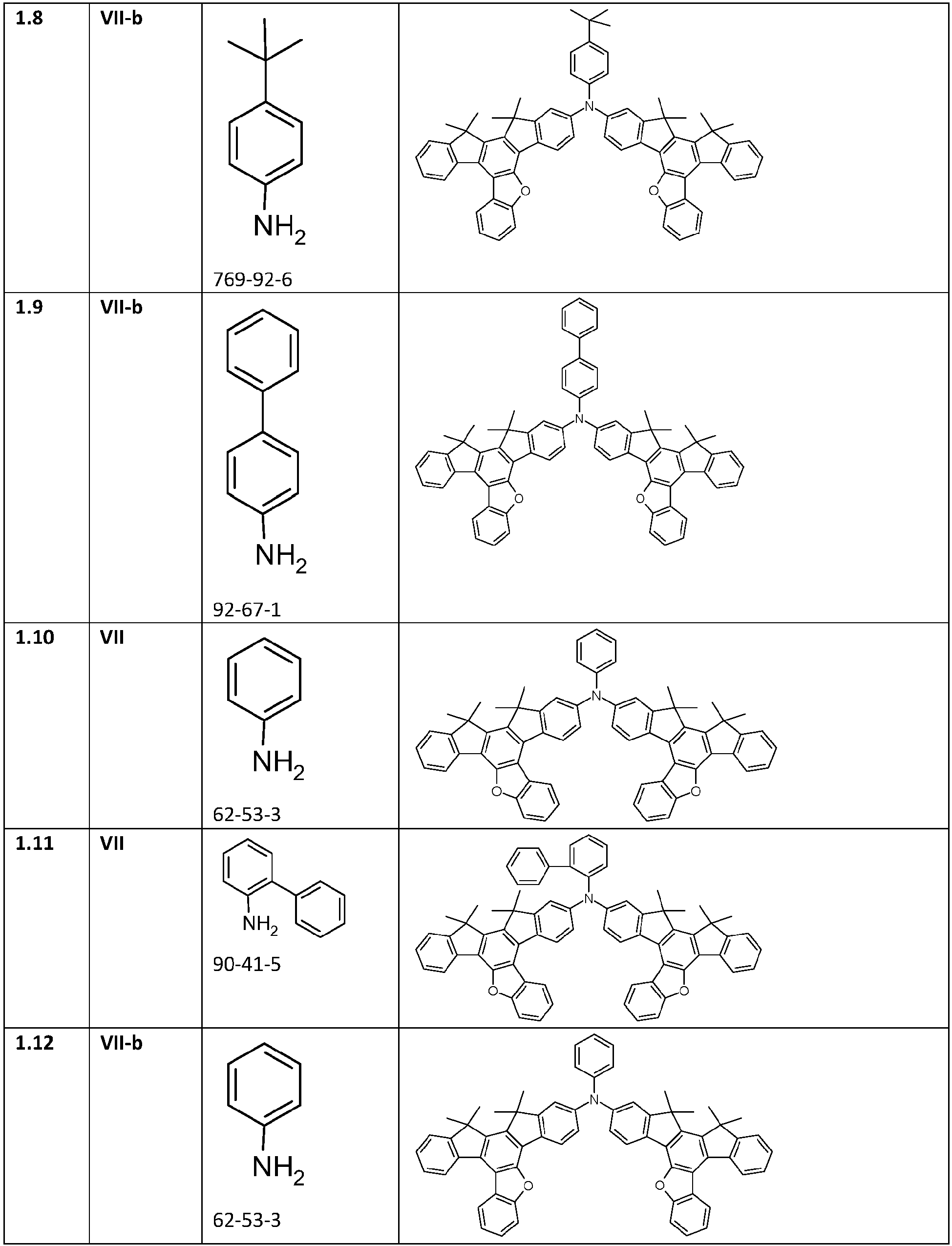

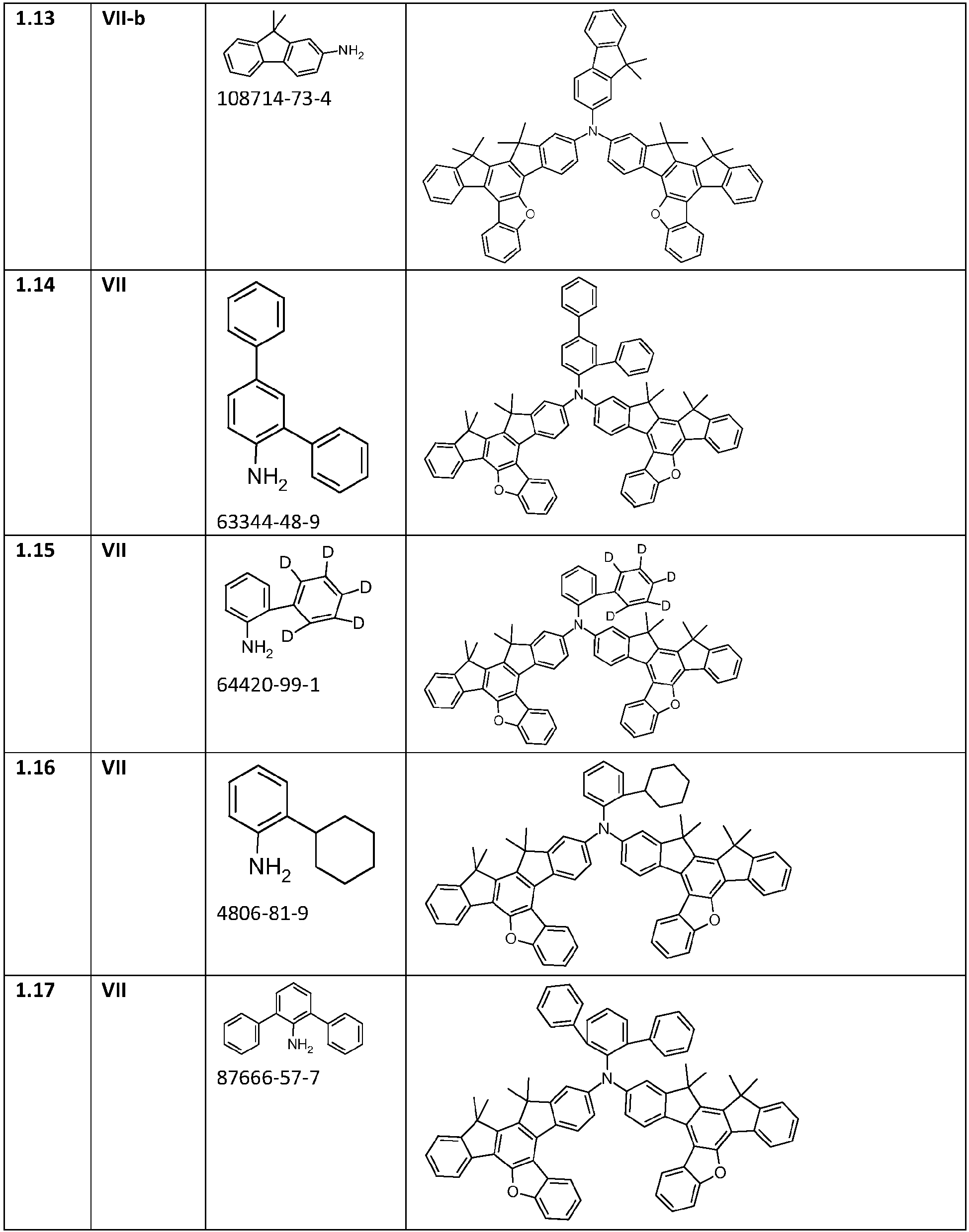

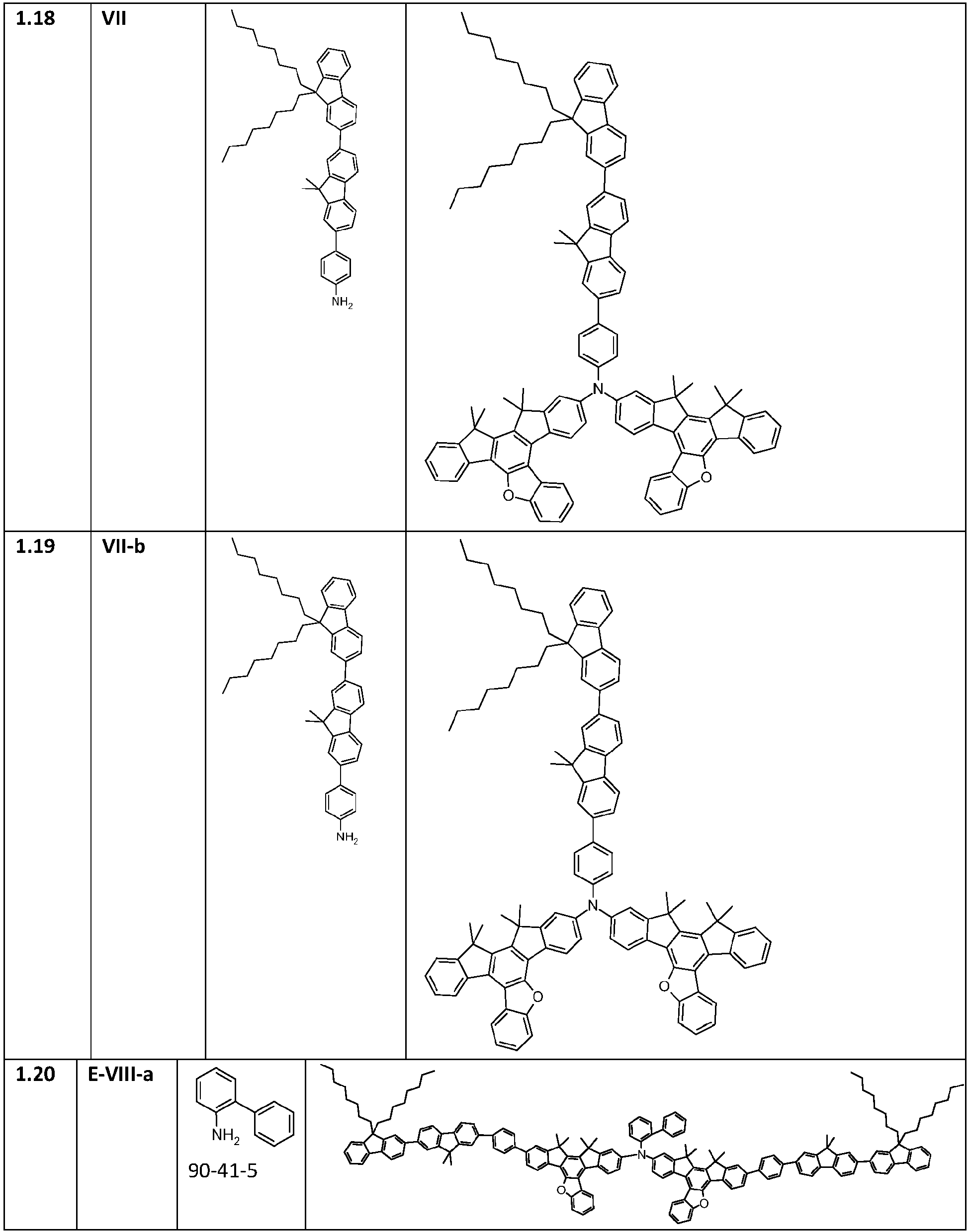

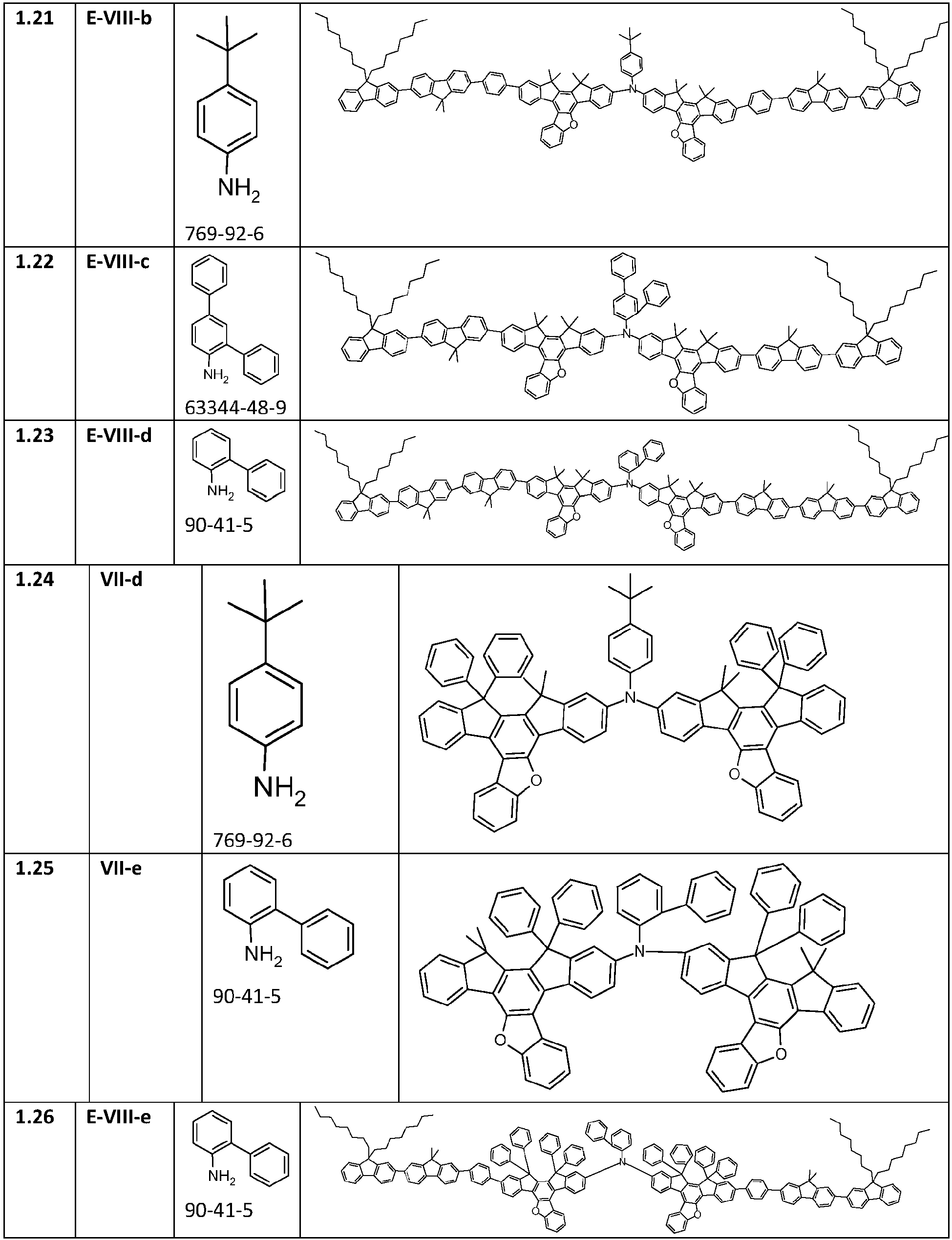

### B) Fabrication of OLEDs

### Fabrication of OLED devices from thermal vapor deposition

The manufacturing of the OLEDs is performed accordingly to WO 04/05891 with adapted film thicknesses and layer sequences. The following examples E1 to E4 (see Table 2) show data of various OLEDs.

### Substrate pre-treatment of examples E1-E4:

Glass plates with structured ITO (50 nm, indium tin oxide) are coated with 20 nm PEDOT:PSS (Poly(3,4-ethylenedioxythiophene) poly(styrene-sulfonate, CLEVIOS^{™} P VP AI 4083 from Heraeus Precious Metals GmbH Germany, spin-coated from a water-based solution) and form the substrates on which the OLEDs are processed.

The OLEDs have in principle the following layer structure:
- Substrate,
- ITO (50 nm),
- Buffer (20 nm),
- Hole injection layer (HTL1 95%, HIL 5%) (20 nm),
- Hole transporting layer (HTL2) (20 nm),
- Emissive layer (EML) (20 nm),
- Electron transporting layer (ETL 50%, EIL 50%) (30 nm),
- Electron injection layer (EIL) (3 nm),
- Cathode.

The cathode is formed by an aluminium layer with a thickness of 100 nm. The materials used for the OLED fabrication are presented in Table 1.

All materials are applied by thermal vapour deposition in a vacuum chamber. The emission layer here always consists of at least one matrix material (host material=H) and an emitting dopant (emitter=D), which is admixed with the matrix material or matrix materials in a certain proportion by volume by co-evaporation. An expression such as H1:D1 (95%:5%) here means that material H1 is present in the layer in a proportion by volume of 95%, whereas D1 is present in the layer in a proportion of 5%. Analogously, the electron-transport layer may also consist of a mixture of two or more materials.

The OLEDs are characterised by standard methods. For this purpose, the electroluminescence spectra and the external quantum efficiency (EQE, measured in % at 1000 cd/m²) are determined from current/voltage/luminance characteristic lines (IUL characteristic lines) assuming a Lambertian emission profile. The electroluminescence (EL) spectra are recorded at a luminous density of 1000 cd/m² and the CIE 1931 x and y coordinates are then calculated from the EL spectrum. The device data of various OLEDs are summarized in Table 2.

In the following section several examples are described in more detail to show the advantages of the inventive OLEDs.

### Use of inventive compounds as emitting material in fluorescent OLEDs

The inventive compounds are especially suitable as an emitter (dopant) when blended into a fluorescent blue matrix to form the emissive layer of a fluorescent blue OLED device. The representative examples are D1, D2, D3 and D4 (device data see Table 2).

| **Table 1: Chemical structures of the materials used for thermally evaporated OLEDs** | |
|---|---|
| 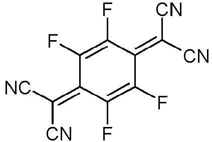 | 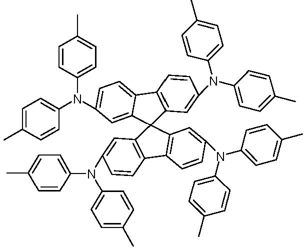 |
| HIL | HTL1 |
| 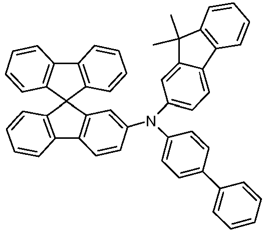 | 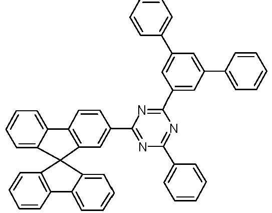 |
| HTL2 | ETL |
| 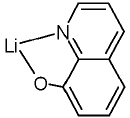 | 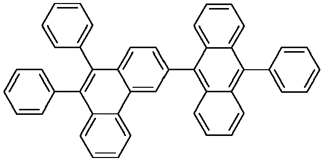 |
| EIL | H1 |
| 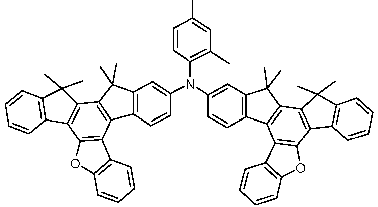 | 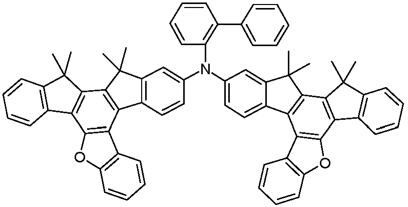 |
| D1 | D2 |
| 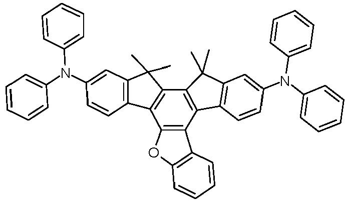 | 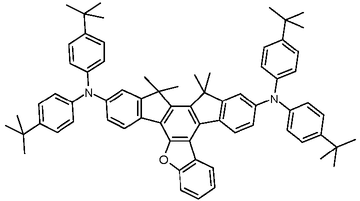 |
| D3* | D4* |

| **Table 2: Device data of OLEDs from thermal evaporation** | | | | | |
|---|---|---|---|---|---|
| ***Example*** | ***Host*** | ***Dopant*** | ***EQE* @ *1000 cd*/*m²*** | ***CIE*** | |
| | *95 %* | *5%* | *%* | *x* | y |
| E1 | H1 | D1 | 6.1 | 0.14 | 0.14 |
| E2 | H1 | D2 | 6.0 | 0.14 | 0.14 |
| E3* | H1 | D3 | 6.4 | 0.14 | 0.10 |
| E4* | H1 | D4 | 6.6 | 0.14 | 0.15 |

| | | | | | |
|---|---|---|---|---|---|
| * Non-inventive example | | | | | |

Table 2 shows that use of materials D1 and D2 according to the present invention results in excellent device data when used as fluorescent blue emitters.

### Fabrication of solution processed OLED devices

The production of solution-based OLEDs has already been described many times in the literature, for example in WO 2004/037887 and WO 2010/097155. The process is adapted to the circumstances described below (layer-thickness variation, materials).

The inventive material combinations are used in the following layer sequence:
- substrate,
- ITO (50 nm),
- Buffer (20 nm),
- hole transport layer (20 nm),
- emission layer (EML) (50 nm),
- electron-transport layer (ETL) (20 nm),
- electron injection layer (EIL) (3 nm),
- cathode (Al) (100 nm).

Glass plates coated with structured ITO (indium tin oxide) in a thickness of 50 nm serve as substrate. These are coated with the buffer (PEDOT) Clevios P VP Al 4083 (Heraeus Clevios GmbH, Leverkusen). The spin coating of the buffer is carried out from water in air. The layer is subsequently dried by heating at 180°C for 10 minutes. The hole transport layers and the emission layers are applied to the glass plates coated in this way.

The hole-transport layer is the polymer HTL of the structure shown in Table 3, which was synthesised based on monomers in accordance with CAS 374934-77-7, WO2013156130 and WO2010/097155. The polymer is dissolved in toluene, so that the solution typically has a solid content of approx. 5 g/l if, as here, the layer thickness of 20 nm which is typical for a device is to be achieved by means of spin coating. The layers are applied by spin coating in an inert-gas atmosphere, in the present case argon, and dried by heating at 220°C for 30 min.

The emission layer is composed of the matrix material (host material) H1 and the emitting dopant (emitter) D2. Both materials are present in the emission layer in a proportion of 92 % by weight H1 and 8 % by weight D2. The mixture for the emission layer is dissolved in toluene. The solids content of such solutions is about 14 mg/ml if, as here, the layer thickness of 40 nm which is typical for a device is to be achieved by means of spin coating. The layers are applied by spin coating in an inert-gas atmosphere and dried by heating at 170°C for 10 minutes. Other used materials, apart the ones shown in Table 1, are depicted in Table 3.

**Table 3: Structural formulae of the additional materials used for the solution processed OLEDs**

| |
|---|
| 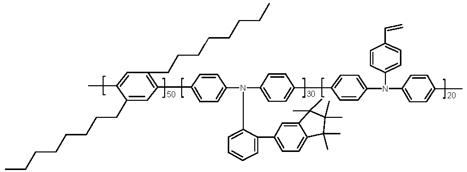 |
| HTL |
| 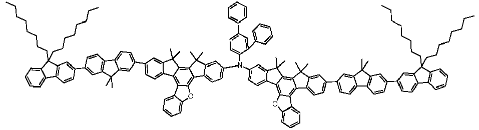 |
| D5 |
| 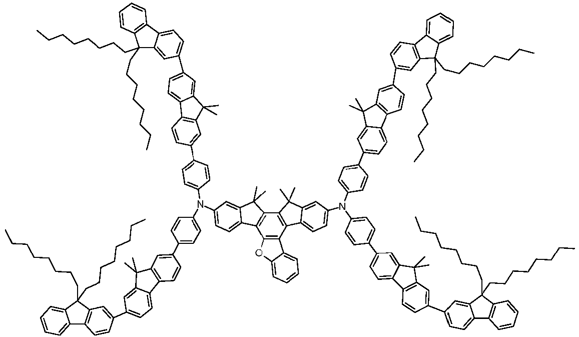 |
| D6* |

| |
|---|
| * non-inventive example |

The materials for the electron-transport layer and the electron injection layer are likewise applied by thermal vapour deposition in a vacuum chamber and are shown in Table 1. The electron-transport layer consists of the material ETL and the electron injection layer consists of EIL. The cathode is formed by the thermal evaporation of an aluminium layer with a thickness of 100 nm. The OLEDs are characterised in the very same way as described for the thermally evaporated OLEDs. Additionally, the lifetime LT80@40mA/cm² is measured, where LT80@40mA/cm² defines the time the initial brightness achieved at a driving current density of 40mA/cm² is dropped by 20% compared to the initial starting level. In the following section several examples are described in more detail to show the advantages of the inventive OLEDs.

### Use of inventive compounds as emitting material in fluorescent OLEDs

The inventive compounds are especially suitable as an emitter (dopant) when blended into a fluorescent blue matrix to form the emissive layer of a fluorescent blue OLED device. The representative examples are D2, D4, D5 and D6 (device data see Table 4).

The properties of the various OLEDs are summarised in Table 4. Examples E5 to E8 show properties of OLEDs containing materials of the present invention.

| **Table 4: Device data of solution processed OLEDs** | | | | | | |
|---|---|---|---|---|---|---|
| ***Example*** | ***Host*** | ***Dopant*** | ***EQE* @ *1000 cd*/*m²*** | ***LT80* @ *40mA*/*cm²*** | ***CIE*** | |
| | *92 %* | *8 %* | *%* | hrs | *x* | y |
| E5 | H1 | D2 | 3.9 | 74 | 0.14 | 0.16 |
| E6 | H1 | D4 | 3.8 | 59 | 0.14 | 0.18 |
| E7 | H1 | D5 | 4.8 | 85 | 0.14 | 0.17 |
| E8* | H1 | D6 | 5.2 | 68 | 0.13 | 0.19 |

| | | | | | | |
|---|---|---|---|---|---|---|
| *non-inventive example | | | | | | |

Table 4 shows that use of materials D2, D4 and D5 according to the present invention results in excellent device data when used as fluorescent blue emitters. The lifetime LT80 at a constant current density of 40 mA/cm² is better for the mono-amine type emitter in comparison with the di-amine-type.

## Claims

1. Compound of the formula (3A), (3B) or (3C), 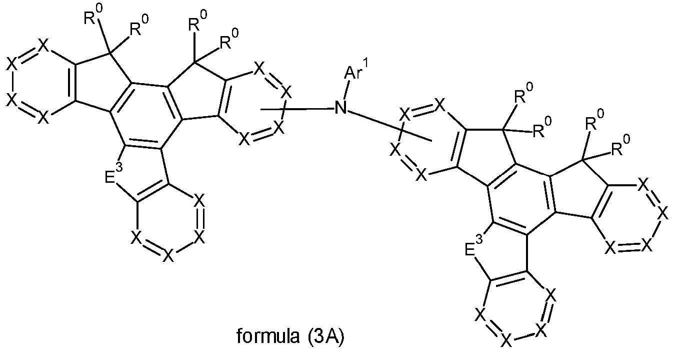 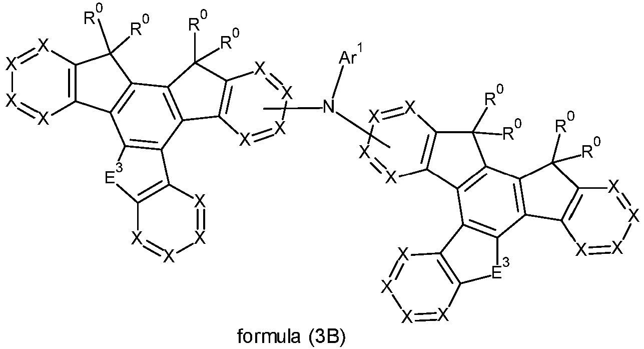 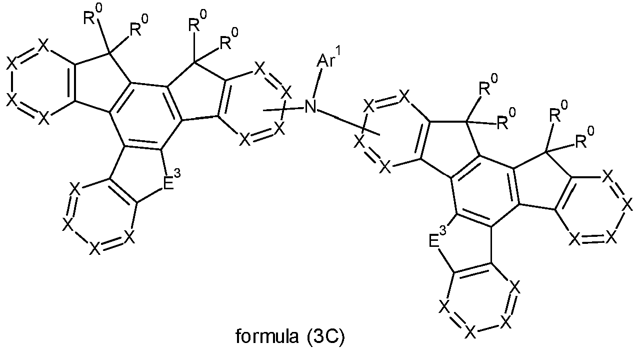 where the following applies to the symbols and indices used:
E³ stands for -O-;
Ar¹ stands on each occurrence, identically or differently, for an aromatic or heteroaromatic ring system having 5 to 30 aromatic ring atoms, which may in each case be substituted by one or more radicals R¹;
X stands for CR² or N; or X stands for C, if it is bonded to the nitrogen atom as depicted in formulae (3A), (3B) and (3C);
R⁰ stands on each occurrence, identically or differently, for H, D, F, a straight-chain alkyl group having 1 to 10 C atoms or branched or a cyclic alkyl group having 3 to 10 C atoms, each of which may be substituted by one or more radicals R, where one or more H atoms may be replaced by D or F, an aromatic or heteroaromatic ring systems having 5 to 18 aromatic ring atoms, which may in each case be substituted by one or more radicals R, where two adjacent radicals R⁰ may form an aliphatic or aromatic ring system together, which may be substituted by one or more radicals R;
R¹, R² stand on each occurrence, identically or differently, for H, D, F, CN, a straight-chain alkyl group having 1 to 10 C atoms or branched or a cyclic alkyl group having 3 to 10 C atoms, each of which may be substituted by one or more radicals R, where one or more H atoms may be replaced by D or F, an aromatic or heteroaromatic ring systems having 6 to 18 aromatic ring atoms, which may in each case be substituted by one or more radicals R;
R stands on each occurrence, identically or differently, for H, D, F, CN, a straight-chain alkyl group having 1 to 10 C atoms or branched or cyclic alkyl group having 3 to 10 C atoms, each of which may be substituted by one or more radicals R', an aromatic or heteroaromatic ring systems having 5 to 30 aromatic ring atoms, which may in each case be substituted by one or more radicals R';
R' stands on each occurrence, identically or differently, for H, D, F, Cl, Br, I, CN, a straight-chain alkyl, alkoxy or thioalkyl groups having 1 to 10 C atoms or branched or cyclic alkyl, alkoxy or thioalkyl groups having 3 to 10 C atoms.

2. Compound according to claim 1, **characterized in that**, when Ar¹ stands for an aromatic or heteroaromatic ring system, then it is selected from the group consisting of benzene, naphthalene, anthracene, phenanthrene, biphenyl, terphenyl, quaterphenyl, fluorene, spirobifluorene, cis- or trans-indenofluorene, truxene, benzofuran, dibenzofuran, benzothiophene, dibenzothiophene, carbazole, indolocarbazole, indenocarbazole, pyridine, pyrimidine, triazine, quinoline, acridine, phenanthridine, benzoquinoline, phenothiazine, phenoxazine, benzopyrimidine, quinoxaline, pyrazine and phenazine, each of which may be substituted by one or more radicals R¹.

3. Compound according to claim 1 or 2, **characterized in that** it is selected from compounds of one of the formulae (3A-1) to (3C-1), 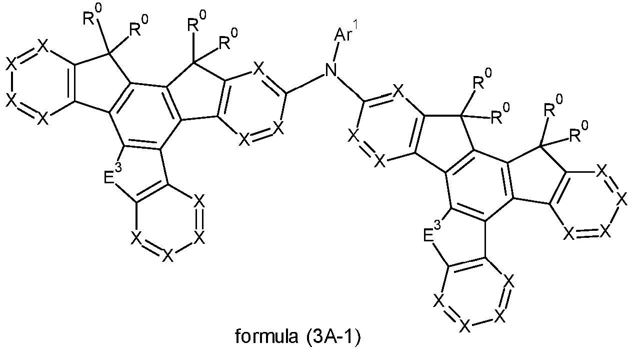 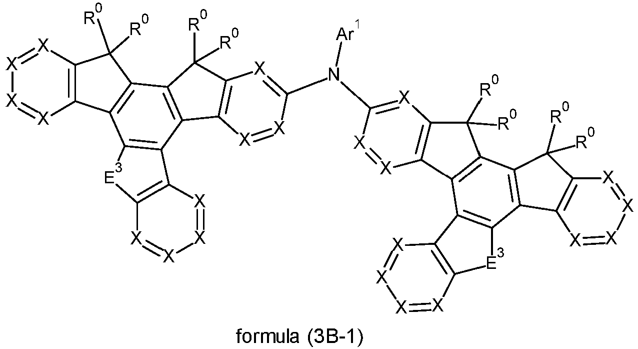 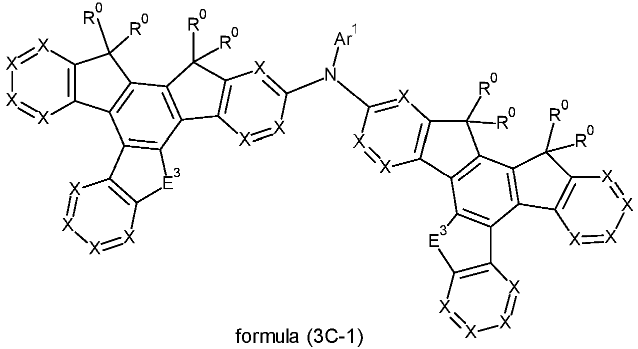
where the symbol E³ stands for -O-;
where X stands for CR² or N; and
where the symbols R⁰, R² and Ar¹ have the same meaning as in claim 1.

4. Formulation comprising at least one compound according to one or more of the claims 1 to 3 and at least one solvent.

5. Electronic device comprising at least one compound according to one or more of claims 1 to 3, selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

6. Electronic device according to claim 5, which is an organic electroluminescent device, **characterized in that** the compound according to one or more of claims 1 to 3 is employed as a fluorescent emitting compound in an emitting layer.

## Patentansprüche

1. Verbindung der Formel (3A), (3B) oder (3C), 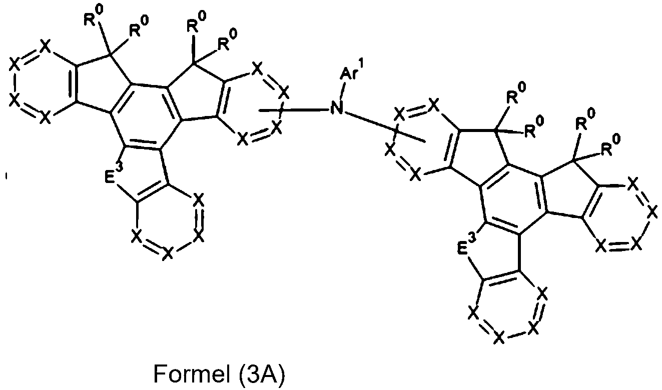 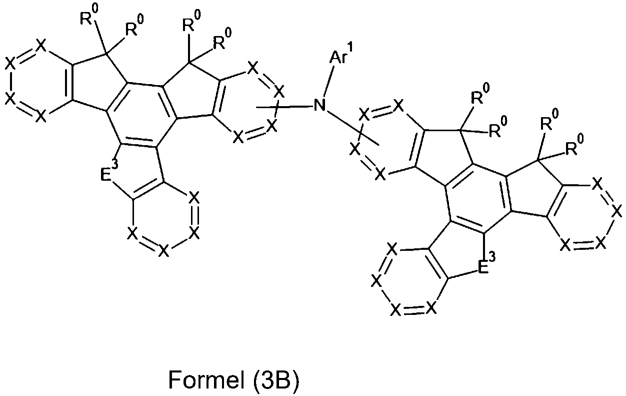 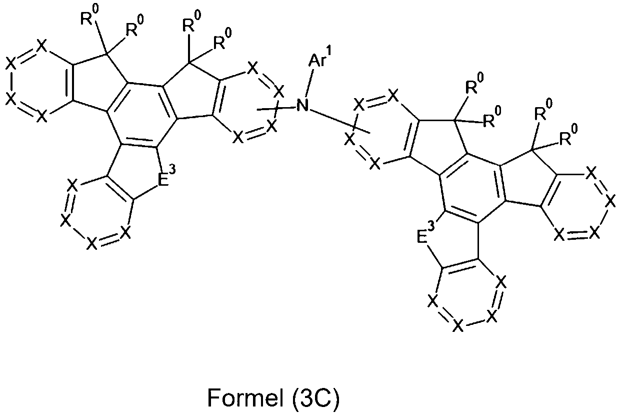 wobei für die verwendeten Symbole und Indizes Folgendes gilt:
E³ steht für -O-;
Ar¹ steht bei jedem Auftreten, gleich oder verschieden, für ein aromatisches oder heteroaromatisches Ringsystem mit 5 bis 30 aromatischen Ringatomen, das jeweils durch einen oder mehrere Reste R¹ substituiert sein kann;
X für CR² oder N steht; oder X für C steht, wenn es an das in den Formeln (3A), (3B) und (3C) dargestellte Stickstoffatom gebunden ist;
R⁰ steht bei jedem Auftreten, gleich oder verschieden, für H, D, F, eine geradkettige Alkylgruppe mit 1 bis 10 C-Atomen oder eine verzweigte oder eine cyclische Alkylgruppe mit 3 bis 10 C-Atomen, die jeweils durch einen oder mehrere Reste R substituiert sein können, wobei ein oder mehrere H-Atome durch D oder F ersetzt sein können, ein aromatisches oder heteroaromatisches Ringsystem mit 5 bis 18 aromatischen Ringatomen, das jeweils durch einen oder mehrere Reste R substituiert sein kann, wobei zwei benachbarte Reste R⁰ gemeinsam ein aliphatisches oder aromatisches Ringsystem bilden können, das durch einen oder mehrere Reste R substituiert sein kann;
R¹, R² stehen bei jedem Auftreten gleich oder verschieden für H, D, F, CN, eine geradkettige Alkylgruppe mit 1 bis 10 C-Atomen oder eine verzweigte oder eine cyclische Alkylgruppe mit 3 bis 10 C-Atomen, die jeweils durch einen oder mehrere Reste R substituiert sein können, wobei ein oder mehrere H-Atome durch D oder F ersetzt sein können, ein aromatisches oder heteroaromatisches Ringsystem mit 6 bis 18 aromatischen Ringatomen, die jeweils durch einen oder mehrere Reste R substituiert sein können;
R steht bei jedem Auftreten, gleich oder verschieden, für H, D, F, CN, eine geradkettige Alkylgruppe mit 1 bis 10 C-Atomen oder eine verzweigte oder cyclische Alkylgruppe mit 3 bis 10 C-Atomen, die jeweils durch einen oder mehrere Reste R' substituiert sein können, ein aromatisches oder heteroaromatisches Ringsystem mit 5 bis 30 aromatischen Ringatomen, die jeweils durch einen oder mehrere Reste R' substituiert sein können;
R' bei jedem Auftreten, gleich oder verschieden, für H, D, F, Cl, Br, I, CN, eine geradkettige Alkyl-, Alkoxy- oder Thioalkylgruppe mit 1 bis 10 C-Atomen oder verzweigte oder cyclische Alkyl-, Alkoxy- oder Thioalkylgruppen mit 3 bis 10 C-Atomen steht.

2. Verbindung nach Anspruch 1, **dadurch gekennzeichnet, dass**, wenn Ar¹ für ein aromatisches oder heteroaromatisches Ringsystem steht, dieses ausgewählt ist aus der Gruppe bestehend aus Benzol, Naphthalin, Anthracen, Phenanthren, Biphenyl, Terphenyl, Quaterphenyl, Fluoren, Spirobifluoren, cis- oder trans-Indenfluoren, Truxen, Benzofuran, Dibenzofuran, Benzothiophen, Dibenzothiophen, Carbazol, Indolocarbazol, Indenocarbazol, Pyridin, Pyrimidin, Triazin, Chinolin, Acridin, Phenanthridin, Benzochinolin, Phenothiazin, Phenoxazin, Benzopyrimidin, Chinoxalin, Pyrazin und Phenazin, von denen jede durch einen oder mehrere Reste R¹ substituiert sein kann.

3. Verbindung nach Anspruch 1 oder 2, **dadurch gekennzeichnet, dass** sie ausgewählt ist aus Verbindungen mit einer der Formeln (3A-1) bis (3C-1), 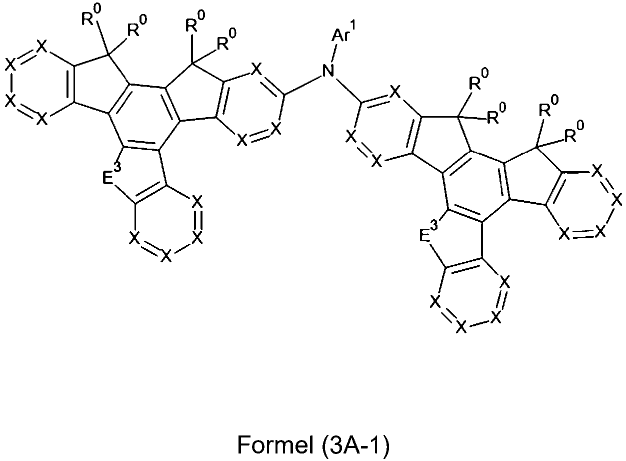 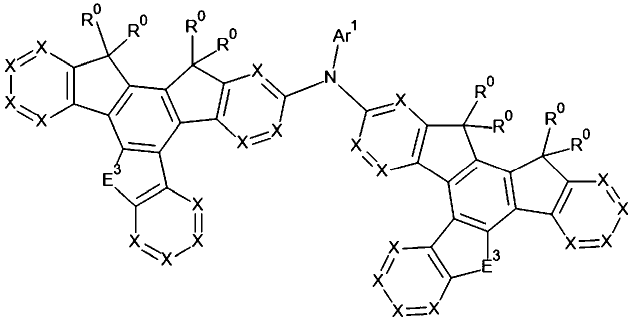 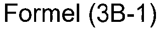 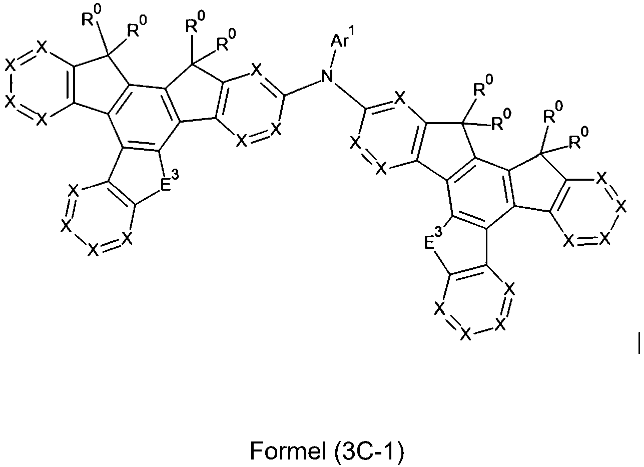
wobei das Symbol E³ für -O- steht;
wobei X für CR² oder N steht; und
wobei die Symbole R⁰, R² und Ar¹ die gleiche Bedeutung wie in Anspruch 1 haben.

4. Formulierung, umfassend mindestens eine Verbindung nach einem oder mehreren der Ansprüche 1 bis 3 und mindestens ein Lösungsmittel.

5. Elektronische Vorrichtung, umfassend mindestens eine Verbindung nach einem oder mehreren der Ansprüche 1 bis 3, ausgewählt aus der Gruppe bestehend aus organischen Elektrolumineszenzvorrichtungen, organischen integrierten Schaltkreisen, organischen Feldeffekttransistoren, organischen Dünnschichttransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Vorrichtungen, lichtemittierenden elektrochemischen Zellen, organischen Laserdioden und organischen Plasmonen emittierenden Vorrichtungen.

6. Elektronische Vorrichtung nach Anspruch 5, die eine organische elektrolumineszente Vorrichtung ist, **dadurch gekennzeichnet, dass** die Verbindung nach einem oder mehreren der Ansprüche 1 bis 3 als fluoreszierende emittierende Verbindung in einer emittierenden Schicht verwendet wird.

## Revendications

1. Composé de la formule (3A), (3B) ou (3C), 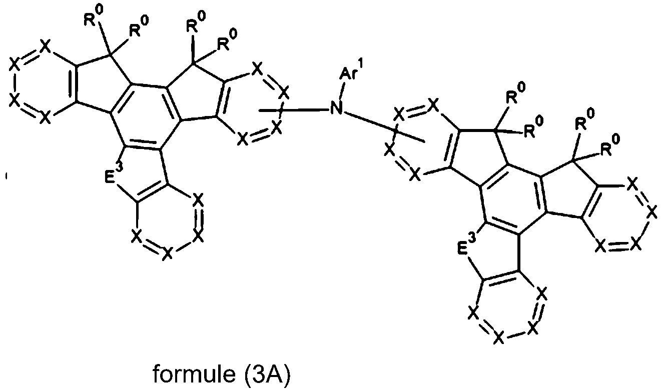 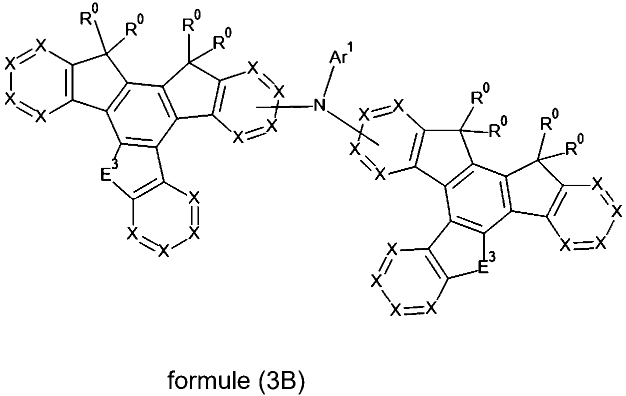 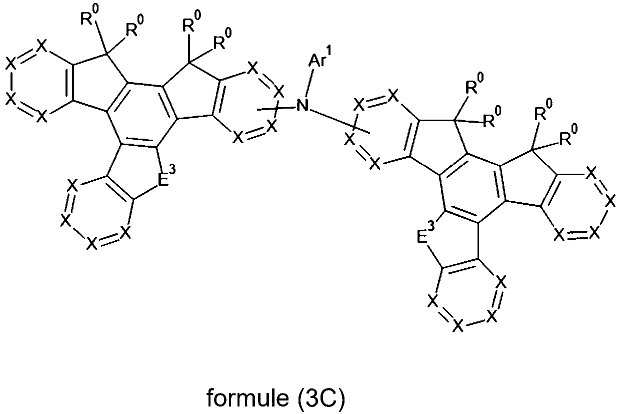 où les symboles et indices suivants s'appliquent:
E³ représente -O-;
Ar¹ désigne à chaque occurrence, de manière identique ou différente, un système de cycle aromatique ou hétéroaromatique comportant de 5 à 30 atomes de cycle aromatique, qui peut dans chaque cas être substitué par un ou plusieurs radicaux R¹;
X représente CR² ou N; ou X représente C, s'il est lié à l'atome d'azote comme indiqué dans les formules (3A), (3B) et (3C);
R⁰ représente, à chaque occurrence, de manière identique ou différente, H, D, F, un groupe alkyle à chaîne linéaire ayant de 1 à 10 atomes de carbone ou un groupe alkyle ramifié ou cyclique ayant de 3 à 10 atomes de carbone, chacun pouvant être substitué par un ou plusieurs radicaux R, où un ou plusieurs atomes de H peuvent être remplacés par D ou F, un système cyclique aromatique ou hétéroaromatique ayant de 5 à 18 atomes d'anneau aromatique, qui peut dans chaque cas être substitué par un ou plusieurs radicaux R, où deux radicaux adjacents R⁰ peuvent former ensemble un système cyclique aliphatique ou aromatique, qui peut être substitué par un ou plusieurs radicaux R ;
R¹, R² représentent à chaque occurrence, de manière identique ou différente, H, D, F, CN, un groupe alkyle à chaîne linéaire ayant de 1 à 10 atomes de carbone ou un groupe alkyle ramifié ou cyclique ayant de 3 à 10 atomes de carbone, chacun pouvant être substitué par un ou plusieurs radicaux R, où un ou plusieurs atomes de H peuvent être remplacés par D ou F, un système cyclique aromatique ou hétéroaromatique ayant de 6 à 18 atomes de cycle aromatique, qui peut dans chaque cas être substitué par un ou plusieurs radicaux R;
R représente à chaque occurrence, de manière identique ou différente, H, D, F, CN, un groupe alkyle à chaîne linéaire ayant de 1 à 10 atomes de carbone ou un groupe alkyle ramifié ou cyclique ayant de 3 à 10 atomes de carbone, chacun pouvant être substitué par un ou plusieurs radicaux R', un système de cycles aromatiques ou hétéroaromatiques ayant de 5 à 30 atomes de cycles aromatiques, qui peuvent dans chaque cas être substitués par un ou plusieurs radicaux R';
R' représente à chaque occurrence, de manière identique ou différente, H, D, F, CI, Br, I, CN, un groupe alkyle, alcoxy ou thioalkyle à chaîne droite ayant de 1 à 10 atomes de carbone ou un groupe alkyle, alcoxy ou thioalkyle ramifié ou cyclique ayant de 3 à 10 atomes de carbone.

2. Composé selon la revendication 1, **caractérisé en ce que**, lorsque Ar¹ représente un système cyclique aromatique ou hétéroaromatique, il est constitué par le benzène, le naphtalène, l'anthracène, le phénanthrène, le biphényle, le terphényle, le quaterphényle, le fluorène, le spirobifluorène, le cis- ou le trans-indénofluorène, le truxène, le spirotruxène, le benzofurane, dibenzofurane, benzothiophène, dibenzothiophène, carbazole, indolocarbazole, indénocarbazole, pyridine, pyrimidine, triazine, quinoléine, acridine, phénanthridine, benzoquinoline, phénothiazine, phénoxazine, benzopyrimidine, quinoxaline, pyrazine et phénazine, chacun pouvant être substitué par un ou plusieurs radicaux R¹, ou par des combinaisons de ces groupes.

3. Composé selon la revendication 1 ou 2, **caractérisé en ce qu'**il est choisi parmi les composés de l'une des formules (3A-1) à (3C-1), 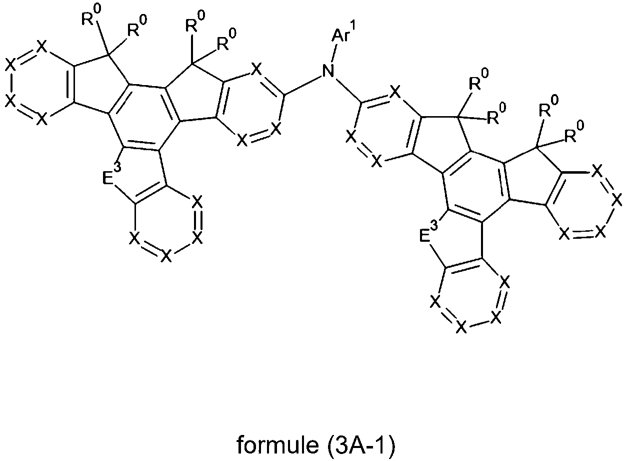 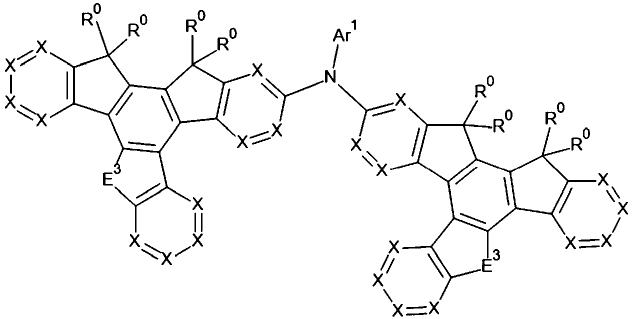 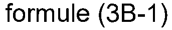 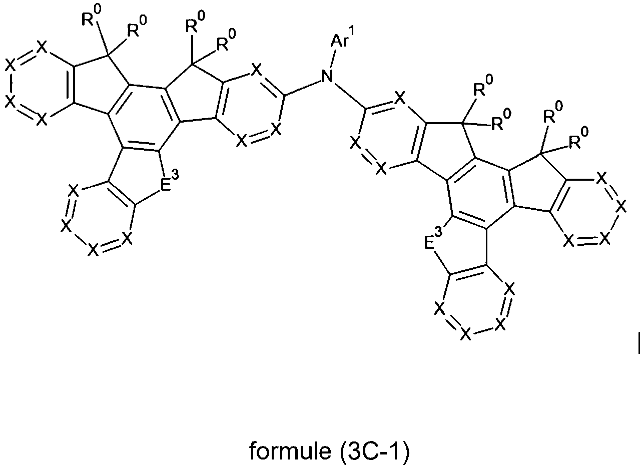
où le symbole E³ représente -O-;
où X représente CR² ou N; et
où les symboles R⁰ et Ar¹ ont la même signification que dans la revendication 1.

4. Formulation comprenant au moins un composé selon une ou plusieurs des revendications 1 à 3 et au moins un solvant.

5. Dispositif électronique comprenant au moins un composé selon une ou plusieurs des revendications 1 à 3, constitué de dispositifs électroluminescents organiques, de circuits intégrés organiques, de transistors à effet de champ organiques, de transistors à couche mince organiques, de transistors électroluminescents organiques, de cellules solaires organiques, de cellules solaires organiques à colorant, de détecteurs optiques organiques, de photorécepteurs organiques, de dispositifs d'extinction de champ organiques, de cellules électrochimiques lumineuses, de diodes laser organiques et de dispositifs électroluminescents organiques.

6. Dispositif électronique selon la revendication 5, qui est un dispositif organique électroluminescent, **caractérisé en ce que** le composé selon une ou plusieurs des revendications 1 à 3 est employé comme composé émetteur fluorescent dans une couche émettrice.
